# EUROPÄISCHE PATENTANMELDUNG

(11) **EP 3 981 442 A1**
(43) Veröffentlichungstag der Anmeldung: **13.04.2022**
(21) Anmeldenummer: 21199489.2
(22) Anmeldetag: 28.09.2021
(51) Int. Cl.: A61L 9/12, A61L 9/16, B01D 46/00, G01N 15/14

(54) **DURCH EINE TEMPERATURDIFFERENZ BETREIBBARE, MOBILE VORRICHTUNG ZUR REINIGUNG UND DESINFIZIERUNG VON RAUMLUFT**

(30) Priorität: 04.10.2020 DE 102020125920; 04.10.2020 DE 202020105699 U
(71) Anmelder: Münch, Elke, 55452 Dorsheim (DE)
(72) Erfinder: Münch, Elke, 55452 Dorsheim (DE); Münch, Volker, 55452 Dorsheim (DE)
(74) Vertreter: Müller, Jochen

(57) **Zusammenfassung**

Durch eine Temperaturdifferenz betriebene, mobile Vorrichtung (1) gemäß Figur 1 zur Reinigung und Desinfizierung von Raumluft, umfassend eine rohrförmige vertikale Außenwand (1.1) mit einer oberen Öffnung (1.5), einem Bodenbereich (1.7), einer horizontalen Standfläche (1.7.1), einem Innenraum (1.7.2) für den Einschub eines aufladbaren Akkumulators (7) und eines elektronischen Steuergeräts (4) zur Regelung des durch den Kamineffekt erzeugten Luftstroms durch eine auswechselbare Kartusche (5) mit einer die obere Öffnung (1.6), einer umlaufenden Kartuschenhalterung (5.4), einem luftdurchlässigen Kartuschenboden (5.3) und einer partikulären, luftdurchlässigen, bakteriziden und/oder viruziden Kartuschenfüllung (5.2) sowie mindestens zwei einander gegenüberliegende, vertikal angeordnete Peltier-Elemente (2), die mit ihren kalten Seiten (2.2) mit einem Kühlring (2.4) in der Kartuschenwand (5.1) und mit ihren heißen Seiten (2.1) in wärmeleitendem Kontakt zu mindestens zwei vertikalen Wärmeleitungen (2.1.1) stehen, mit denen eine auf dem Bodenbereich (1.7) angeordnete horizontale Heizplatte (2.1.2) erwärmt wird, sowie mindestens einen Heizraum (2.1.6) oberhalb der Heizplatte (2.1.2) zum Aufheizen der durch mindestens zwei Lufteinlässe (3) einströmenden Luft (3.1), wobei eine Wärmedämmbeschichtung (2.1.4) die mindestens zwei Peltier-Elemente (2) seitlich und die wärmeleitenden Bauteile (2.1.1) gänzlich und die Heizplatten (2.1.2) bis auf deren Oberseite umhüllt; sowie ein Verfahren zur Reinigung und Desinfizierung von Raumluft.

## Beschreibung

### Gebiet der Erfindung

Die vorliegende Erfindung betrifft eine durch eine Temperaturdifferenz betreibbare, mobile Vorrichtung zur Reinigung und Desinfizierung von Raumluft.

Außerdem betrifft die vorliegende Erfindung ein Verfahren zur Reinigung und Desinfizierung von Raumluft mithilfe einer durch eine Temperaturdifferenz betriebenen, mobilen Vorrichtung.

Des Weiteren betrifft die vorliegende Erfindung die Verwendung der durch eine Temperaturdifferenz betreibbaren, mobilen Vorrichtung zur Abtötung von Mikroorganismen, speziell Viren und Virionen, die im Folgenden zusammenfassend als Viren bezeichnet werden.

### Stand der Technik

Mikroorganismen sind Archaeen, Bakterien, Eukaryoten, Protisten, Pilze und Grünalgen. Es ist immer noch ein Gegenstand von Diskussionen, ob Viren oder Virionen überhaupt als Organismen anzusehen sind. Die Mikroorganismen und Viren sind die Quelle einer Reihe von schweren Krankheiten, von Epidemien und Pandemien wie die gegenwärtige Covid-19-Pandemie.

Zahlreiche Pestizide wie Fungizide, Herbizide, Insektizide Algizide, Molluskizide, Rodentizide, Akarizide und Schleimbekämpfungsmittel sind entwickelt worden, um die schädlichen Wirkungen auf multizelluläre Menschentieren und Pflanzen zu bekämpfen.

In gleicher Weise sind zahlreiche antimikrobielle Wirkstoffe wie Germicide, Antibiotika, Bakterizide, Viruzide, Antimykotika, Antiprotozoenmittel und Antiparasitenmittel entwickelt worden um die Krankheiten, die durch die Mikroorganismen ausgelöst werden, zu heilen.

Die permanente Bedrohung durch Mikroorganismen, insbesondere durch Viren und Virionen und ganz speziell durch das Coronavirus SARS-Co-V2 hat eine wachsende Nachfrage nach effizienten und effektiven Methoden für die Dekontamination und Desinfektion hervorgerufen. Die "List N: Products with Emerging Viral Pathogens AND Human Coronavirus Claims for Use against SARS-CoV-2, Date Accessed: 05/31/2020 of the EPA lists, US Govt." führt zahlreiche organische und anorganische aktive Verbindungen wie HOCI, Peroxoessigsäure, quaternäres Ammonium, Kaliumperoxomonosulfat, Chlordioxid, Wasserstoffperoxid, Zitronensäure, Milchsäure, Dichlorisocyanurat, Natriumhypochlorit oder Ethanol. Diese Desinfektionsmittel können aber nur in Reinigungslösungen oder Wischlösungen verwendet werden und haben keine dauerhafte desinfizierende Wirkung.
Propioconazol (±)-1-{[2-(2,4-Dichlorphenyl)-4-propyl-1,3-dioxolan-2-yl]methyl}-*H*-1,2,4-triazol (IUPAC),
Folpet
   *N*-(Trichlormethylthio)phthalimid,
Chlorkresole,
Fludioxonil
   4-(2,2-Difluor-benzo[1,3]dioxol-4-yl)pyrrol-3-carbonitril (IUPAC), and
Azoxvstrobin Methyl-(*E*)-2-{2-[6-(2-cyanophenoxy)pyrimidin-4-yloxyl]phenyl}-3-methoxyacrylat (IUPAC)
werden als genehmigte Schutzmittel für Fasern, Leder, Gummi und polymerisierte Materialien in dem"Helpdesk - Genehmigte Wirkstoffe - Bundesanstalt für Arbeitsschutz und Arbeitsmedizin":
https://www.reach-clp-biozid-helpdesk.de/DE/Biozide/Wirkstoffe/Genehmigte-Wirkstoffe/Genehmigte-Wirkstoffe-0.html#PT9
aufgeführt.

Diese Wirkstoffe sind jedoch niedermolekulare Verbindungen, sodass stets die Gefahr besteht, dass sie aus den geschützten Materialien ausgelaugt werden.

Deswegen hat es schon zahlreiche Versuche gegeben, die desinfizierenden Wirkstoffe und Pharmazeutika zu immobilisieren, um eine dauerhafte desinfizierende und/oder pharmazeutisch wirksame Oberfläche zu erzielen.

So offenbart die internationale Patentanmeldung WO 96/39821 Reagenzien und Verfahren um Textilien mit dem Ziel zu modifizieren, dass sie Viren beim Kontakt desaktivieren. Dazu werden die Textilien modifiziert, indem man hydrophile Polymere, die quaternäre Ammoniumgruppen und Kohlenwasserstoffketten enthalten, photochemisch immobilisiert, wodurch man eine Oberfläche erhält, die in der Lage ist, lipidumhüllte Viren beim Kontakt zu zerreißen. Wenn diese hydrophilen Polymere aber auf Vliesstoffe aufgetragen werden, besteht keine Garantie, dass sie durch das Licht vollständig vernetzt werden, weil Teilbereiche notwendigerweise beschattet werden. Demzufolge bleibt stets ein gewisser Anteil der Polymere löslich.

Aus dem amerikanischen Patent US 5,883,155 gehen Filme aus Elastomeren hervor, worin aktive Chemikalien wie Biozide für medizinische Zwecke gleichmäßig in der Form von Geleinschlüssen dispergiert sind. Beispielsweise enthält der elastomere Film als Wirkstoffe quaternäres Ammonium, Phthalaldehyd, Phenolderivate, Formalin, nichtionische Tenside, die mindestens einen Polyoxyethylen-Block enthalten, Hexamidin, Iodverbindungen, oberflächenaktive Substanzen mit viruzider Wirkung, Natrium- und Kaliumdichromat und Hydrochlorite. Diese Wirkstoffe sind jedoch toxisch und krebserzeugend und werden in die Umwelt abgegeben.

Das amerikanische Patent US 6,180,584 B1 offenbart desinfizierende Mischungen mit länger anhaltender biozider Wirkung. Die Mischungen bilden einen haftfähigen, transparenten, wasserunlöslichen Polymerfilm auf den Substratoberflächen, der eine länger anhaltende antimikrobielle desinfizierende Wirkung hat. Die Wirkung hält auch ohne einen neuen Auftrag länger an. Die desinfizierende Wirkung der Oberfläche beruht auf dem direkten Kontakt, und die Bestandteile werden nicht in eine kontaktierende Lösung in einer Menge freigesetzt, die die Lösung desinfizieren würde. Der Wirkstoff ist ein metallisches Material, insbesondere Silberiodid. Dieses Salz ist aber lichtempfindlich sodass sich im Lauf der Zeit dunkle Flecken in der Mischung bilden.

Die amerikanische Patentanmeldung 2007/0031512 A1 offenbart, dass schichtförmige Phyllosilikate geeignet sind, Viren zu adsorbieren und/oder zu binden und sie so zu desaktivieren. Die schichtförmigen Phyllosilicate können in die menschlichen Nasenöffnungen gesprüht werden oder können in einer Gesichtsmaske enthalten sein, um Infektionen zu verhindern. Sie können zur Inaktivierung der Viren in Wasser, das für den Hautkontakt gedacht ist, suspendiert werden oder Teil eines HVAC Filters sein, der den Transfer von Viren von Zimmer zu Zimmer, zum Beispiel in einem Krankenhaus, verhindert. Die Phyllosilicate können in einem Papier oder einem Wischtuch enthalten sein, um damit Viren auf Möbeln in Krankenhäusern und Operationssälen sowie chirurgischen Geräten zu deaktivieren. Darüber hinaus können die schichtförmigen Phyllosilicate in Anstrichen für Reinräume verwendet werden.

Die internationale Patentanmeldung WO 2007/120509 offenbart eine Maske, die eine Vielzahl von Schichten enthält, wobei die erste Schicht eine Säure oder ein Salz oder einen Ester der Säure enthält. Die zweite Schicht enthält eine Base oder ein Salz oder einen Ester der Base. Die dritte Schicht der Maske enthält ein metallisches Germicid, ausgewählt aus der Gruppe bestehend aus Zink, Kupfer, Nickel, Iod, Mangan, Zinn, Bor, Silber und deren Salze, Komplexierungsmittel und Tenside. Offenbar enthält insbesondere die dritte Schicht toxische Substanzen.

Die amerikanische Patentanmeldung US 2007/0292486 A1 offenbart biozide Polymer-Nano/Mikropartikel-Komposite die ein ionisches Polymer und biozide Metallsalze, insbesondere Silberbromid, enthalten. Das Silberbromid ist gleichmäßig in der Polymermatrix verteilt. Es wird angenommen, dass die biozide Wirkung auf Silberpartikeln, die Silberionen und Bromidionen, freisetzen, beruht. Außerdem wird angenommen, dass die Bromidionen Textilien flammhemmend machen. Die Nachteile dieser Komposite sind der hohe Preis von Silberbromid, die Lichtempfindlichkeit des Salzes, durch die dunkle Verfärbungen in den Kompositschichten hervorgerufen werden, und das Auslaugen von toxischen Silberionen. Darüber hinaus sind die bioziden Metallsalze nicht an der Oberfläche der Komposite konzentriert, sodass der größte Teil der Metallsalze nicht in Kontakt mit den Mikroorganismen kommt.

Die internationale Patentanmeldung WO 2008/127416 A2 offenbart hydrophobe polymere Beschichtungen, die nicht-kovalent auf feste Oberflächen von Metallen, Kunststoffen, Glas, Polymeren, Textilien und anderen Substraten wie Geweben, Verbandsmull, Bandagen, Tüchern und Fasern in der gleichen Weise wie Anstriche durch Pinselauftrag, Sprühen oder Tauchen appliziert werden können, um die Oberflächen biozid oder bakterizid zu machen. Die hydrophoben Polymere enthalten quaternäre Ammoniumgruppen mit langkettigen aliphatischen Gruppen, die mehr als 10 Kohlenstoffatome enthalten. Die hydrophoben Polymere können jedoch durch organische Lösemittel geschädigt und sogar von den Oberflächen ganz entfernt werden.

Die amerikanische Patentanmeldung US 2009/0081249 A1 offenbart antimikrobielle Zusammensetzungen, die zwei oder mehr antivirale Wirkstoffe enthalten, die kovalent an ein Polymer gebunden sind. Geeignete antivirale Wirkstoffe sind Sialinsäure, Zanamivir, Oseltamivir, Amantadin und Rimantadin. Das Polymer ist vorzugsweise wasserlöslich wie Poly(isobutylen-altmaleinsäureanhydrid), Polyasparaginsäure, Poly(l-glutaminsäure), Chitosan, Carboxymethylcellulose, Carboxymethyldextran oder Polyethylenimin. Die Zusammensetzungen können für die enterale oder parenterale Applikation zubereitet werden. Die antimikrobiellen Zusammensetzungen sind jedoch nur schwer in industriellem Maßstab herzustellen.

Die amerikanische Patentanmeldung US 2009/0320849 A1 offenbart eine Gesichtsmaske, die ein Filtermaterial aus einem faserförmigen Substrat enthält. Dessen Fasern enthalten auf ihrer Oberfläche insbesondere ein Vlies aus Polypropylen oder Polyester, das ein saures Polymeren insbesondere vom Polycarbonsäure-Typ enthält. Die Gesichtsmaske hat eine antivirale Wirkung gegen eingeatmete oder ausgeatmete Luft. Da aber die Polycarbonsäuren wie Polyacrylsäure wasserlöslich sind, können sie durch wässrige Aerosole korrodiert werden

Die amerikanische Patentanmeldung US 2012/0016055 A1 offenbart biozide Beschichtungszusammensetzungen, die ein Biozid und nicht-ionische Polymere und Lösemittel enthalten. Die Beschichtungszusammensetzungen bilden klare und nicht klebrige Filme und Oberflächen, die aber wegen ihrer Löslichkeit leicht entfernt werden können.

Die amerikanische Patentanmeldung US 2013/0344122 A1 offenbart medizinische Artikel mit antimikrobiellen Eigenschaften und guten Barriereeigenschaften. Die medizinischen Artikel enthalten Vliesstoffe aus Polypropylen und eine Beschichtung die Chlorhexidinacetat und Trichlosan enthält. Diese Pharmazeutika werden beispielsweise in Ethanol gelöst und auf das Gewebe gesprüht, bis dieses gleichmäßig gesättigt ist. Danach werden die Stoffproben getrocknet. Die medizinischen Artikel können Kleider, Überschuhe, Abdecktücher, Wickeltücher, Mützen, Laborkittel und Gesichtsmasken sein. Der Nachteil dieser medizinischen Artikel ist, dass die Pharmazeutika nicht fest an die Fasern des Vliesmaterials gebunden sind und leicht davon als Staub entfernt oder durch Lösemittel ausgewaschen werden können.

Die internationale Patentanmeldung WO 2014/149321 A1 offenbart eine Beschichtung mit einer reaktiven Oberfläche, die desinfizierende und biozide Eigenschaften hat. Die reaktiven Zus oder ammensetzungen sind erneuerbar oder "wieder aufladbar" durch die erneute Applikation der aktiven Komponente und müssen nicht entfernt, entsorgt oder ersetzt werden. Die reaktive Zusammensetzung enthält einen hygroskopischen Polymerfilm wie beispielsweise vernetztes Polyvinylpyrrolidon, das mit einem flüssigen oder gasförmigen Oxidationsmittel wie Wasserstoffperoxid, Chlor, Peressigsäure, lod oder Mischungen hiervon so lange behandelt worden ist, dass das Oxidationsmittel mit dem Polymerfilm reagiert hat oder darin absorbiert ist. Die Nachteile dieser reaktiven Oberflächenbeschichtungen sind, dass sie mit toxischen und korrosiven oder gasförmigen Oxidationsmittel aktiviert werden müssen, wobei diese Oxidationsmittel wieder von den Beschichtungen abgegeben werden.

Die amerikanische Patentanmeldung US 2014/0127517 A1 offenbart Filme von linearen oder verzweigten Polyethylenminen mit antiviralen Eigenschaften. Diese Filme sind kovalent an Oberflächen gebunden und mit hydrophoben Seitenketten quaternisiert und mit aktinischer Strahlung vernetzbaren Gruppen modifiziert. Die Nachteile dieser Filme sind, dass die Polethylenimine durch polymeranaloge Reaktionen modifiziert werden müssen. Nach ihrer Applikation auf Oberflächen müssen sie mit UV-Licht bestrahlt werden. Wenn Sie jedoch auf Vliesmaterialien appliziert werden, kann nicht sichergestellt werden, dass das gesamte modifizierte Polyethylenimin von der UV-Strahlung erreicht und vernetzt wird.

Die internationale Patentanmeldung WO 2016/116259 A1 offenbart biozide Materialien, die eine organische Polymermatrix oder eine anorganische Keramikmatrix enthalten, worin biozide Polyoxometallate inhomogen verteilt sind. So kann die Konzentration der Polyoxometalate an der Oberfläche der Matrices höher als in deren Inneren sein.

Die amerikanische Patentanmeldung 2017/0275472 A1 offenbart antimikrobielle Beschichtungsmaterialien für die Oberflächenbeschichtung, die (i) Biozide wie Chlordioxid, Wasserstoffperoxid, Peroxysäuren, Alkohole, essenzielle Öle, antimikrobielle Bestandteile von essenziellen Ölen, Bleichmittel, Antibiotika, Phytochemikalien und Mischungen hiervon, (ii) anorganisch-organische Hohlkörper, die für Biozide durchlässig sind, wobei die anorganischen Materialien Metalloxide, Metallkomplexe, Metallsalze, Metallpartikel und Gemische hiervon sind und die organischen Materialien nicht-ionische Polymere wie Polyethylenglycol oder Polyvinylpyrrolidon sind. Es wird angenommen, dass das antimikrobielle Beschichtungsmaterial eine dauerhafte und vielseitige antimikrobielle Wirkung bei hohen Temperaturen durch die Abtötung durch Kontakt, durch Freisetzung, durch Antihaftwirkung und durch Selbstreinigung hat. Der Nachteil ist, dass flüchtige Biozide verwendet werden, die permanent von den Beschichtungsmaterialien freigesetzt werden.

Das amerikanische Patent 10,227,495 B2 beansprucht biozide Biopolymerbeschichtungen aus vernetzten funktionalisierten Triglyceriden und kovalent gebundenen quaternären Ammoniumverbindungen. Die Vernetzung kann durch Bestrahlung mit aktinischem Licht oder durch Polyisocyanate erfolgen. Nachteilig ist, dass die biozide Wirkung auf die Verwendung einer Klasse von Verbindungen, nämlich quaternäre Ammoniumverbindungen, beschränkt ist.

Seit dem Beginn der SARS-Co-V2 Pandemie sind zahlreiche Versuche zur Entwicklung neuer Methoden und Materialien zur Verhinderung der Ausbreitung des Virus gemacht worden.

So beschreiben A. J. Galante et al. vom Department of Industrial Engineering, University of Pittsburgh, and The Department of Ophthalmology, Charles T. Campbell Laboratory of Ophthalmic Microbiology, University of Pittsburgh, School of Medicine, superhemophobe Anti-Virofouling-Beschichtungen für medizinische Kleidung (siehe auch: SpecialChem, The material selection platform, Coating Ingredients, "Researchers Create New Washable, Textile, Coating the Can Repel Viruses.", Published on 2020-05-26"; und "New coating could improve medical gear by making the coronavirus slide right off."; https://www.zmescience.com/science/newsscience/coating-personal-protection-equipment-252342/). Die widerstandsfähigen, mehrschichtigen Beschichtungen werden durch Sintern von Polytetrafluoroethylen(PTFE)-Nanopartikeln in einem Lösemittel auf Polypropylen-Mikrofasern hergestellt. Nachteilig ist, dass ihre Herstellung viel Energie und Lösemittel sowie teures PTFE verbraucht. Außerdem töten sie nicht die Viren ab.

Forscher von der Ben Gurion University, Israel, haben Beschichtungen auf Basis von Nanopartikeln entwickelt, um die Verbreitung des Coronavirus zu verhindern. Sie haben herausgefunden, dass Kupfernanopartikel in dieser Hinsicht am effektivsten sind. Die antiviralen Beschichtungen können auf Oberflächen gepinselt oder gesprüht werden. Es können übliche und bekannte Polymere, die Nanopartikel von Kupfer enthalten, verwendet werden. Die Nanopartikel ermöglichen die kontrollierte Freisetzung von Metallionen auf die beschichteten Oberflächen (siehe auch SpecialChem, The material selection platform, Coating Ingredients, published on 2020-05-21). Die Kupfernanopartikel und die Kupferionen sind aber nicht nur für Mikroorganismen und Viren toxisch, sondern auch für höhere Tiere und Menschen.

Bio-Fence, Inc., Israel, hat neue antimikrobielle Beschichtungen entwickelt, die einen dauerhaften Schutz gegen Coronaviren bieten sollen. Offenbar enthalten die Beschichtungen ein Polymer, das aktives Chlor umfasst, dass durch Hydrochlorit-Lösungen wiederaufgefrischt werden kann (siehe SpecialChem, The material selection platform, Coating Ingredients, published on 2020-05-12). Der Nachteil dieser Beschichtungen ist die Verwendung von korrosivem Hypochlorit und aktivem Chlor, das vermutlich an Stickstoffatome in der Form von >N-CI Gruppen gebunden ist. Somit sind diese Materialien toxisch und haben einen intensiven unangenehmen Geruch.

Am 30.6.2020 veröffentlichte die SpecialChem website eine Notiz betreffend "New Hybrid Coatings to Protect Interior Walls from Microbial Contamination" auf der Basis von Polyacrylaten, die als Comonomer 3-(Methacrylamino)propyltrimethylammoniumchlorid enthalten. Die seitenständigen quaternären Ammoniumgruppen fungieren als biozide Zentren:
https://phvsicsworld.com/a/cellular-nanosponqes-could-neutralize-sars-cov-2/?utm medium=email&utm source=iop&utm term=&utm campaiqn=14258-46562&utm content=Title%3A%20Cellular%20nanosponges%20could%20neutralize%20SARS -CoV-2%20%20-%20research update&Campaign+Owner=

Ein anderer Ansatz wird an der Concordia University, Canada, und dem Canada-wide research network based at Concordia, verfolgt. Dabei handelt es sich um Kupfer- und Titandioxid-Spritzlacke, die die Verbreitung von Covid 19 verhindern sollen:
https://www.conordia.ca/content/shared/en/news/stories/2020/05/28/a-canada-wide-research-network-based-at-concordia-is-readv-to-make-work-surfeces-safer-for-frontline-staff.html

Noch ein weiterer Ansatz wird von der Firma TriOptoTec von Forschern der Universitätskliniken der Universität Regensburg, Deutschland, verfolgt. (Siehe "SpecialChem" 29.6.2020:
https://coatings.specialchem.com/news/industry-news/sieawerk-to-distribute-varcotecantimicrobial-coatino-innovation-000221983?lr=ipc20061570&li=200165733&utm source=NL&utm medium=EML&utm campai qn=ipc20061570&m i=NcWvxMS IgE4uDtFR2SUvnvKpGP6scLXvpSt5MIN3KriGtDbdz%2Bxz VTOAM%2Bqw0%2BV%2B21wdqflu13qOabGieKUHdjkvRbBNp

Der betreffende Lack enthält offenbar Dioctylnatriumsulfosuccinat in Butyldiglycol. Er enthält außerdem 10H-Benzo[G]pteridin-2,4-dion-Derivate (vgl. die amerikanischen Patente US 10,227,348 B2 und US 9,796,715 B2) oder Phenalen-1-on-Derivate (vgl. das amerikanische Patent US 9,302,004 B2) als Fotosensibilisatoren. Der Lack wird vor allem auf Papier oder Karton appliziert. Beim Bestrahlen mit sichtbarem Licht produziert der Fotosensibilisator Singulettsauerstoff, der die Mikroorganismen auf der Oberfläche des Papiers oder des Kartons abtötet. Der Nachteil ist, dass diese Reaktion nur im Licht aber nicht im Schatten stattfindet, sodass zahlreiche Anwendungen ausgeschlossen sind.

Auf dem Gebiet der Oberflächentechnologie ist es allgemein bekannt, dass man einen besonders starken Lotus-Effekt oder einer Superhydrophobie durch ein hierarchisch strukturiertes Oberflächendesign erreichen kann. So offenbart die amerikanische Patentanmeldung US 2014/0238646 A1 eine Methode für die Herstellung von hierarchisch angeordneten Strukturen von nanoskaligen anorganischen Phosphatpartikeln, die homogen auf der Oberfläche von mikrometerskaligen Phyllosilikatpartikeln verteilt sind. Weil diese Oberflächen nicht benetzt werden und kondensierte Feuchtigkeit sofort Tropfen bildet, die von den Oberflächen herunterrollen, ist die Kontaktzeit zu kurz für eine biozide oder viruzide Wirkung.

Aditya Kumar, Kalpita Nath und Poonam Chauhan vom, Department of Chemical Engineering haben eine superhydrophobe antivirale Beschichtung mit selbstreinigenden Eigenschaften auf der Basis von Silbernanopartikeln, die mit UV-Strahlung bestrahlt und anschließend mit Perfluorodecyltriethoxysilan behandelt werden, entwickelt:
https://coatings.specialchem.com/news/industry-news/new-superhydrophobic-antiviral-coatingself-cleaning-properties-000221961?lr=ipc20061569&li=200165733&utm source=NL&utm medium=EML&utm campai qn=ipc20061569&m_i=owCobZpJ7BFfD70L%2BHEhcWDRZ0rH4AKAPPd55vGetHRPb8itAEQ FCfeJRcddTTWGNwEzLArkBh9IQcRenmgXfr87TrjboV
https://www.technicaltextile.net/news/iit-ism-s-silver-nanoparticle-anti-viral-textile-coating-268082.html

(Vgl. auch SpecialChem for Coatings, Industry News, 23. 6. 2020). Wegen der Superhydrophobie sind aber auch hier die vorstehend geschilderten Nachteile zu erwarten.

Noch ein weiterer Ansatz wird von J. Mostaghimi von der Universität Toronto, Kanada verfolgt. Siehe dazu "SpecialChem The material selection platform, 7.9.2020", Twin-wire Arc Spray Technology to Deposit Cu on Fabrics):
https://coatinas.specia!chem.com/news/industry-news/new-wav-cu-coatinas-masks-covid19-transmission-000222593?lr=ipc20091591&li=200165733&utm_source=NL&utm_medium=EML&utm_campai gn=ipc20091591&m_i=LKHowSxEDOQqaf6IRKqtvs82gOFcOeUHDQqfVqznX2JyZxquM0NNd HnP8g95KPhnJ0ofLb9h8b0_4U4K4g4szOnptKr2LD&status=valid
und "Anti-viral copper coatings could help slow the transmission of COVID 19", Department of Mechanical & Industrial Engineering, University of Toronto, lynsev@mie.utoronto.ca; 31.8.2020.

Jinghzi Pu et al. von der School of Science an der IUBUI, Iniana, USA, Maskenfilter entwickelt, der die innere Struktur von Fischkiemen nachahmt. Die Herstellung der komplexen Strukturen erfolgt durch 3-D-Drucken und anschließender Beschichtung der Oberfläche mit Kupfer durch Elektroplattieren. Durch die Erhöhung der Oberfläche, über die die Luft hinwegstreicht, soll sich die biozide Wirkung des Kupfers erhöhen. Die Entwickler spekulieren, dass diese Strukturen auch für Filter für Klimaanlagen in Gebäuden und Flugzeugen geeignet sein könnten (vgl. SpecialChem, Industry News, Researchers Use Cu Coating on Plastic Mask Filters to Reduce Virus Spread, Publ. 17.9.2020:
https://coatings.specialchem.com/news/industry-news/cu-coating-plastic-mask-filters-reducevirus-spread-000222707?lr=ipc20091594&li=200165733&utm_source=NL&utm_medium=EML&utm_campai gn=ipc20091594&m_i=fM1fEM0ZwQ1A6LPH0ipPWKoH2EusD4Rm30xmwWHtDbV2rPu33i6w C0fu0%2Bwv4Rm1vNWcGxaS2K9Fxo WpaHR7r3%2B8aWffp&status=valid
vgl. auch:
https://news.iu.edu/stories/2020/09/iupui/releases/16-copper-coating-3d-printed-plastic-fillers-pandemic-fighter.html).

Es steht indes zu befürchten, dass bei einem hohen Luftdurchsatz mit hohen Strömungsgeschwindigkeiten diese komplexen Strukturen intensive Geräusche wie Rauschen oder Pfeifen erzeugen.

Luftreiniger sind mobile Vorrichtungen zur Reinigung von Luft mithilfe von Filtern. Nach ihrer Abscheidungswirksamkeit können die Filter in
- Hochleistungs-Partikelfilter (EPA = Efficient Particulate Air filter), kleinste filtrierbare Teilchengröße: 100 nm,
- Schwebstofffilter (HEPA = High Efficiency Particulate Air filter), kleinste filtrierbare Teilchengröße: 100 nm,
- Hochleistungs-Schwebstofffilter (ULPA = Ultra Low Penetration Air filter), kleinste filtrierbare Teilchengröße: 50 nm,
- Medium-Filter, kleinste filtrierbare Teilchengröße: 300 nm,
- Vorfilter, kleinste filtrierbare Teilchengröße: 1000 nm, und
- Automobilinnenraumfilter, kleinste filtrierbare Teilchengröße: 500 nm unterteilt werden. Für den Bereich von 1 nm bis 50 nm stehen somit keine Filter zur Verfügung.

Je nach Partikelgröße beruht ihre Filterwirkung auf den folgenden Effekten:
- Diffusionseffekt: Sehr kleine Partikel (Partikelgröße: 50 nm bis 100 nm) folgen nicht dem Gasstrom, sondern haben durch ihre Zusammenstöße mit den Gasmolekülen einer der Brownschen Bewegung ähnliche Flugbahn und stoßen dadurch mit den Filterfasern zusammen, woran sie haften bleiben. Dieser Effekt wird auch als Diffusionscharakteristik (diffusion regime) bezeichnet.
- Sperreffekt: Kleinere Partikel (Partikelgröße: 100 nm bis 500 nm), die dem Gasstrom um die Faser folgen, bleiben haften, wenn sie der Filterphase zu nahekommen. Dieser Effekt wird auch als Abfangcharakteristik (interception regime) bezeichnet.
- Trägheitseffekt: Größere Partikel (Partikelgröße: 500 nm bis >1 µm) folgen nicht dem Gasstrom um die Faser, sondern prallen aufgrund ihrer Trägheit dagegen und bleiben daran haften. Dieser Effekt wird auch als inerte Einschlags- und Abfangcharakteristik (inertial impaction regime) bezeichnet.

In dem Partikelgrößenbereich von 100 nm bis 500 nm treten der Diffusionseffekt und der Sperreffekt gemeinsam auf. In dem Partikelgrößenbereich von 500 nm bis >1 µm treten der Trägheitseffekt und der Sperreffekt ebenfalls gemeinsam auf.

Gemäß den Filtereffekten sind Teilchen einer Teilchengröße von 200 nm bis 400 nm am schwersten abzuscheiden. Sie werden auch als MMPS = most penetrating particle size bezeichnet. Die Filtereffizienz sinkt in diesem Größenbereich auf 50 %. Größere und kleinere Teilchen werden aufgrund ihrer physikalischen Eigenschaften besser abgeschieden.

Man klassifiziert EPA, HEPA und UPLA nach der Effektivität für diese Korngrößen mittels eines Prüfaerosols aus Di-2-ethylhexyl-sebacat (DEHS). K. W. Lee und B. Y. H. Liu geben in ihrem Artikel "On the Minimum Efficiency and the Most Penetrating Particle Size for Fibrous Filters" in Journal of the Air Pollution Control Association, Bd. 30, Nr. 4, April 1980, Seiten 377 bis 381, Formeln an, die es gestatten, die kleinste Effizienz und MMPS für Faserfilter aufgrund des Diffusionseffekts und des Trägheitseffekts zu berechnen. Die Ergebnisse zeigen, dass MMPS mit steigender Filtriergeschwindigkeit und mit steigendem Faservolumenanteil abnimmt und mit zunehmender Fasergröße zunimmt.

Besonders kritisch ist aber auch die Tatsache, dass es für Nanopartikel einer mittleren Teilchengröße d₅₀ von 1 nm bis <50 nm keine Filter gibt. Ausgerechnet diese Teilchen lagern sich leicht in Bronchien und Lungenbläschen ab und haben generell die höchste Mortalität und Toxizität. Sie können daher Krankheiten wie Asthma, Bronchitis, Arteriosklerose, Arrythmie, Dermitis, Autoimmunerkrankungen, Krebs, Morbus Crohn oder Organversagen hervorrufen.

Das ist besonders kritisch, da die Tiefenfilter oder Schwebstofffilter unter anderem im medizinischen Bereich wie Operationsräumen, Intensivstationen und Laboratorien sowie in Reinräumen, in der Kerntechnik und in Luftwäschern eingesetzt werden.

Eine weitere in dieser Hinsicht problematische Technologie sind Elektrofilter für die elektrische Gasreinigung, Elektro-Staubfilter oder Elektrostate, die auf der Abscheidung von Partikeln aus Gasen mittels des elektrostatischen Prinzips beruhen. Die Abscheidung im Elektrofilter kann in fünf getrennten Phasen stattfinden:
1. Freisetzung von elektrischen Ladungen, meist Elektronen,
2. Aufladung der Staubpartikel im elektrischen Feld oder Ionisator,
3. Transport der geladenen Staubteilchen zu der Niederschlagselektrode
4. Anhaftungen der Staubpartikel an der Niederschlagselektrode und
5. Entfernung der Staubschicht von der Niederschlagselektrode.

Es gelingt indes nicht Partikel im Nanometerbereich vollständig abzutrennen, sodass die Gefahr einer Kontamination mit lungengängigen Partikeln in der Umgebung solcher Anlagen besteht.

Diese Elektro-Staubfilter werden häufig in der Abgasaufbereitung eingesetzt. Dabei werden Amine, Kohlendioxid, Ammoniak, Salzsäure, Schwefelwasserstoff und andere giftige Gase mithilfe von Membranen dem Abgasstrom entzogen. Da die Elektro-Staufilter die feinsten Partikel nicht vollständig entfernen können, schädigen diese die Membranen und erniedrigen deren Trennleistung.

Zu Einzelheiten betreffend die Toxikologie wird auf die Übersichtsartikel von Günter Oberdörster, Eva Oberdörster und Jan Oberdörster, "Nanotoxicology. An Emerging Discipline Evolving from Studies of Ultrafine Particles", in Environmental Health Perpectives Volume 113. (7), 2005, 823-839, und Günter Oberdörster, Vicki Stone und Ken Donaldson, "Toxicology of nanoparticles: A historical perspective", Nanotoxicology, March 2007; 1(1): 2-25, verwiesen.

Außerhalb von Zellen vorliegende Viren werden wissenschaftlich als Virionen bezeichnet. Sie haben einen Durchmesser von 15 nm bis 440 nm und sind deutlich kleiner als Bakterien, von denen die meistens einen Durchmesser von 1 µm bis 5 µm haben. Die Viren oder Virionen weisen somit Größen auf, die in die "Filterlücken" von 1 nm bis 50 nm und von 200 nm bis 400 nm fallen.

Daher können die mit Filtern ausgestatteten Luftreiniger bestenfalls die Konzentration von Viren oder Virionen in der Raumluft senken, aber sie können sie nicht vollständig entfernen oder vernichten, weil eine desinfizierende Wirkung fehlt.

Durch Menschen und Tiere erzeugte, nicht sedimentierende Aerosole, insbesondere Aerosole, die durch Atmen, Husten oder Niesen entstehen und sich in geschlossenen Räumen sehr rasch in großen Volumina verteilen, spielen eine zentrale Rolle für die Übertragung von Viren von Mensch zu Mensch, von Tier zu Mensch, von Tier zu Tier und von Mensch zu Tier. Sie tragen wesentlich zur Verbreitung von Krankheiten bei. Da die nicht sedimentierenden Aerosole im Allgemeinen einen Teilchendurchmesser von 0,1 nm bis 100 nm haben, lassen sie sich - wenn überhaupt - nur unvollständig durch die Luftfilter abfangen.

Will man daher die Konzentration von Viren und Virionen in der Luft in geschlossenen Räumen unterhalb einer Schwelle halten, ab der eine hochgradige Infektionsgefahr besteht, muss die Luft permanent in großen Mengen umgewälzt werden. Dies funktioniert aber derzeit nur mit stationären Klimaanlagen, die aber häufig nicht nachträglich in Gebäude, Transportmittel usw. eingebaut werden können. Ein weiterer Nachteil ist, dass leistungsstarke Klimaanlagen, Gebläse und mobile Luftreiniger häufig laut rauschen, was als störend empfunden wird. Ihre besonders starken Luftströme verursachen häufig gesundheitliche Probleme wie Erkältungen und Gelenkschmerzen.

In der Firmenschrift von "LUFTREINIGERDEPOT Ihr Spezialist für gesunde Raumluft", https://www.luftreinigerdepot.de/gegen/bakterien-und-viren?p=1&o=2&n=20&f=784
heruntergeladen am 25.8.2020 werden noch einmal die Probleme verdeutlicht (Originalzitat Anfang):

***"Luftreiniger gegen Viren* & *Bakterien* -** ***auch gegen den Coronavirus?** Ein Luftreiniger gegen **Bakterien und Viren** ist besonders sinnvoll für Warteräume von **Arztpraxen**, für **Büros** oder Pakete, für andere **öffentliche Räume** wie **Kantinen**, **Friseursalons** oder **Nagelstudios**. Überall dort, wo Menschen zusammenkommen und die Luft mehr oder weniger steht, steigt nämlich die Ansteckungsgefahr, die durch den Einsatz von Luftreinigern gesenkt werden kann. Ob Luftreiniger auch konkret gegen den Covid 19 Coronavirus wirken, ist aufgrund dessen, dass es Ihn noch nicht lange gibt noch nicht getestet worden. Da es kein komplett neuer Virus ist, sondern eine mutierte Form bereits bekannter Viren, spricht jedoch sehr viel für eine Wirksamkeit.*

*Welche Luftreiniger besonders gut gegen Bakterien und Viren geeignet sind, erfahren Sie weiter unten.*

### WICHTIGER HINWEIS:

***Bitte beachten Sie, dass Luftreiniger zwar die Konzentration von Viren und Bakterien in der Luft erheblich reduzieren, jedoch nicht komplett verhindern können. Der beste Schutz vor dem Coronavirus ist die Meidung sozialer Kontakte sowie häufiges und gründliches Händewaschen. Bitte leisten Sie in jedem Fall den Auflagen der Bundes- und Landesregierungen folge.***

*"Aufgrund der geringen Größe von Bakterien und Viren müssen Luftreiniger über die richtigen Filter verfügen, um schwebende Gefahren aus der Luft sicher einfangen zu können. Luftreiniger mit **HEPA-Filter** arbeiten sehr effektiv gegen mikroskopisch kleine Infektionsherde und filtern auch besonders winzige Bakterien mit einer Partikelgröße von nur 0,3 Mikrometer (µm) sicher aus der Raumluft. Zusätzliche Methoden wie **photokatalytische Filter, Nano-Silber-Filter** oder zugeschaltete **Ionisatoren** können dabei helfen, den Wirkungsgrad von Luftreinigern noch weiter zu verbessern. Um Bakterien und Viren möglichst schnell in die verfügbaren Filter einzufangen ist auch ein **hoher Luftdurchsatz** bei Luftreinigern wichtig. Ein Luftreiniger sollte in der Lage sein, die komplette Raumluft **mindestens zwei Mal pro Stunde** zu reinigen. Hersteller von Premium Geräten visieren eine komplette Reinigung der Raumluft bis zu 5 Mal pro Stunde an.*"

### (Originalzitat Ende).

Eine weitere Möglichkeit, die Konzentration von Virionen und Aerosolen in geschlossenen Räumen zu reduzieren, ist eine intensive Stoßlüftung durch Außenluft. Dies setzt zum einen voraus, dass die Räume auf der Außenseite von Gebäuden liegen, damit solche Lüftungsmöglichkeiten überhaupt vorhanden sind und wenn ja, dass die Wetterbedingungen eine Lüftung gestatten. Dies dürfte bei tiefen Außentemperaturen, Schlagregen, Gewitter, Hagel, Schnee, Eisregen usw. schwierig werden.

### Aufgabe der vorliegenden Erfindung

Der vorliegenden Erfindung lag die Aufgabe zu Grunde, eine geräuscharme oder im Idealfall geräuschlose, mobile Vorrichtung zur Reinigung und Desinfizierung von Raumluft vorzuschlagen, die die Nachteile des Standes der Technik nicht mehr länger aufweist, sondern die es gestattet, kontaminierte Raumluft von den unterschiedlichsten Noxen und Mikroorganismen dauerhaft zu befreien. Insbesondere soll die mobile Vorrichtung Bakterien und/oder Viren sowie durch Bakterien und/oder Viren kontaminierte Aerosole, speziell SARS-Co-V2-Viren und durch SARS-Co-V2-Virenzu kontaminierte Aerosole, eliminieren und die dabei resultierenden Zerfallsprodukte absorbieren und/oder adsorbieren.

Dadurch soll auch erreicht werden, dass das Luftvolumen, das umgewälzt werden muss, um eine nachhaltige Wirkung zu erzielen, signifikant verringert werden kann. Dadurch soll auch der Energieverbrauch nachhaltig verringert werden. Insgesamt soll eine optimale Balance zwischen dem umzuwälzenden Luftvolumen, der Kontaktzeit der Viren und Bakterien mit den Bioziden und der Größe der Vorrichtungen erzielt werden.

Des Weiteren sollen die Vorrichtungen in einfacher Weise herstellbar sein und nur bakterizide und viruzide Substanzen enthalten, die behördlich zu diesen Zwecken zugelassen sind.

Darüber hinaus sollen die Vorrichtungen keine Stäube, Dämpfe oder Aerosole in die gereinigte und desinfizierte Raumluft abgeben.

### Die erfindungsgemäße Lösung

Demgemäß wurde die durch Temperaturdifferenz betreibbare, mobile Vorrichtung zur Reinigung und Desinfizierung von Raumluft gemäß dem unabhängigen Patentanspruch 1 gefunden, die nachstehend als »erfindungsgemäße Vorrichtung« bezeichnet wird. Vorteilhafte Ausführungsformen der erfindungsgemäßen Vorrichtung sind Gegenstand der abhängigen Patentansprüche 2 bis 23.

Außerdem wurde das Verfahren zur Reinigung und Desinfizierung von Raumluft mithilfe der erfindungsgemäßen Vorrichtung gemäß dem Patentanspruch 24 gefunden. Das Verfahren wird im Folgenden als »erfindungsgemäßes Verfahren« bezeichnet. Vorteilhafte Ausführungsformen des erfindungsgemäßen Verfahrens sind Gegenstand der abhängigen Patentansprüche 25 bis 28.

Ferner wurde die Verwendung der erfindungsgemäßen Vorrichtung und des erfindungsgemäßen Verfahrens zur Eliminierung von freien oder an Aerosole gebundenen Bakterien, Viren und anderen Noxen in der Luft gemäß dem unabhängigen Patentanspruch 29 gefunden. Im Folgenden wird diese Verwendung als »erfindungsgemäße Verwendung« bezeichnet.

### Vorteile der Erfindung

Im Hinblick auf den Stand der Technik war es überraschend und für den Fachmann nicht vorhersehbar, dass die Aufgabe, die der vorliegenden Erfindung zu Grunde lag, mithilfe der erfindungsgemäßen Vorrichtung, des erfindungsgemäßen Verfahrens und der erfindungsgemäßen Verwendung gelöst werden konnte.

Insbesondere überraschte, dass die erfindungsgemäße Vorrichtung die Nachteile des Standes der Technik nicht mehr länger aufwies, sondern es gestattete, kontaminierte Raumluft von den unterschiedlichsten Noxen und Mikroorganismen dauerhaft zu befreien. Insbesondere konnten mithilfe der erfindungsgemäßen Vorrichtung und des erfindungsgemäßen Verfahrens Bakterien und Viren sowie durch Bakterien und/oder Viren kontaminierte Aerosole, speziell SARS-Co-V2-Viren und durch SARS-Co-V2-Virenzu kontaminierte Aerosole, durch Kontakt eliminiert und die dabei resultierenden Zerfallsprodukte absorbiert und/oder adsorbiert werden.

Dadurch konnte auch erreicht werden, dass das Luftvolumen, das umgewälzt werden musste, um eine nachhaltige Wirkung zu erzielen, signifikant verringert werden konnte. Dadurch wurde auch der Energieverbrauch nachhaltig gesenkt. Insgesamt wurde eine optimale Balance zwischen dem umzuwälzenden Luftvolumen, der Kontaktzeit der Viren und Bakterien mit den Bioziden und der Größe der Vorrichtungen erzielt.

Des Weiteren konnte die erfindungsgemäße Vorrichtung in einfacher Weise hergestellt und automatisch betrieben werden und enthielt nur bakterizide und viruzide Substanzen, die auch behördlich zu diesen Zwecken zugelassen sind. Außerdem konnten sämtliche Bauteile der erfindungsgemäßen Vorrichtung gleichfalls mit einer Beschichtung mit permanenter viruzider und bakterizider Wirkung versehen werden, was die Gesamtwirkung in vorteilhafter Weise weiter steigerte.

Darüber gab die erfindungsgemäße Vorrichtung keine Stäube, Dämpfe oder Aerosole in die gereinigte und desinfizierte Raumluft ab.

Die erfindungsgemäße Vorrichtung und das erfindungsgemäße Verfahren konnten in den unterschiedlichsten geschlossenen Räumen angewandt werden, wobei sich ihre Mobilität als weiterer besonderer Vorteil erwies.

Ein weiterer wesentlicher Vorteil der erfindungsgemäßen Vorrichtung war, dass verbrauchte viruzide und/oder bakterizide Schüttungen und die Adsorbentien und Adsorbentien der erfindungsgemäß zu verwendenden Kartusche in einfacher Weise direkt durch mechanochemische Verfahren, bei denen schädliche organische Abfallprodukte in Kohlenstoff umgewandelt wurden, dekontaminiert werden konnten.

Wegen ihren im Vergleich zu Lüftern mit Filtern oder elektrostatischen Lüftern deutlich geringeren Abmessungen und Platzbedarf sind die erfindungsgemäßen Vorrichtungen besonders hervorragend dazu geeignet, die Raumluft auch in den Ecken von Räumen geräuschlos oder nahezu geräuschlos zu reinigen und zu dekontaminieren. Deshalb können sie auch besonders gut in Klassenräumen oder Konferenzräumen verwendet werden, worin stark rauschende Lüfter besonders störend sind.

Weitere Vorteile der Erfindung gehen aus der nachfolgenden Beschreibung hervor.

### Ausführliche Beschreibung der Erfindung

Die erfindungsgemäße Vorrichtung dient der Reinigung und Desinfizierung von Raumluft in Räumen aller Art. Insbesondere dient sie der Eliminierung von freien oder an Aerosole gebundenen Bakterien, Viren und anderen Noxen in der Luft von Wohnräumen, Krankenzimmern, Operationssälen, Behandlungsräumen in Arztpraxen und physiotherapeutischen Einrichtungen, Laboratorien aller Art, Gaststätten, Restaurants, Bistros, Hotelzimmern, Klassenzimmern, Unterrichtsräumen, Fitnesscentern, Zügen, Autos, Bussen, Taxis, Wohnwagen, Wohnmobilen, Campingzelten, Flugzeugen, Schiffskabinen, Büros, Konferenzräumen, Versammlungsräumen, Theatern, Kinos, Schiffsterminals, Bahnhöfen, Flughafenterminals, Aufzügen, Werkstätten, Fabrikhallen, Treppenhäusern und Geschäften aller Art.

Die erfindungsgemäße Vorrichtung umfasst die nachstehend beschriebenen Bauteile.

In einer vorteilhaften Ausführungsform der erfindungsgemäßen Vorrichtung sind die Oberflächen der Bauteile, insbesondere die Oberflächen, die mit Luft in Kontakt kommen, mit mindestens einer der nachfolgend beschriebenen bakteriziden und/oder viruziden Beschichtungen versehen.

Die erfindungsgemäße Vorrichtung weist eine vertikale, rohrförmige Außenwand mit einem Bodenbereich mit einem horizontalen Standboden und eine obere Öffnung am oberen Ende auf.

In Bodenbereich befinden sich mindestens ein, insbesondere ein, Raum für den Einschub mindestens eines wieder aufladbaren Akkumulators und eines elektronischen Steuergeräts zu Regelung des durch den Kamineffekt erzeugten Luftstroms durch die nachstehend beschriebene Kartusche.

Der Durchmesser der rohrförmigen Außenwand und damit der erfindungsgemäßen Vorrichtung kann breit variieren und richtet sich in erster Linie nach dem Luftvolumen, das dekontaminiert werden soll. Vorzugsweise liegt der Durchmesser bei 5 cm bis 50 cm. Bevorzugt ist die Wandstärke 0,2 cm bis 2 cm, besonders bevorzugt 0,3 cm bis 1,5 cm und insbesondere 0,5 cm bis 1 cm. Insbesondere liegt die lichte Weite zur Aufnahme der Bauteile in der rohrförmigen Außenwand zwischen 4,6 cm und 49,6 cm.

Der Höhe der rohrförmigen Außenwand und damit der erfindungsgemäßen Vorrichtung kann ebenfalls breit variieren und richtet sich in erster Linie nach dem Luftvolumen, das dekontaminiert werden soll. Vorzugsweise ist die Höhe 30 cm bis 300 cm, bevorzugt 50 cm bis 300 cm und insbesondere 60 cm bis 300 cm.

Der Umriss der rohrförmigen Außenwand ist vorzugsweise viereckig, sechseckig oder achteckig. Die Verbindungslinien zwischen den Ecken können geradlinig oder konvex nach außen gebogen sein. Bevorzugt sind bei einem viereckigen Umriss zwei einander gegenüberliegende Flächen geradlinig, wogegen die beiden anderen gegenüberliegenden Flächen geradlinig oder konvex nach außen gebogen sein können. Bevorzugt liegen bei einem sechseckigen Umriss drei geradlinige Flächen vor, die durch geradlinige oder konvex nach außen gebogene Flächen verbunden sind. Bevorzugt sind bei einem achteckigen Umriss vier einander gegenüberliegende Flächen geradlinig wogegen die anderen vier verbindenden Flächen geradlinig oder konvex nach außen gebogen sein können.

Die Außenwand kann aus den unterschiedlichsten Materialien aufgebaut sein. Vorzugsweise haben diese Materialien eine niedrige Wärmeleitfähigkeit λ. Vorzugsweise liegt die Wärmeleitfähigkeit λ unterhalb von 80 W/(m. K). Beispiele geeigneter Materialien sind Kunststoffe, Gläser, Harthölzer und Metalle sowie Verbundmaterialien hiervon.

Geeignete Kunststoffe sind übliche und bekannte lineare und/oder verzweigte und/oder blockartig, kammartig und/oder statistisch aufgebaute Polyadditionsharze, Polykondensationsharze und/oder (Co)Polymerisate von ethylenisch ungesättigten Monomeren in Betracht.

Beispiele geeigneter (Co)Polymerisate sind (Meth)Acrylat(co)polymerisate und/oder Polystyrol, Polyvinylester, Polyvinylether, Polyvinylhalogenide, Polyvinylamide, Polyacrylnitrile Polyethylene, Polypropylene, Polybutylene, Polyisoprene und/oder deren Copolymerisate.

Beispiele geeigneter Polyadditionsharze oder Polykondensationsharze sind Polyester, Alkyde, Polylactone, Polycarbonate, Polyether, Proteine, Epoxidharz-Amin-Addukte, Polyurethane, Alkydharze Polysiloxane, Phenol-Formaldehyd-Harze, Harnstoff-Formaldehyd-Harze, MelaminFormaldehyd-Harze, Cellulose, Polysulfide, Polyacetale, Polyethylenoxide, Polycaprolactame, Polylactone, Polylactide, Polyimide, und/ oder Polyharnstoffe.

Bekanntermaßen werden die Duroplaste aus mehrfach funktionellen, niedermolekularen und/oder oligomeren Verbindungen durch thermisch und/oder mit aktinischer Strahlung initiierte (Co)Polymerisation hergestellt.

Die Kunststoffe können übliche und bekannte Verstärkungsfasern und Füllstoffe enthalten, sofern Letztere nicht die Wärmeleitfähigkeit λ über 80 W/(m.K) erhöhen.

Beispiele geeigneter Metalle sind Chromstahl, hochlegierter Stahl, niedriglegierter ferritischer Stahl, unlegierter Stahl und Titan.

Beispiele geeigneter Harthölzer sind Buchenholz, Eichenholz und Eschenholz.

Beispiele geeigneter Gläser werden in Römpp-Online unter den Stichworten »Glas«, »Hartglas« oder »Sicherheitsglas« oder in der deutschen Übersetzung des europäischen Patents EP 0 847 965 B1 mit dem Aktenzeichen DE 697 312 168 T2, Seite 8, Absatz [0053], beschrieben. Beispiele besonders gut geeigneter Gläser sind nicht vorgespanntes, teilvorgespanntes und vorgespanntes Floatglas, Gussglas oder Keramikglas.

Die vertikale, rohrförmige Außenwand weist mindestens eine umlaufende Trennstelle und vorzugsweise zwei umlaufende Trennstellen auf, an der oder an denen die Außenwand auseinandergenommen und wieder zusammengefügt werden kann. Bei diesen Trennstellen handelt es sich insbesondere um Steckverbindungen oder Flanschverbindungen. Die Trennstellen erleichtern den Zusammenbau und die Wartung der erfindungsgemäßen Vorrichtung.

Bevorzugt haben die Peltier-Elemente einen kreisrunden, quadratischen oder rechteckigen Umriss.

Die Anzahl, die Größe, die Form, die Anzahl der Stufen (einstufig oder mehrstufig) und die Leistungsabgabe der Peltier-Elemente kann breit variieren und richtet sich in erster Linie nach dem gewünschten Temperaturunterschied zwischen den heißen und den kalten Seiten sowie der Luftmenge, die während einer Zeiteinheit umgesetzt werden soll, und damit nach der Größe der erfindungsgemäßen Vorrichtung. Der Fachmann kann anhand dieser Vorgaben geeignete, im Handel erhältliche Peltier-Elemente leicht auswählen.

Die heißen Seiten der Peltier-Elemente stehen in wärmeleitendem Kontakt zu mindestens zwei, insbesondere vier, vertikalen Wärmeleitungen von hoher Wärmeleitfähigkeit λ. Der Endbereiche stehen wiederum in wärmeleitendem Kontakt mit der auf dem Bodenbereich angeordneten horizontalen Heizplatte, die von den heißen Seiten der vertikalen Peltier-Elemente über die vertikalen Wärmeleitungen aufgeheizt wird.

Vorzugsweise haben die mindestens zwei, insbesondere mindestens vier, Wärmeleitungen Kontaktflächen, die so groß wie die heißen Seiten der vertikalen Peltier-Elemente sind. Unterhalb der Kontaktflächen können die Wärmeleitungen geringere Breiten haben. Vorzugsweise sind die Wärmeleitungen 0,2 cm bis 1 cm, bevorzugt 0,3 cm bis 0,8 cm und insbesondere 0,13 m bis 0,7 cm dick. Ihre Länge richtet sich im Grunde nach den Abmessungen der erfindungsgemäßen Vorrichtung und insbesondere nach der Höhe des Heizraums.

Bevorzugt haben der umlaufende Kühlring, die Wärmeleitungen und die Heizplatten eine Wärmeleitfähigkeit λ von 20 W/(m.K) bis 430 W/(m.K). Die Bauteile können aus den verschiedensten festen Materialien aufgebaut sein, solange diese die gewünschte Wärmeleitfähigkeit haben. Beispiele geeigneter Materialien dieser Art sind niedriglegierter ferritischer Stahl, Chromstahl, unlegierter Stahl, Blei, Titan, Tantal, Zinn, Platin, Eisen, Nickel, Wolfram, Zink, Messing, Molybdän, Aluminium Aluminiumlegierungen, Gold, Kupferlegierungen, Kupfer und Silber.

Oberhalb des oberen Niveaus der Heizplatte befinden sich mindestens zwei, vorzugsweise mindestens vier, einander gegenüberliegender Lufteinlässe in der Form von Luftrohren durch die Außenwand und durch die Wärmedämbeschichtung. An die Lufteinlässe können an ihren Einlassöffnungen jeweils ein Gitter oder einen Vorfilter angebracht werden, mit deren Hilfe vermieden wird, dass größere Teile in den Heizraum eindringen.

Die mindestens zwei, bevorzugt die mindestens vier und insbesondere mindestens acht Luftrohre können verschiedene Querschnitte aufweisen. So kann der Querschnitt kreisförmig, elliptisch, oval, rechteckig, quadratisch oder spaltenförmig sein. Die mindestens zwei Luftrohre können geradlinig oder gebogen verlaufen, sodass der Lufteintritt in den Heizraum tangential erfolgt, wodurch in der einströmenden Luft Wirbel gebildet werden, die die Wärmeübertragung fördern. Dabei kann sich die lichte Weite des Querschnitts von dem Einlass bis dem Auslass hin trichterförmig oder hornartig erweitern. Zur besseren Luftlenkung zum Einlass der mindestens zwei Luftrohre hin kann die Außenwand jeweils eine entsprechend geformte Luftleitfurche aufweisen. Die Wandung der mindestens zwei Luftrohre kann aus den unterschiedlichsten Materialien aufgebaut sein, wobei vorzugsweise die vorstehend beschriebenen Materialien mit niedriger Wärmeleitfähigkeit in Betracht kommen.

Die horizontalen Peltier-Elemente sind seitlich, d. h. bis auf die Kontaktflächen mit den Wärmeleitungen und dem Kühlring, mit einer Wärmedämmbeschichtung umgeben. Ebenso sind die vertikalen Wärmeleitungen bis auf die Kontaktflächen mit der horizontalen Heizplatte mit einer Wärmedämmbeschichtung umhüllt. Die verbleibende freie vertikale umlaufende Oberfläche der Heizplatte sowie ihr Boden sind ebenfalls mit einer Wärmedämmbeschichtung versehen. Die Innenfläche des Außenrohrs weist ebenfalls eine Wärmedämmbeschichtund auf.

Vorzugsweise hat die Wärmedämmbeschichtung eine Wärmeleitfähigkeit λ von 0,001 W/(m.K) bis 1,0 W/(m.K).

Vorzugsweise werden die Wärmedämmbeschichtungen aus Dämmfarben, ausgewählt aus der Gruppe bestehend aus Korkfarben, diffusionsoffenen Dämmfarben auf Silikonbasis, Dämmfarben mit einem hohen Anteil an keramischen Hohlkugeln, Dämmfarben mit Aerogelen, Schaumzement und Dämmfarben mit mikrofeinen Hohlglaskugeln, hergestellt.

Oberhalb des Heizraums über der Heizplatte ist die vorstehend beschriebene, auswechselbare, oben offene Kartusche mit der thermisch und elektrisch isolierenden Kartuschenwand, dem luftdurchlässigen, siebartigen Kartuschenboden, dessen Öffnungen einerseits so klein sind, dass die nachstehend beschriebenen Schüttungen nicht nach unten herausfallen können, und die andererseits so groß sind, dass der Luftwiderstand gering bleibt, angeordnet.

In der Ausführungsform, in der die Kartusche die Form eines Venturirohrs hat, liegen (i) die Schüttung von mindestens einer Art eines partikulären, anorganisch, bakterizid und viruzid beschichteten, inerten Trägermaterials und/oder die Schüttung von mindestens einer Art eines partikulären, anorganischen und/oder einem partikulären, anorganischen, bakteriziden und/oder viruziden Materials und (ii) die Schüttung aus mindestens einer Art von partikulären, anorganischen und/oder metallorganischen Adsorbentien und/oder Absorbentien als gemischte Schüttung vor.

Dies wird vorteilhafterweise dadurch erreicht, dass an der Innenwand der Kartusche umlaufende Auflagen für luftdurchlässige Zwischenböden angebracht sind, deren Öffnungen einerseits so klein sind, dass die Schüttungen nicht nach unten herausfallen können, und die andererseits so groß sind, dass der Luftwiderstand gering bleibt. Vorzugsweise sind die Zwischenböden aus demselben Material wie die Kartuschenwand aufgebaut.

In der Ausführungsform, in der die Kartusche die Form eines Venturirohrs hat, liegen (i) die Schüttung von mindestens einer Art eines partikulären, anorganisch, bakterizid und viruzid beschichteten, inerten Trägermaterials und/oder die Schüttung von mindestens einer Art eines partikulären, anorganischen und/oder einem partikulären, anorganischen, bakteriziden und/oder viruziden Materials und (ii) die Schüttung von mindestens einer Art von partikulären partikulären, anorganischer Absorbentien und/oder Adsorbentien als gemischte Schüttung vor.

Vorzugsweise wird die mindestens eine Art eines partikulären, anorganischen, bakterizid und/oder viruzid beschichteten, inerten Trägermaterials aus der Gruppe, bestehend aus Füllkörpern für Kolonnen wie Raschigringe, Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen von Schichtsilikaten, Schaumgläsern, Schaumglasschottern Bimssteinen, Zeolithen, Blähtonen, Blähgläsern, Blähglimmern, Blähperliten, Schaumzementen und Keramikschäumen, ausgewählt.

Vorzugsweise wird die mindestens eine Art eines der nachstehend beschriebenen, partikulären, anorganischen, bakteriziden und/oder viruziden Materialien aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen und Schäumen, ausgewählt.

Wegen ihrer strukturellen Besonderheit werden Schichtsilikate bevorzugt verwendet oder zumindest mitverwendet. Die elementare Zusammensetzung und die Struktur der Schichtsilikat-Mikropartikel können sehr weit variieren. Bekannt ist beispielsweise die Einteilung der Silikate in die folgenden Strukturen:
- Inselsilikate
- Gruppensilikate
- Ringsilikate
- Ketten- und Bandsilikate
- Übergangsstrukturen zwischen Ketten- und Schichtsilikaten
- Schichtsilikate
- Gerüstsilikate

Schichtsilikate sind Silikate, deren Silikationen aus Schichten eckenverknüpfter SiO₄- Tetraeder bestehen. Diese Schichten und/oder Doppelschichten sind untereinander nicht weiter verknüpft. Die technisch wichtigen und in Sedimentgestein verbreiteten Tonminerale sind ebenfalls Schichtsilikate. Der schichtartige Aufbau dieser Minerale bestimmt die Form und die Eigenschaften der Kristalle. Sie sind meist tafelig bis blättrig mit guter bis perfekter Spaltbarkeit parallel zu den Schichten. Die Zähligkeit der Ringe, aus denen sich die Silikatschichten zusammensetzen, bestimmt oft die Symmetrie und Form der Kristalle. Zwischen den Schichten können sich Wassermoleküle, große Kationen und/oder Lipide einlagern.

In der nachstehenden Tabelle 1 sind Schichtsilikate beispielhaft und nicht abschließend aufgelistet. Der Fachmann ohne Weiteres kann für den jeweiligen Einzelfall besonders gut geeignete weitere Schichtsilikate auswählen.

**Tabelle 1: Summenformeln von geeigneten Schichtsilikaten ^{a)}**

| Nr. | Typ | Summenformel |
|---|---|---|
| 1 | Martinit | (Na,Ca)₁₁Ca₄(Si,S,B)₁₄B₂O₄₀F₂·4(H₂O) |
| 2 | Apophyllit-(NaF) | NaCa₄Si₈O₂₀F·8H₂O |
| 3 | Apophyllit-(KF) | (K,Na)Ca₄Si₈O₂₀)(F,OH)·8H₂O |
| 4 | Apophyllit-(KOH) | KCa₄Si₈O₂₀(OH,F)·8H₂O |
| 5 | Cuprorivait | CaCuSi₄O₁₀ |
| 6 | Wesselsit | (Sr,Ba)Cu[Si₄O_{10]} |
| 7 | Effenbergerit | BaCu[Si₄O_{10]} |
| 8 | Gillespit | BaFe²⁺Si₄O₁₀ |
| 9 | Sanbornit | BaSi₂O₅ |
| 10 | Bigcreekit | BaSi₂O₅·4H₂O |
| 11 | Davanit | K₂TiSi₆O₁₅ |
| 12 | Dalyit | K₂ZrSi₆O₁₅ |
| 13 | Fenaksit | KNaFe²⁺Si₄O₁₀ |
| 14 | Manaksit | KNaMn²⁺[Si₄O₁₀] |
| 15 | Ershovit | K₃Na₄(Fe,Mn,Ti)₂[Si₈O₂₀)(OH)₄]·4H₂O |
| 16 | Paraershovit | Na₃K₃Fe³⁺₂Si₈O₂₀)(OH)₄·4H₂O |
| 17 | Natrosilit | Na₂Si₂O₅ |
| 18 | Kanemit | NaSi₂O₆·3H₂O |
| 19 | Revdit | Na₁₆Si₁₆O₂₇(OH)₂₆·28H₂O |
| 20 | Latiumit | (Ca,K)₄(Si,Al)₅O₁₁(SO₄,CO₃) |
| 21 | Tuscanit | K(Ca,Na)₆(Si,Al)₁₀O₂₂(SO₄,CO₃,(OH)₂)·H₂O |
| 22 | Carletonit | KNa₄Ca₄Si₈O₁₈(CO₃)₄(OH,F)·H₂O |
| 23 | Pyrophyllit | Al₂Si₄O₁₀(OH)₂ |
| 24 | Ferripyrophyllit | Fe³⁺Si₂O₅(OH) |
| 25 | Macaulayit | (Fe³⁺,Al)₂₄Si₄O₄₃(OH)₂ |
| 26 | Talk | Mg₃Si₄O₁₀(OH)₂ |
| 27 | Minnesotait | Fe²⁺₃Si₄O₁₀(OH)₂ |
| 28 | Willemseit | (Ni,Mg)₃Si₄O₁₀(OH)₂ |
| 29 | Pimelit | Ni₃SiO₁₀(OH)₂·4H₂0 |
| 30 | Kegelit | Pb₄Al₂Si₄O₁₀(SO₄)(CO₃)₂(OH)₄ |
| 31 | Aluminoseladonit | K(Mg,Fe²⁺)Al[(OH)₂\|Si₄O₁₀] |
| 32 | Ferroaluminoseladonit | K(Fe²⁺,Mg)(Al,Fe³⁺)[(OH)₂\|Si₄O₁₀] |
| 33 | Seladonit | K(Mg,Fe²⁺)(Fe³⁺,Al)Si₄O₁₀(OH)2 |
| 34 | Chromseladonit | KMgCr[(OH)₂\|Si₄O₁₀] |
| 35 | Ferroseladonit | K(Fe²⁺,Mg)(Fe³+,Al)[(OH)₂\|Si₄O₁₀] |
| 36 | Paragonit | NaAl₂(Si₃Al)O₁₀(OH)₂ |
| 37 | Boromuskovit | KAl₂(Si₃B)O₁₀(OH,F)₂ |
| 38 | Muskovit | KAl₂(Si₃Al)O₁₀(OH,F)₂ |
| 39 | Chromphyllit | K(Cr,Al)₂[(OH,F)₂\|AlSi₃O₁₀] |
| 40 | Roscoelith | K(V,Al,Mg)₂AlSi₃O₁₀(OH)₂ |
| 41 | Ganterit | (Ba,Na,K)(Al,Mg)₂[(OH,F)₂\|(Al,Si)Si₂O₁₀] |
| 42 | Tobelith | (NH₄,K)Al₂(Si₃Al)O₁₀(OH)₂ |
| 43 | Nanpingit | CsAl₂(Si,Al)₄O₁₀(OH,F)₂ |
| 44 | Polylithionit | KLi₂AlSi₄O₁₀(F,OH)₂ |
| 45 | Tainiolith | KLiMg₂Si₄O₁₀F₂ |
| 46 | Norrishit | KLiMn³⁺₂Si₄O₁₂ |
| 47 | Shirokshinit | KNaMg₂[F₂\|Si₄O₁₀] |
| 48 | Montdorit | KMn_{0.5}²⁺Fe_{1.5}²⁺Mg_{0.5}[F₂\|Si₄O₁₀] |
| 49 | Trilithionit | KLi_{1.5}Al_{1.5}[F₂\|AlSi₃O₁₀] |
| 50 | Masutomilith | K(Li,Al,Mn²⁺)₃(Si,Al)₄O₁₀(F,OH)₂ |
| 51 | Aspidolith-1M | NaMg₃(AlSi₃)O₁₀(OH)₂ |
| 52 | Fluorophlogopit | KMg₃(AlSi₃)O₁₀F₂ |
| 53 | Phlogopit | KMg₃(Si₃Al)O₁₀(F,OH)₂ |
| 54 | Tetraferriphlogopit | KMg₃[(F,OH)₂\|(Al,Fe³⁺)Si₃O₁₀] |
| 55 | Hendricksit | K(Zn,Mn)₃Si₃AlO₁₀(OH)₂ |
| 56 | Shirozulith | K(Mn²⁺,Mg)₃[(OH)₂\|AlSi₃O₁₀] |
| 57 | Fluorannit | KFe₃²⁺[(F,OH)₂\|AlSi₃O₁₀] |
| 58 | Annit | KFe²⁺₃(Si₃Al)O₁₀(OH,F)₂ |
| 59 | Tetraferriannit | KFe²⁺₃(Si₃Fe³⁺)O₁₀(OH)₂ |
| 60 | Ephesit | NaLiAl₂(Al₂Si₂)O₁₀(OH)₂ |
| 61 | Preiswerkit | NaMg₂Al₃Si₂O₁₀(OH)₂ |
| 62 | Eastonit | KMg₂Al[(OH)₂\|Al₂Si₂O₁₀] |
| 63 | Siderophyllit | KFe₂²⁺Al(Al₂Si₂)O₁₀(F,OH)₂ |
| 64 | Anandit | (Ba,K)(Fe²⁺,Mg)₃(Si,Al,Fe)₄O₁₀(S,OH)₂ |
| 65 | Bityit | CaLiAl₂(AlBeSi₂)O₁₀(OH)₂ |
| 66 | Oxykinoshitalith | (Ba,K)(Mg,Fe²⁺,Ti⁴⁺)₃(Si,Al)₄O₁₀O₂ |
| 67 | Kinoshitalith | (Ba,K)(Mg,Mn,Al)₃Si₂Al₂O₁₀(OH)₂ |
| 68 | Ferrokinoshitalith | Ba(Fe²⁺,Mg)₃[(OH,F)₂\|Al₂Si₂O₁₀] |
| 69 | Margarit | CaAl₂(Al₂Si₂)O₁₀(OH)₂ |
| 70 | Chernykhit | BaV₂(Si₂Al₂)O₁₀(OH)₂ |
| 71 | Clintonit | Ca(Mg,Al)₃(Al₃Si)O₁₀(OH)₂ |
| 72 | Wonesit | (Na,K,)(Mg,Fe,Al)₆(Si,Al)₈O₂₀(OH,F)₄ |
| 73 | Brammallit | (Na,H₃O)(Al,Mg,Fe)₂(Si,Al)₄O₁₀[(OH)₂,H₂O] |
| 74 | IIIit | (K,H₃O)Al₂(Si₃Al)O₁₀(H₂O,OH)₂ |
| 75 | Glaukonit | (K,Na)(Fe³⁺,Al,Mg)₂(Si,Al)₄O₁₀(OH)₂ |
| 76 | Agrellit | NaCa₂Si₄O₁₀F |
| 77 | Glagolevit | NaMg₆[(OH,O)₈\|AlSi₃O₁₀]·H₂O |
| 78 | Erlianit | Fe²⁺₄Fe³⁺₂Si₆O₁₅(OH)₈ |
| 79 | Bannisterit | (Ca,K,Na)(Mn²⁺,Fe²⁺,Mg,Zn)₁₀(Si,Al)₁₆O₃₈(OH)₈·nH₂O |
| 80 | Bariumbannisterit | (K,H₃O)(Ba,Ca)(Mn²⁺,Fe²⁺,Mg)₂₁(Si,Al)₃₂O₈₀(O,OH)₁₆·4-12 H₂O |
| 81 | Lennilenapeit | K₆₋₇(Mg,Mn,Fe²⁺,Fe³⁺,Zn)₄₈(Si,Al)₇₂(O,OH)₂₁₆·16H₂O |
| 82 | Stilpnomelan | K(Fe²⁺,Mg,Fe³⁺,Al)₈(Si,Al)₁₂(O,OH)₂₇·2H₂O |
| 83 | Franklinphilit | (K,Na)₁₋ₓ(Mn²⁺,Mg,Zn,Fe³⁺)₈(Si,Al)₁₂(O,OH)₃₆·nH₂O |
| 84 | Parsettensit | (K,Na,Ca)_{7.5}(Mn,Mg)₄₉Si₇₂O₁₆₈(OH)₅₀·nH₂O |
| 85 | Middendorfit | K₃Na₂Mn₅Si₁₂(O,OH)₃₆·2H₂O |
| 86 | Eggletonit | (Na,K,Ca)₂(Mn,Fe)₈(Si,Al)₁₂O₂₉(OH)₇·11H₂O |
| 87 | Ganophyllit | (K,Na)ₓMn²⁺₆(Si,Al)₁₀O₂₄(OH)₄·nH₂O {x = 1-2}{n = 7-11} |
| 88 | Tamait | (Ca,K,Ba,Na)₃₋₄Mn²⁺₂₄[(OH)₁₂\|{(Si,Al)₄(O,OH)₁₀}₁₀]·21H₂O |
| 89 | Ekmanit | (Fe²⁺,Mg,Mn,Fe³⁺)₃(Si,Al)₄O₁₀(OH)₂·2H₂O |
| 90 | Lunijianlait | Li_{0.7}Al_{6.2}(Si₇AlO₂₀)(OH,O)₁₀ |
| 91 | Saliotit | Na_{0.5}Li_{0.5}Al₃[(OH)₅\|AlSi₃O₁₀] |
| 92 | Kulkeit | Na_{0.35}Mg₈Al(AlSi₇)O₂₀(OH)₁₀ |
| 93 | Aliettit | Ca_{0.2}Mg₆(Si,Al)₈O₂₀(OH)₄·4H₂O |
| 94 | Rectorit | (Na,Ca)Al₄(Si,Al)₈O₂₀(OH)₄·2H₂O |
| 95 | Tarasovit | (Na,K,H₃O,Ca)₂Al₄[(OH)₂\|(Si,Al)₄O₁₀]₂·H₂O |
| 96 | Tosudit | Na_{0.5}(Al,Mg)₆(Si,Al)₈O₁₈(OH)₁₂·5H₂O |
| 97 | Corrensit | (Ca,Na,K)(Mg,Fe,Al)₉(Si,Al)₈O₂₀(OH)₁₀·nH₂O |
| 98 | Brinrobertsit | (Na,K,Ca)_{0.3}(Al,Fe,Mg)₄(Si,Al)₈O₂₀)(OH)₄·3.5H₂O |
| 99 | Montmorillonit | (Na,Ca)_{0.3}(Al,Mg)₂Si₄O₁₀(OH)₂·nH₂O |
| 100 | Beidellit | (Na,Ca_{0.5})_{0.3}Al₂(Si,Al)₄O₁₀(OH)₂·4H₂O |
| 101 | Nontronit | Na_{0.3}Fe₂³⁺(Si,Al)₄O₁₀(OH)₂·4H₂O |
| 102 | Volkonskoit | Ca_{0.3}(Cr³⁺,Mg,Fe³⁺)₂(Si,Al)₄O₁₀(OH)₂·4H₂O |
| 103 | Swinefordit | (Ca,Na)_{0.3}(Al,Li,Mg)₂(Si,Al)₄O₁₀(OH,F)₂·2H₂O |
| 104 | Yakhontovit | (Ca,Na,K)_{0.3}(CuFe²⁺Mg)₂Si₄O₁₀(OH)₂·3H₂O |
| 105 | Hectorit | Na_{0.3}(Mg,Li)₃Si₄O₁₀(F,OH)₂ |
| 106 | Saponit | (Ca\|₂,Na)_{0.3}(Mg,Fe²⁺)₃(Si,Al)₄O₁₀(OH)₂·4H₂O |
| 107 | Ferrosaponit | Ca_{0.3}(Fe²⁺,Mg,Fe³⁺)₃[(OH)₂\|(Si,Al)Si₃O₁₀]·4H₂O |
| 108 | Spadait | MgSiO₂(OH)₂H₂O |
| 109 | Stevensit | (Ca\|₂)_{0.3}Mg₃Si₄O₁₀(OH)₂ |
| 110 | Sauconit | Na_{0.3}Zn₃(Si,Al)₄O₁₀(OH)₂·4H₂O |
| 111 | Zinksilit | Zn₃Si₄O₁₀(OH)₂·4H₂O |
| 112 | Vermiculit | Mg_{0.7}(Mg,Fe,Al)₆(Si,Al)₈O₂₀(OH)₄·8H₂O |
| 113 | Rilandit | (Cr³⁺,Al)₆SiO₁₁·5H₂O |
| 114 | Donbassit | Al_{2.3}[(OH)₈\|AlSi₃O₁₀] |
| 115 | Sudoit | Mg₂Al₃(Si₃Al)O₁₀(OH)₈ |
| 116 | Klinochlor | (Mg,Fe²⁺)₅Al(Si₃Al)O₁₀(OH)₈ |
| 117 | Chamosit | (Fe²⁺,Mg,Fe³⁺)₅Al(Si₃Al)O₁₀(OH,O)₈ |
| 118 | Orthochamosit | (Fe²⁺,Mg,Fe³⁺)₅Al(Si₃Al)O₁₀(OH,O)₈ |
| 119 | Baileychlor | (Zn,Fe²⁺,Al,Mg)₆(Si,Al)₄O₁₀(OH)₈ |
| 120 | Pennantit | Mn²⁺₅Al(Si₃Al)O₁₀(OH)₈ |
| 121 | Nimit | (Ni,Mg,Fe²⁺)₅Al(Si₃Al)O₁₀(OH)₈ |
| 122 | Gonyerit | Mn²⁺₅Fe³⁺(Si₃Fe³⁺O₁₀)(OH)₈ |
| 123 | Cookeit | LiAl₄(Si₃Al)O₁₀(OH)₈ |
| 124 | Borocookeit | Li_{1-1.5}Al_{4-3.5}[(OH,F)₈\|(B,Al)Si₃O₁₀] |
| 125 | Manandonit | Li₂Al₄[(Si₂AlB)O₁₀](OH)₈ |
| 126 | Franklinfurnaceit | Ca₂(Fe³⁺Al)Mn³⁺Mn₃²⁺Zn₂Si₂O₁₀(OH)₈ |
| 127 | Kämmererit(Var.v. Klinochlor) | Mg₅(Al,Cr)₂Si₃O₁₀(OH)₈ |
| 128 | Niksergievit | (Ba, Ca)₂Al₃[(OH)₆\|CO₃\|(Si, Al)₄O₁₀]·0.2 H₂O |
| 129 | Surit | Pb₂Ca(Al,Mg)₂(Si,Al)₄O₁₀(OH)₂(CO₃,OH)₃·0.5 H₂O |
| 130 | Ferrisurit | (Pb,Ca)₂₋₃(Fe³⁺,A)₂[(OH,F)_{2.5-3}\|(CO₃)_{1.5-2}\|Si₄O₁₀]·0.5 H₂O |
| 131 | Kaolinit | Al₂Si₂O₅(OH)₄ |
| 132 | Dickit | Al₂Si₂O₅(OH)₄ |
| 133 | Halloysit-7Ä | Al₂Si₂O₅(OH)₄ |
| 134 | Sturtit | Fe³⁺(Mn²⁺,Ca,Mg)Si₄O₁₀(OH)₃·10 H₂O |
| 135 | Allophan | Al₂O₃·(SiO₂)_{1.3-2}·(H₂O)_{2.5-3} |
| 136 | Imogolith | Al₂SiO₃(OH)₄ |
| 137 | Odinit | (Fe³⁺,Mg,Al,Fe²⁺,Ti,Mn)_{2.4}(Si_{1.8}Al_{0.2})O₅(OH)₄ |
| 138 | Hisingerit | Fe₂³⁺Si₂O₅(OH)₄·2H₂O |
| 139 | Neotokit | (Mn,Fe²⁺)SiO₃·H₂O |
| 140 | Chrysotil | Mg₃Si₂O₅(OH)₄ |
| 141 | Klinochrysotil | Mg₃Si₂O₅(OH)₄ |
| 142 | Maufit | (Mg,Ni)Al₄Si₃O₁₃·4H₂O |
| 143 | Orthochrysotil | Mg₃Si₂O₅(OH)₄ |
| 144 | Parachrysotil | Mg₃Si₂O₅(OH)₄ |
| 145 | Antigorit | (Mg,Fe²⁺)₃Si₂O₅(OH)₄ |
| 146 | Lizardit | Mg₃Si₂O₅(OH)₄ |
| 147 | Karyopilit | Mn²⁺₃Si₂O₅(OH)₄ |
| 148 | Greenalith | (Fe²⁺,Fe³⁺)₂₋₃Si₂O₅(OH)₄ |
| 149 | Berthierin | (Fe²⁺,Fe³⁺,Al)₃(Si,Al)₂O₅(OH)₄ |
| 150 | Fraipontit | (Zn,Al)₃(Si,Al)₂O₆(OH)₄ |
| 151 | Zinalsit | Zn₇Al₄(SiO₄)₆(OH)₂·9H₂O |
| 152 | Dozyit | Mg₇(Al,Fe³⁺,Cr)₂[(OH)₁₂\|Al₂Si₄O₁₅] |
| 153 | Amesit | Mg₂Al(SiAl)O₅(OH)₄ |
| 154 | Kellyit | (Mn²⁺,Mg,Al)₃(Si,Al)₂O₅(OH)₄ |
| 155 | Cronstedtit | Fe₂²⁺Fe³⁺(SiFe³⁺)O₅(OH)₄ |
| 156 | Karpinskit | (Mg,Ni)₂Si₂O₆(OH)₂ |
| 157 | Nepouit | (Ni,Mg)₃Si₂O₅(OH)₄ |
| 158 | Pecorait | Ni₃Si₂O₆(OH)₄ |
| 159 | Brindleyit | (Ni,Mg,Fe²⁺)₂Al(SiAl)O₅(OH)₄ |
| 160 | Carlosturanit | (Mg,Fe²⁺,Ti)₂₁(Si,Al)₁₂O₂₈(OH)₃₄·H₂O |
| 161 | Pyrosmalith-(Fe) | (Fe²⁺,Mn)₈Si₆O₁₅(Cl,OH)₁₀ |
| 162 | Pyrosmalith-(Mn) | (Mn,Fe²⁺)₈Si₆O₁₅(OH,Cl)₁₀ |
| 163 | Brokenhillit | (Mn,Fe)₈Si₆O₁₅(OH,Cl)₁₀ |
| 164 | Nelenit | (Mn,Fe²⁺)₁₆Si₁₂As³⁺₃O₃₆(OH)₁₇ |
| 165 | Schallerit | (Mn²⁺,Fe²⁺)₁₆Si₁₂As³⁺₃O₃₆(OH)₁₇ |
| 166 | Friedelit | Mn²⁺₈Si₆O₁₅(OH,Cl)₁₀ |
| 167 | Mcgillit | Mn²⁺₈Si₆O₁₅(OH)₈Cl₂ |
| 168 | Bementit | Mn₇Si₆O₁₅(OH)₈ |
| 169 | Varennesit | Na₈(Mn,Fe³⁺,Ti)₂[(OH,Cl)₂\|(Si₂O₅)₅]·12H₂O |
| 170 | Naujakasit | Na₆(Fe²⁺,Mn)Al₄Si₈O₂₆ |
| 171 | Manganonaujakasit | Na₆(Mn²⁺,Fe²⁺)Al₄[Si₈O₂₆] |
| 172 | Spodiophyllit | (Na,K)₄(Mg,Fe²⁺)₃(Fe³⁺,Al)₂(Si₈O₂₄) |
| 173 | Sazhinit-(Ce) | Na₂CeSi₆O₁₄(OH)·nH₂O |
| 174 | Sazhinit-(La) | Na₃La[Si₆O₁₅]·2H₂O |
| 175 | Burckhardtit | Pb₂(Fe³⁺Te⁶⁺)[AlSi₃O₈]O₆ |
| 176 | Tuperssuatsiait | Na₂(Fe³⁺,Mn²⁺)₃Si₈O₂₀(OH)₂·4H₂O |
| 177 | Palygorskit | (Mg,Al)₂Si₄O₁₀)(OH)·4H₂O |
| 178 | Yofortierit | Mn²⁺₅Si₈O₂₀(OH)₂·7H₂O |
| 179 | Sepiolith | Mg₄Si₆O₁₅(OH)₂·6H₂O |
| 180 | Falcondoit | (Ni,Mg)₄Si₆O₁₅(OH)₂·6H₂O |
| 181 | Loughlinit | Na₂Mg₃Si₆O₁₆·8H₂O |
| 182 | Kalifersit | (K,Na)₅Fe₇³⁺[(OH)₃Si₁₀O₂₅]₂·12H₂O |
| 183 | Minehillit | (K,Na)₂₋₃Ca₂₈(Zn₄Al₄Si₄₀)O₁₁₂(OH)₁₆ |
| 184 | Truscottit | (Ca,Mn)₁₄Si₂₄O₅₈(OH)₈·2H₂O |
| 185 | Orlymanit | Ca₄Mn₃²⁺Si₈O₂₀(OH)₆·2H₂O |
| 186 | Fedorit | (Na,K)₂₋₃(Ca,Na)₇[Si₄O₈(F,Cl,OH)2\|(Si₄O₁₀)₃]·3.5H₂O |
| 187 | Reyerit | (Na,K)₄Ca₁₄Si₂₂Al₂O₅₈(OH)₈·6H₂O |
| 188 | Gyrolith | NaCa₁₆Si₂₃AlO₆₀(OH)₈·14H₂O |
| 189 | Tungusit | Ca₁₄Fe₉²⁺[(OH)₂₂\|(Si₄O₁₀)₆] |
| 190 | Zeophyllit | Ca₄Si₃O₈(OH,F)₄·2H₂O |
| 191 | Armstrongit | CaZr(Si₆O₁₅)· 3 H₂O |
| 192 | Jagoit | Pb₁₈Fe³⁺₄[Si₄(Si,Fe³⁺)₆][Pb₄Si₁₆(Si,Fe)₄]O₈₂Cl₆ |
| 193 | Hyttsjöit | Pb₁₈Ba₂Ca₅Mn₂²⁺Fe₂³⁺[Cl\|(Si₁₅O₄₅)₂]·6H₂0 |
| 194 | Maricopait | Ca₂Pb₇(Si₃₆,Al₁₂)(O,OH)₉₉·n(H₂O,OH) |
| 195 | Cavansit | Ca(VO)Si₄O₁₀·4H₂O |
| 196 | Pentagonit | Ca(VO)Si₄O₁₀·4H₂O |
| 197 | Weeksit | (K,Ba)₂[(UO₂)₂\|Si₅O₁₃]·4H₂O |
| 198 | Coutinhoit | Th_{0.5}(UO₂)₂Si₅O₁₃·3H₂O |
| 199 | Haiweeit | Ca[(UO₂)₂\|Si₅O₁₂(OH)₂]·6H₂O |
| 200 | Metahaiweeit | Ca(UO₂)₂Si₆O₁₅·nH₂O |
| 201 | Monteregianit-(Y) | KNa₂YSi₈O₁₉·5H₂O |
| 202 | Mountainit | KNa₂Ca₂[Si₈O₁₉(OH)]·6H₂O |
| 203 | Rhodesit | KHCa₂Si₈O₁₉·5H₂O |
| 204 | Delhayelith | K₇Na₃Ca₅Al₂Si₁₄O₃₈F₄Cl₂ |
| 205 | Hydrodelhayelith | KCa₂AlSi₇O₁₇(OH)₂·6H₂O |
| 206 | Macdonaldit | BaCa₄Si₁₆O₃₆(OH)₂·10H₂O |
| 207 | Cymrit | Ba(Si,Al)₄(O,OH)₈·H₂O |
| 208 | Kampfit | Ba₁₂(Si₁₁Al₅)O₃₁(CO₃)₈Cl₅ |
| 209 | Lourenswalsit | (K,Ba)₂(Ti,Mg,Ca,Fe)₄(Si,Al,Fe)₆O₁₄(OH)₁₂ |
| 210 | Tienshanit | (Na,K)₉₋₁₀(Ca,Y)₂Ba₆(Mn²⁺,Fe²⁺,Ti⁴⁺,Zn)₆(Ti,Nb) [(O,F,OH)₁₁\|B₂O₄\|Si₆O₁₅]₆ |
| 211 | Wickenburgit | Pb₃CaAl[Si₁₀O₂₇]·3H₂O |
| 212 | Silhydrit | Si₃O₆·H₂O |
| 213 | Magadiit | Na₂Si₁₄O₂₉·11H₂O |
| 214 | Strätlingit | Ca₂Al[(OH)₆AlSiO₂(OH)_{4]}·2.5 H₂O |
| 215 | Vertumnit | Ca₄Al₄Si₄O₆(OH)₂₄·3H₂O |
| 216 | Zussmanit | K(Fe²⁺,Mg,Mn)₁₃(Si,Al)₁₈O₄₂(OH)₁₄ |
| 217 | Coombsit | K(Mn²⁺,Fe²⁺,Mg)₁₃[(OH)₇\|(Si,Al)₃O₃\|Si₆O₁₈]₂ |

### a) vgl. Mineralienatlas, Mineralklasse VIII/H - Schichtsilikate (Phyllosilikate), Strunz 8 Systematik

Ganz besonders bevorzugt wird Bentonit aus der Gruppe der Montmorillonite ((Na,Ca)_{0.3}(Al,Mg)₂Si₄O₁₀(OH)₂·nH₂O) verwendet. Bentonit ist eine Mischung aus verschiedenen Tonmineralien und enthält als wichtigsten Bestandteil Montmorillonit.

Weitere geeignete Trägermaterialien sind Sklerite und Skelette von Diatomeen, Radiolarien, Seesternen, Korallen und Schwämmen.

Vorzugsweise weist das Trägermaterial eine Wärmeleitfähigkeit λ von 0,001 W/(m.K) bis 1,0 W/(m.K) auf.

Vorzugsweise wird die mindestens eine Art eines partikulären Adsorbens und/oder Absorbens aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen von partikulären Adsobentien und/oder Adsorbentien, ausgewählt.

Vorzugsweise werden partikulären Absobentien und/oder Adsorbentien aus der Gruppe, bestehend aus, Aktivkoks, Aktivkoks/Kalkhydrat, Phyllosilikaten, Zeolithen, Kieselgelen, Aluminiumoxid, Tonerden, Aktivtonerden, metallorganischen Gerüstverbindungen (MOFs) und Trass, ausgewählt.

Bevorzugt weisen die partikulären, anorganischen Trägermaterialien, die partikulären, anorganischen, bakterizid und/oder viruziden Materialien und die partikulären Adsobentien und/oder Absorbentien eine durch Siebanalyse ermittelte mittlere Teilchengröße von 0,5 cm bis 10 cm auf.

Gemäß der vorliegenden Erfindung weist das partikuläre, anorganische, bakterizid und/oder viruzid beschichtete, inerte Trägermaterial eine Beschichtung auf, die vorzugsweise eine Stärke von 100 nm bis 100 µm hat. Bevorzugt hat die Beschichtung einen Kontaktwinkel θ mit Wasser von ≤90°. Ohne an eine Theorie gebunden zu sein wird, wird vermutet, dass dadurch Aerosole die viruziden und/oder bakteriziden Beschichtungen besser benetzen und so der Kontakt der darin enthaltenen Mikroorganismen, insbesondere der Bakterien und/oder Viren, mit den Bioziden intensiviert wird.

Die Beschichtung enthält mindestens ein Biozid, insbesondere mindestens ein Bakterizid und/oder Viruzid, insbesondere mindestens ein Pharmazeutikum und/oder Desinfektionsmittel, das oder die an der ausgehärteten Bindemittelmatrix der Beschichtung in der Form von Mikropartikeln fixiert und/oder in der ausgehärteten Bindemittelmatrix dispergiert oder gelöst sind.

Vorzugsweise handelt es sich bei den Bioziden um Feststoffe oder Flüssigkeiten mit einer Siedetemperatur bei Atmosphärendruck oberhalb von 150 °C oder einer Zersetzungstemperatur oberhalb 150°. Oder aber sie haben keinen Siedepunkt und/oder einen sehr niedrigen Dampfdruck bei ihrer Gebrauchstemperatur. Sie emittieren keine unangenehmen Gerüche und schädliche, toxische und/oder korrosive Substanzen wie Chlor, Brom, Iod, organische Halogenverbindungen, organische und anorganische Säuren, Ammoniak, organische Amine, anorganische und organische Schwefelverbindungen wie Schwefelwasserstoff oder Mercaptane, Ozon, organische oder anorganische Phosphorverbindungen wie Phosphine oder organische Verbindungen wie Formaldehyd, Ketone, Ester oder Terpene bei den Temperaturen, bei denen die beschichteten Trägermaterialien verwendet werden.

Vorzugsweise sind die festen Biozide, insbesondere die festen Bakterizide und/oder Viruzide, bei ihrer Gebrauchstemperatur Mikropartikel, die beispielsweise durch Sprühtrocknung hergestellt werden können (vgl. R. Vehring in"Pharmaceutical Particle Engineering via Spray Drying", in Pharmaceutical Research 2007, Expert Review).

Die Mikropartikel können wie die Trägermaterialien die unterschiedlichsten Morphologien aufweisen wie Kugeln, Hohlkugeln, Scherben, Granulate, gemahlenen Brocke, Scherben und Granulate, Pellets, Ringen Kugeln mit Kern-Schale-Strukturen, Ellipsoide, Würfel, Quader, Pyramiden, Kegel, Zylinder, Rhomben, Dodekaeder, abgestumpfte Dodekaeder, Ikosaeder, abgestumpfte Ikosaeder, Hantel, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten und/oder in mindestens einer Richtung des Raumes gebogenen Scherben, Ringen, Hanteln, Tori, Nadeln und Plättchen. Außerdem können die Ecken und Kanten abgerundet sein. Zwei oder mehr Mikropartikel können Aggregate oder Agglomerate bilden. Dabei können die Mikropartikel von gleicher oder unterschiedlicher Art sein. Zwei oder drei zylinderförmige Mikropartikel können aneinander in Form eines T oder eines Y gebunden sein. Nicht zuletzt können ihre Oberfläche Vertiefungen enthalten, sodass erdbeerförmige, himbeerförmige oder brombeerförmige Morphologien resultieren.

Im Rahmen der vorliegenden Erfindung gilt der Durchmesser von Mikropartikeln, die keine Kugelform haben, als die längste, durch die Mikropartikel gelegte Strecke.

Vorzugsweise wird der mittlere Durchmesser oder die mittlere Teilchengröße der Mikropartikel durch Lichtmikroskopie, Trockensiebung, Nasssiebung, Coulter-Counter-Messungen oder Fraunhofer-Streuung bestimmt.

Vorzugsweise werden Biozide, insbesondere Bakterizide und/oder Viruzide verwendet, die von staatlichen oder zwischenstaatlichen Behörden zugelassen sind. Insbesondere werden die Biozide aus der folgenden Gruppe ausgewählt:
Biphenyl-2ol
DCCP
   1-(2,3-Dichlorphenyl)piperazin
L(+)-Milchsäure
Zitronensäure
Ascorbinsäure
MIT
   Methylisothiazolinon
CMIT, CMI, MCI
   Chloromethylisothiazolinon
BIT
   Benzisothiazolinon
OIT, OI
   Octylisothiazolinon
DCOIT, DCOI
   Dichlorooctylisothiazolinon
BBIT
   Butylbenzisothiazolinon
PHMB polvhexanid
   Poly(iminocarbonylimidoyl-iminocarbonylimidoylimino-1,6-hexanediyl)-hydrochlorid
Propioconazol (±)-1-{[2-(2,4-Dichlorphenyl)-4-propyl-1,3-dioxolan-2-yl]methyl}-*H*-1,2,4-triazol (IUPAC)
Folpet
   *N*-(Trichlormethylthio)phthalimid
Chlorkresole,
Fludioxonil
   4-(2,2-Difluor-benzo[1,3]dioxol-4-yl)pyrrol-3-carbonitril (IUPAC),
Azoxvstrobin
   Methyl-(*E*)-2-{2-[6-(2-cyanophenoxy)pyrimidin-4-yloxyl]phenyl}-3-methoxyacrylat(IUPAC),
Quaternäres Ammonium
   Reaktionsprodukt von N-(C₁₀-C₁₆)-N-Trimethylamin mit Chloressigsäure
Calcium/Magnesium-Oxid
Calcium/Magnesium-Oxidtetrahydrate
Kalkhydrat
Kalk
IPBC
   3-Iod-2-propinyl-butylcarbamat
Bronopol
   2-Brom-2-nitropropan-1,3-diol
1R-trans-Phenothrine 3-Phenoxybenzyl-(1R,3R)-2,2-dimethyl-3-(2-methylprop-1-enyl)cyclopropancarboxylat
2-Phenoxvethanol
Diamin
   N-(3-Aminopropyl)-N-dodecylpropan-1,3-diamin
DTBMA
   2,2'-Dithiobis[N-methylbenzamid]
DBDCB
   2-Brom-2-(brommethyl)pentandinitril
IPBC
   3-Iod-2-propynylbutylcarbamat
DDAC (C8-10)
   Didecyldimethylammoniumchlorid
BBIT
   2-Butyl-benzo[d]isothiazol-3-on
PHMB (1600;1.8)
   Polyhexamethylenebiguanidehydrochlorid mit einem zahlenmittleren Molekulargewicht (Mn) von 1600 und einer mittleren Polydispersität (PDI) von 1.8
MBIT
   Poly[iminocarbonimidoyliminocarbonimidoylimino-1, 6-hexandiyl]hydrochloride 49
Mischung von CMIT/MIT
Natriumpyrithion
   2-Methyl-1,2-benzothiazol-3(2H)-on
Pvridin-2-thiol-1-oxid- Natriumsalz
Reaktionsmasse von Titandioxid und Silberchlorid
DBNPA
   2,2- Dibrom-2-cyanoacetamide
Zinkpyrithion
Dodecylguanidinmonohydrochlorid
p-Diiodmethyl)sulphonyl]toluol
Dichlofluanid, Euparen
   *N*-(Dichlorfluormethylthio)-*N*',*N*'-dimethyl-*N*-phenylsulfamid (IUPAC)
Thiachloprid, Calypso {(2Z)-3-[(6-Chlor-3-pyridinyl)methyl]-1,3-thiazolidin-2-yliden}cyanamid (IUPAC)
Chlothianidin
   (*E*)-1-(2-chlor-1,3-thiazol-5-ylmethyl)-3-methyl-2-nitroguanidine
Etofenprox, Trebon
   2-(4-Ethoxyphenyl)-2-methylpropyl-3-phenoxybenzylether
Tebuconazol
   (*RS*)-1-*tert*-Butyl-1-(4-chlorphenethyl)-2-(1*H*-1,2,4-triazol-1-yl)ethanol
K-HDO
   N-Cyclohexylhydroxydiazen-1-oxid-Natriumssalz
Thiabendazol
   2-(4-Thiazolyl)-1*H*-benzimidazol
Thiamethoxam
   3-(2-Chlor-thiazol-5-ylmethyl)-5-methyl(1,3,5)oxadiazinan-4-yliden-*N*-nitroamin (Zersetzung bei 147°C)
Fenpropimorph
   (±)-*cis*-4-[3-(4-*tert*-Butylphenyl)-2-methylpropyl]-2,6-dimethylmorpholin (IUPAC; bp. 120°C)
Borsäure
Boroxid
Dinatriumoctaborattetrahydrat
Dinatriumoctaboratpentahydrat
Dinatriumtetraboratdecahvdrat
Tolvlfluanid
   *N*-[Dichlor(fluor)methyl]sulfanyl-*N*-(dimethylsulfamoyl)-4-methylanilin (IUPAC; Zersetzung ab 150 °C)
Dazomet
   3,5-Dimethylperhydro-1,3,5-thiadiazin-2-thion (Schmelzpunkt 140°C unter Zersetzung)
Fenoxycarb
   Ethyl-N-[2-(4-phenoxyphenoxy)ethyl]carbamat
Bifentrin (1*R**,3*R**)-3-(2-Chlor-3,3,3-trifluor-1-propenyl)-2,2-dimethylcyclopropan-carbonsäure(2-methylbiphenyl-3-yl)methylester
DCOIT
   4,5-Dichlor-2-*n*-octyl-4-isothiazolin-3-on
Kupferhydroxid
Basisches Kupfercarbonat
Flufenoxuron *N*-[[4-[2-Chlor-4-(trifluormethyl)phenoxy]-2-fluorphenyl]carbamoyl]-2,6-difluorbenzamid
DDA carbonate
   *N,N*-Didecyl-*N,N*-dimethylammoniumcarbonat
ADBAC
   *N*-Alkyl-*N*-benzyl-*N,N*-dimethylammoniumchlorid
Chlorfenpyr
   4-Bromo-2-(4-chlorphenyl)-1-ethoxymethyl-5-trifluormethyl-pyrrol-3-carbonitril
Cypermethrin (*R*,*S*)-α-Cyano-3-phenoxybenzyl-(1*RS*)-*cis,trans*-3-(2,2-dichlorvinyl)-2,2-dimethylcyclopropan-carboxylat
Cu-HDO
   Bis-(N-cyclohexyldiazeniumdioxy)-kupfer
Cyproconazol
   2-(4-Chlorphenyl)-3-cyclopropyl-1-(1*H*-1,2,4-triazol-1-yl)butan-2-ol (IUPAC)
Permethrin 3-(2,2-Dichloroethenyl)-2,2-dimethylcyclopropancarbonsäure-(3-phenoxyphenyl)methylester
Natriumsorbat
Granuliertes Kupfer
Didecylmethylpoly(oxvethyl)ammoniumpropionat
ATMAC/TMAC
   Cocoalkyltrimethylammoniumchlorid
OIT
   2-Octyl-2*H*-isothiazol-3-on (Siedepunkt 120°C)
Penflufen
   2'-[(*RS*)-1,3-Dimethylbutyl]-5-fluor-1,3-dimethylpyrazol-4-carboxanilid
MBM
   Natriumdiethyldithiocarbamat
Difethialon 3-[3-(4'-Brom[1,1'-biphenyl]-4-yl)-1,2,3,4-tetrahydronaphth-1-yl]-4-hydroxy-2*H*-1-benzothiopyran-2-on
Difenacoum 3-(3-(1,1'-Biphenyl)-4-yl-1,2,3,4-tetrahydro-1-naphthalenyl)-4-hydroxy-2*H*-1-benzopyran-2-on
Chloralose
   C1,2-O-(2,2,2-Trichlorethyliden)-α-D-glucofuranose
Bromadiolon 3-(3-(4'-Brom(1,1'-biphenyl)-4-yl)-3-hydroxy-1-phenyl-propyl)-4-hydroxy-2*H*-1-benzopyran-2-on
Chlorphacinon
   (*RS*)-2-(a-(4-Chlorphenyl)phenylacetyl)indan-1,3-dion
Coumatetralvl
   4-Hydroxy-3-(1,2,3,4-tetrahydro-1-naphthyl)cumarin
Flocumafen 4-Hydroxy-3-(1,2,3,4-tetrahydro-3-(4-(4-trifluormethylbenzyloxy)phenyl)-1-naphthyl)-cumarin
Warfarin
   (RS)-4-Hydroxy-3-(3-oxo-1-phenyl-butyl)-cumarin (IUPAC)
Warfarin Natrium
Brodifacum 3-[3-(4'-Bromo-1,1'-biphenyl-4-yl)-1,2,3,4-tetrahydro-1-naphthyl]-4-hydroxycumarin
Cholecalciferol 3-[2-[7a-Methyl-1-(6-methylheptan-2-yl)- 2,3,3a,5,6,7-hexahydro-1*H*-inden-4-yliden]ethyliden]-4-methyliden-cyclohexan-1-ol
Indoxocarb (*RS*)-Methyl-7-chlor-2,3,4a,5-tetrahydro-2-[methoxycarbonyl-(4-trifluormethoxyphenyl)carbamoyl]indeno[1,2-*e*][1,3,4]oxadiazin-4a-carboxylat
Methofluthrin (1*RS*,3*RS*;1*SR*,3*SR*)-2,2-Dimethyl-3-(*EZ*)-(prop-1-enyl)cyclopropancarbonsäure-2,3,5,6-tetrafluoro-4-(methoxymethyl)benzylester
Spinosad 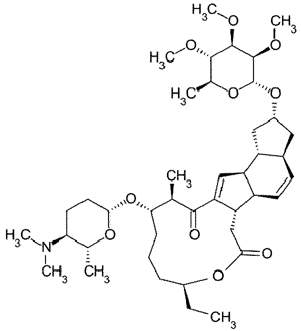
Imidacloprid
   1-(6-Chlor-3-pyridinylmethyl)-*N*-nitroimidazolidin-2-ylidenamin
Abamectin 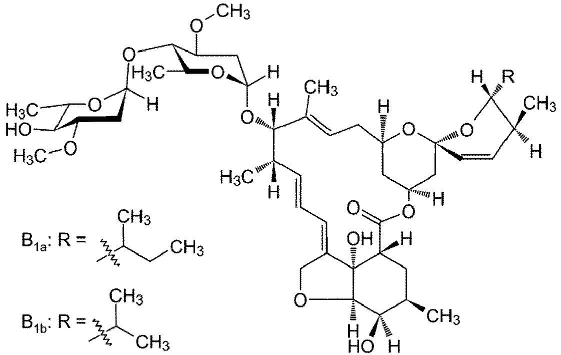
Fipronil (*RS*)-5-Amino-1-(2,6-dichlor-α,α,α-trifluor-*p*-tolyl)-4-trifluormethylsulfinyl-1*H*-pyrazol-3-carbonitril
Lamba-Cvhalotrin 3-(2-Chlor-3,3,3-trifluor-1-propenyl)-2,2-dimethyl-cyclopropan-carbonsäure-cyano(3-phenoxyphenyl)methylester
Deltamethrin
   (1*R*,3*R*)-[(*S*)-α-Cyano-3-phenoxybenzyl-3-(2,2-dibromvinyl)]-2,2-dimethylcyclopropancarboxylat (IUPAC)
Bendiocarb
   2,2-Dimethyl-1,3-benzodioxol-4-yl-*N*-methylcarbamat
Pvriproxvfen
   (*RS*)-4-Phenoxyphenyl2-(2-pyridyloxy)propylether
Diflubenzuron
   *N*-{[(4-Chlorphenyl)amino]carbonyl}-2,6-difluorbenzamid
1R-trans-Phenothrin 2,2-Dimethyl-3-(2-methylpropenyl)cyclopropancarbonsäure-m-phenoxybenzylester
S-Methopren
   11-Methoxy-3,7,11-trimethyl-2,4-dodecadiensäure-1-methylethylester
Transfluthrin (1*R*)-*trans*-3-(2,2-Dichlorvinyl)-2-dimethylcyclopropancarbonsäure-2,3,5,6-tetrafluorbenzylester
Synthetisches amorphes Siliziumdioxid
Oberflächenmodifiziertes synthetisches amorphes Siliziumdioxid
Dinotefuran
   (*RS*)-*N*-Methyl-*N*′-nitro-*N*ʺ-[(tetrahydro-3-furanyl)methyl]guanidin
Hexaflumuron 1-[3,5-Dichlor-4-(1,1,2,2-tetrafluorethoxy)phenyl]-3-(2,6-difluorbenzoyl)harnstoff (IUPAC)
Cvromazin
   *N*-Cyclopropyl-1,3,5-triazin-2,4,6-triamin
Cyfluthrin alpha-Cyano-4-fluor-3-phenoxybenzyl-3-(2,2-dichlorvinyl)-2,2-dimethylcyclopropancarboxylat
PBO
   5-[2-(2-Butoxyethoxy)ethoxymethyl]-6-propyl-1,3-benzodioxol
Epsilon-Momfluorothrin 2,3,5,6-Tetrafluor-4-(methoxymethyl)benzyl (1*R*,3*R*)-3-[(*Z*)-2-cyanoprop-1-enyl]-2,2-dimethylcyclopropancarboxylat
Imiprothrin (2,5-Dioxo-3-prop-2-inylimidazolidin-1-yl)methyl-2,2-dimethyl-3-(2-methylprop-1-enyl)cyclopropan-1-carboxylat
Acetamiprid (*E*)-*N*¹-[(6-Chlor-3-pyridyl)methyl]-*N*²-cyano-*N*¹-methylacetamidin (IUPAC)
Cyphenotrin [Cyano-(3-phenoxyphenyl)methyl]-2,2-dimethyl-3-(2-methylprop-1-enyl)cyclopropan-1-carboxylat
DEET
   *N*,*N*-Diethyl-3-methylbenzamid
Nonansäure
Decansäure
(Z.E)-TDA
   (Z, E)-Tetradeca-9, 12-dienylacetat
Methyl nonyl ketone
   2-Undecanon
Zineb
   Zink-ethylen-1,2-bis-dithiocarbamat
cis-9-Tricosen
DCOIT
   Dichloroctyl-isothiazolinone
OBPA
   10,10'-Oxybisphenoxoarsin
Kupfer-Pvrithion
   Pyridin-2-thiol-1-oxid-Kupfer
Trichlosan
   Polychlorierte Phenoxyphenole
Medetomidin
   (*RS*)-4-[1-(2,3-Dimethylphenyl)ethyl]-1*H*-imidazol
Tralopyril 4-Brom-2-(4-chlorphenyl)-5-(trifluormethyl)-1*H*-pyrrol-3-carbonitril (IUPAC)
Kupferflocken
   beschichtet mit einem Film aus einer aliphatischen Carbonsäuren
Dikupferoxid-Mikropartikel
Kupferoxid-Mikropartikel
Kupferthiocvanat-Mikropartikel.
Silbermikropartikel
Siberchloridmikropartikel
Chlorhexidin
   (RS)-4-[1-(2,3-Dimethylphenyl)ethyl]-1H-imidazolchlorid und -gluconat
Octenidin
   *N*,*N*'-(Decan-1,10-diyldi-1(4*H*-pyridyl-4-yliden)bis(octylammonium)dichlorid
Taurolidin 4-[(1,1-Dioxo-1,2,4-thiadiazinan-4-yl)methyl]-1,2,4-thiadiazinan-1,1-dioxide
2-Oxido-4-[(2-oxido-1-oxo-1,2,4-thiadiazinan-2-ium-4-yl)methyl]-1.2.4-thiadiazinan-2-ium-1-oxide
4-[(1,1-Dioxothiadiazinan-4-yl)methyl]thiadiazinan-1,1-dioxid
2-[(1,1-Dioxo-1,2,4-thiadiazinan-2-yl)methyl]-1,2,4-thiadiazinan-1,1-dioxid
3-[(1,1-Dioxo-1,2,4-thiadiazinan-3-yl)methyl]-1,2,4-thiadiazinan-1,1-dioxid
2-Hydroxy-4-[(2-hydroxy-1-oxo-1,2,4-thiadiazinan-4-yl)methyl]-1,2,4-thiadiazinan-1-oxid
N-(4-Azidobutyl)-2-methylsulfonylethansulfonamid
4-N-Cyclopropylpiperazin-1,4-disulfonamid
(2,2-Dimethyl-1-methylsulfonylpropyl)-(methyldiazenyl)sulfonyldiazen
6-Methyl-N-[1-(methylamino)ethenyl]-1,1-dioxo-1,2,6-thiadiazinan-2-sulfonamid
Azodicarbonamid
Cicloxolone Natrium
   18beta-glycyrrhetinsäure-(H)-(1R)-cis-cyclohexan-1,2-dicarboxylat
Dichlorisocyanursäure-Natriumsalz
Kongorot Dinatrium-3,3'-[4,4'-biphenyldiyldi(*E*)-2,1-diazenediyl]bis(4-amino-1-naphthalinsulfonat) (IUPAC)
Para-aminobenzoesäure
Bis(monosuccinamid)-Derivat von p,p'-Bis(2-aminoethyl)diphenyl-C60 (Fulleren)
Merocyanin
   1-Methyl-4-[(oxocyclohexadienyliden)ethyliden]-1,4-dihydropyridin
Bengal Rosa. Acid Red 94 4,5,6,7-Tetrachloro-3',6'-dihydroxy-2',4',5',7'-tetraiodspiro[isobenzofuran-1(3*H*),9'-[9*H*]xanthen]-3-on
Hypericin 1,3,4,6,8,13-Hexahydroxy-10,11-dimethylphenanthro[1,10,9,8-*opqra*]perylen-7,14-dion (IUPAC)
Hypocrellin (12*R*,13*S*)-12-Acetyl-9,13,17-trihydroxy-5,10,16,21-tetramethoxy-13-methylhexacyclo[13.8.0.0^{2,11}.0^{3,8}.0^{4,22}.0^{18,23}]tricosa-1(15),2(11),3(8),4(22),5,9,16,18(23),20-nonaen-7,19-dion
Von Pflanzen extrahierte Anthrachinone
Sulfonierte Anthrachinone
Anthrachinonderivative
   Acid blue 40 und 129, Acid black 48, Alizarin violet R, Reactive blue 2
Gramicidin
   Lineares Pentadecapeptid
Gossypol
   2,2'-Bis(formyl-1,6,7-trihydroxy-5-isopropyl-3-methylnaphthalin)
Extrakte von ledum palustre, leonurus cardiaca, Celandine, Schwarzer Johannisbeere. Preiselbeere und Blaubeere
Alkaloide und Phvtostervlester Marigenolkonzentrate, enthaltend Taxol und/oder Taxanester als Wirkstoffe
Extrakte von Cordia salicifolia
Dampfdestillat von Houttuynia cordata (Saururaceae) und seinen Bestandteilen
5,6,7-Trimethoxyflavon von Calicarpa iaponica
Isocullarein 5,7,8,4'-Tetrahydroxyflavon von Scutellaria baikalensis undnd Isocutellarein-8-methylether
Amantadin
   1-Tricyclo[3.3.1.1^{3,7}]decylamin (IUPAC)
Sialinsäure
   Neuraminsäure
Zanamivir (4*S*,5*R*,6*R*)-5-Acetylamino-4-guanidino-6-[(1*R*,2*R*)-1,2,3-thhydroxypropyl]-5,6-dihydro-4*H-*pyran-2-carbonsäure
Oseltamivir (3*R*,4*R*,5*S*)-4-Acetamido-5-amino-3-(1-ethylpropoxy)cyclohex-1-en-1-carbonsäureethylester
Rimantadin
   (RS)-Adamantan-1-yl-ethylamin (IUPAC)
Diuron
   3-(3,4-Dichlorphenyl)-1,1-dimethylharnstoff
Quaternisiertes Chitosan (vg., A. Domard et al., "New method for the quaternization of chitosan", International Journal of Biological Macromolecules, Band 8, Ausgabe 2, April, 1986, Seiten 105-107).

(Eine detaillierte Beschreibung einiger dieser Viruzide findet sich in A. S. Galabov, "Virucidal agents in the of manorapid synergy™", in GMS Krankenhaushygiene Interdisziplinär, 2007 2(1): Doc 18).

Weitere geeignete Bakterizide und/oder Viruzide sind Polyoxometallate, die im Folgenden mit der Abkürzung »POM« bezeichnet werden.

Die elementare Zusammensetzung und die Struktur der POM können sehr breit variieren.

Bekannt ist beispielsweise die Einteilung der POM in die folgenden Strukturen:
- das Lindquist-Hexamolybdatanion, Mo₆O₁₉²⁻,
- das Decavanadatanion, V₁₀O₂₈⁶⁻,
- das Paratungstatanion, H₂W₁₂O₄₂¹⁰⁻,
- Mo₃₆-Polymolybdate, Mo₃₆O₁₁₂(H₂O)⁸⁻,
- die Strandberg-Struktur, HP₂Mo₅O₂₃⁴⁻,
- die Keggin-Struktur, XM₁₂O₄₀ⁿ⁻,
- die Doppel-Keggin-Struktur,
- die Keggin-Sandwichstruktur,
- die monolacunare Keggin-Struktur,
- die dilacunare Keggin-Struktur,
- die Wells-Dawson-Struktur, X₂M₁₈O₆₂ⁿ⁻,
- die Anderson-Struktur, XM₆O₂₄ⁿ⁻,
- die Allman-Waugh-Struktur, X₁₂M₁₈O₃₂ⁿ⁻,
- die Weakley-Yamase- Struktur, XM₁₀O₃₆ⁿ⁻ und
- die Dexter-Silverton-Struktur, XM₁₂O₄₂ⁿ⁻.

Die Hochzahl n ist hier eine ganze Zahl von 3 bis 20 bezeichnet die Wertigkeit eines Anions, die in Abhängigkeit von den Variablen X und M variiert.

Als ein weiteres Ordnungsprinzip für POM können die Formeln I bis XIII dienen:
- (BW₁₂O₄₀)⁵⁻ (I)

   ,
- (W₁₀O₃₂)⁴⁻ (II)

   ,
- (P₂W₁₈O₆₂)⁶⁻ (III)

   ,
- (PW₁₁O₃₉)⁷⁻ (IV)

   ,
- (SiW₁₁O₃₉)⁸⁻ (V)

   ,
- (HSiW₉O₃₄)⁹⁻ (VI)

   ,
- (HPW₉O₃₄)⁸⁻ (VII)

   ,
- (TM)₄(PW₉O₃₄)^{t-} (VIII)

   ,
- (TM)₄(P₂W₁₅O₅₆)₂^{t-} (IX)

   ,
- (NaP₅W₃₀O₁₁₀)¹⁴⁻ (X)

   ,
- (TM)₃(PW₉O₃₄)₂¹²⁻ (XI)

   und
- (P₂W₁₈O₆)⁶⁻ (XII)

   .

In den Formeln I bis XII steht TM für ein zweiwertiges oder dreiwertiges Übergangsmetallion wie Mn²⁺, Fe²⁺, Fe³⁺, Co²⁺, Co³⁺, Ni²⁺, Cu²⁺ und Zn²⁺. Die Hochzahl t ist eine ganze Zahl und bezeichnet die Wertigkeit eines Anions, die in Abhängigkeit von der Wertigkeit der Variable TM variiert.

Des Weiteren kommen POM der allgemeinen Formel XIII in Betracht:
- (AₓGa_{y}NbₐO_{b})^{z-} (XIII)

   .

In der Formel XIII steht die Variable A für Phosphor, Silicium oder Germanium und der Index x steht für 0 oder für eine ganze Zahl von 1 bis 40. Der Index y steht für eine ganze Zahl von 1 bis 10, der Index a steht für eine ganze Zahl von 1 bis 8 und der Index b ist eine ganze Zahl von 15 bis 150. Die Hochzahl z variiert in Abhängigkeit von der Natur und dem Oxidationsgrad der Variable A. Es kommen auch die Aquakomplexe und die aktiven Fragmente der POM XIII in Betracht.

Wenn der Index x gleich 0 ist, ist y bevorzugt gleich 6-a, wobei der Index a gleich einer ganzen Zahl von 1 bis 5 ist und der Index b gleich 19 ist.

Wenn die Variable A gleich Silicium oder Germanium ist, ist der Index x gleich 2, der Index y gleich 18, der Index a gleich 6 und der Index b gleich 77.

Wenn die Variable A gleich P ist, ist der Index x gleich 2 oder 4, der Index y gleich 12, 15, 17 oder 30, der Index a gleich 1, 3 oder 6 und der Index b gleich 62 oder 123.

Außerdem kommen die Isomere der POM in Betracht. So hat die Keggin-Struktur fünf Isomere, die alpha, beta, gamma, delta, und epsilon-Struktur. Des Weiteren kommen Defektstrukturen oder lacunare Strukturen sowie Teilstrukturen in Betracht.

Vorzugsweise werden die Anionen I bis XIII in der Form von Salzen mit Kationen, die für die Reinigung und Körperpflege und die pharmazeutische Anwendung zugelassen sind, angewandt.

Beispiele geeigneter Kationen sind
- einwertige Kationen wie Wasserstoff-, Lithium-, Natrium-, Kalium-, Rubidium-, Cäsium-, Kupfer(I)- und Silberionen;
- zweiwertige Kationen wie Magnesium-, Calcium-, Strontium-, Barium-, Kupfer (II)-, Eisen (II)-, Nickel-, Kobalt-, Zinn (II) und Zinkionen;
- dreiwertige Kationen wie Aluminium-, Eisen (III)-, Lanthanoid- und Actinoidionen;
- vierwertige Kationen wie Blei (IV)-Kationen;
- Mono-, Di-, Tri- oder Tetra-(C₁-C₂₀-alkylammonium) wie Pentadecyldimethylferrocenylmethylammonium, Undecyldimethylferrocenylmethylammonium, Hexadecyltrimethylammonium, Octadecyltrimethylammonium, Didodecyldimethylammonium, Ditetradecyldimethylammonium, Dihexadecyl-dimethylammonium, Dioctadecyldimethylammonium, Dioctadecylviologen, Trioctadecylmethylammonium und Tetrabutylammonium;
- Mono-, Di-, Tri- oder Tetra-(C₁-C₂₀-alkanolammonium) wie Ethanolammonium Diethanolammonium und Triethanolammonium; und
- Monokationen natürlich vorkommender Aminosäuren wie Histidinium (HISH⁺), Argininium (ARGH⁺) oder Lysinium (LYSH⁺) oder Oligo- oder Polypeptide mit einem oder mehreren protonierten basischen Aminosäurerest(en).

### [Vgl. z.B. US 6,020,369, Spalte 3, Zeile 6, bis Spalte 4, Zeile 29]

Es kommen auch natürliche, modifizierte natürliche und synthetische kationische Oligomere und Polymere, d.h., Oligomere und Polymere, die primäre, sekundäre, tertiäre und quartäre Ammoniumgruppen, primäre, sekundäre und tertiäre Sulfoniumgruppen und/oder primäre, sekundäre und tertiäre Phosphoniumgruppen tragen in Betracht. Synthetische Oligomere und Polymere sind üblich und bekannt und werden beispielsweise in Elektrotauchlacken verwendet. Beispiele für natürliche kationische Oligomere und Polymere sind Polyaminoaccharide wie Polyglucosamine wie Chitin, N-Acetylglykoside und insbesondere Chitosan.

Die Kettenlänge und das Molekulargewicht des Chitosans können sehr breit variiert werden. So können Chitosane mit kurzer und langer Kettenlänge und/oder niedrigem und hohem Molekulargewicht verwendet werden. Sie können mit den POM durch chemische Vernetzung, Verstrickung (Entanglement) mit den polymeren Ketten und/oder durch kovalente und/oder ionische Bindungen, durch Wasserstoffbrückenbindungen und/oder durch Van der Waalskräfte und/oder London-Kräfte verbunden sein.

Beispiele geeigneter POM gehen aus der Tabelle 2 hervor.

**Tabelle 2: Summenformeln von geeigneten POM^{a})**

| **Nr.** | **Summenformel** | **Strukturfamilie** |
|---|---|---|
| 1 | [(NMP)₂H]₃PW₁₂O₄₀ | |
| 2 | [(DMA)₂H]₃PMo₁₂O₄₀ | |
| 3 | (NH₄)₁₇Na[NaSb₉W₂₁O₈₆] | **Anorganisches Kryptat** |
| 4 | a- und b-H₅BW₁₂O₄₀ | " |
| 5 | a- und b-H₆ZnW₁₂O₄₀ | " |
| 6 | a- und b-H₆P₂W₈O ₆₂ | " |
| 7 | alpha-(NH₄)₆P₂W₈O₆₂ | **Wells-Dawson-Struktur** |
| 8 | K₁₀Cu₄(H₂O)₂(PW₉O₃₄)₂.20H₂O | " |
| 9 | K₁₀Co₄(H₂O)₂(PW₉O₃₄)₂.20H₂O | " |
| 10 | Na₇PW₁₁O₃₉ | " |
| | Na₇PW₁₁O₃₉.20H₂O + 2 C₆H₆P(O)(OH)₂ | " |
| 11 | [(n-Butyl)₄N]₄H₃PW₁₁O₃₉ | " |
| 12 | b-Na₈HPW₉O₃₄ | " |
| 13 | [(n-Butyl)₄N]₃PMoW₁₁O₃₉ | " |
| 14 | a-[(n-Butyl)₄N]₄Mo8O26 | " |
| 15 | [(n-Butyl)₄N]₂W₆O₁₉ | " |
| 16 | [(n-Butyl)₄N]₂Mo₆O₁₉ | " |
| 17 | a-(NH₄)ₙH₍₄₋ₙ₎SiW₁₂O₄₀ | " |
| 18 | a-(NH₄)ₙH₍₅₋ₙ₎BW₁₂O₄₀ | " |
| 19 | a-K₅BW₁₂0₄₀ | " |
| 20 | K₄W₄O₁₀(O₂)₆ | " |
| 21 | b-Na₉HSiW₉O₃₄ | " |
| 22 | NaₑH₂W₁₂O₄₀ | |
| 23 | (NH₄)₁₄[NaP₅W₃₀O₁₁₀] | **Preyssler-Struktur** |
| 24 | a-(NH₄)₆BW₂O₄₀ | " |
| 25 | a-Na₅BW₁₂O₄₀ | " |
| 26 | (NH₄)₄W₀O₃₂ | " |
| "27 | (Me₄N)₄W₁₀O₃₂ | " |
| 28 | (HISH⁺), H₍₅₋ₙ₎BW₁₂O₄₀ | " |
| 29 | (LYSH⁺)ₙH₍₅₋ₙ₎BW₁₂O₄₀ | " |
| 30 | (ARGH⁺)ₙH₍₅₋ₙ₎BW₁₂O₄₀ | " |
| 31 | (HISH⁺)ₙH₍₄₋ₙ₎SiW₁₂O₄₀ | " |
| 32 | (LYSH⁺)ₙH₍₄₋ₙ₎SiW₁₂O₄₀ | " |
| 34 | (ARGH⁺)ₙH₍₄₋ₙ₎SiW₁₂O₄₀ | " |
| 35 | K₁₂[EuP₅W₃₀O₁₁₀].22H₂O^{b)} | " |
| 36 | a-K₈SiW₁₁O₃₉ | " |
| 37 | K₁₀(H₂W₁₂O₄₂) | " |
| 38 | Ki₂Ni₃(II)(PW₉O₃₄)₂.nH₂O | " |
| 39 | (NH₄)₁₀Co₄(II)(PW₅O₃₄)₂.nH₂O | " |
| 40 | K₁₂Pd₃(II)(PW₉O₃₄)₂.nH₂O | " |
| 41 | Na₁₂P₂W₁₅O₅₆.18H₂O | **Lacunare (defekte) Struktur** |
| 42 | Na₁₆Cu₄(H₂O)₂(P₂W₁₅O₅₆)₂.nH₂O | " |
| 43 | Na₁₆Zn₄(H₂O)₂(P₂W₁₅O₅₆)₂.nH₂O | " |
| 44 | Na₁₆Co₄(H₂O)₂(P₂W₁₅O₅₆)₂.nH₂O | " |
| 45 | Na₁₆N₁₄(H₂O)₂(P₂W₁₅O₅₆)₂. nH₂O | **Wells-Dawson-Sandwich-Struktur** |
| 46 | Na₁₆Mn₄(H₂O)₂(P₂W₁₅O₅₆)₂.nH₂O | " |
| 47 | Na₁₆Fe₄(H₂O)₂(P₂W₁₅O₅₆)₂.nH₂O | " |
| 48 | K₁₀Zn₄(H₂O)₂(PW₉O₃₄)₂.20H₂O | **Keggin-Sandwich-Struktur** |
| 49 | K₁₀Ni₄(H₂O)₂(PW₉O₃₄)₂.nH₂O | " |
| 50 | K₁₀Mn₄(H₂O)₂(PW₉O₃₄)₂.nH₂O | " |
| 51 | K₁₀Fe₄(H₂O)₂(PW₉O₃₄)₂.nH₂O | " |
| 52 | Ki₂Cu₃(PW₉O₃₄)₂.nH₂O | " |
| 53 | K₁₂(CoH₂O)₃(PW₉O₃₄)₂.nH₂O | " |
| 54 | K₁₂Zn₃(PN₉O₃₄)₂.15H₂O | " |
| 55 | K₁₂Mn₃(PW₉O₃₄)₂.15H₂O | " |
| 56 | K₁₂Fe₃(PW₉O₃₄)₂.25H₂O | " |
| 57 | (ARGH⁺)₁₀(NH₄ₕNa[NaSb₉W₂₁O₈₆] | " |
| 58 | (ARGH⁺)₅HW₁₁O₃₉.17H₂O | " |
| 59 | K₇Ti₂W₁₀O₄₀ | " |
| 60 | [(CH₃)₄N]₇Ti₂W₁₀O₄₀ | " |
| 61 | Cs₇Ti₂W₁₀O₄₀ | " |
| 62 | [HISH']₇Ti₂W₁₀O₄₀ | " |
| 63 | [LYSH⁺]ₙNa₇₋ₙPTi₂W₁₀O₄₀ | " |
| 64 | [ARGH⁺]ₙNa₇₋ₙPTi₂W₁₀O₄₀ | " |
| 65 | [n-Butyl₄N⁺]₃H₃V₁₀O₂₈ | " |
| 66 | K₇HNb₆O₁₉.13H2O | " |
| 67 | [(CH₃)₄N⁺]₄SiW₁₁O₃₉- | **Organisch modifizierte Struktur** |
| | O[SiCH₂CH₂C(O)OCH₃]₂ | |
| 68 | [(CH₃)₄N⁺]₄PW₁₁O₃₉-(SiCH₂CH₂CH₂CN) | " |
| 69 | [(CH₃)₄N⁺]₄PW₁₁O₃₉-(SiCH₂CH₂CH₂Cl) | " |
| 70 | [(CH₃)₄N⁺]₄PW₁₁O₃₉-(SiCH₂=CH₂) | " |
| 71 | Cs₄[SiW11O₃₉-(SiCH₂CH₂C(O)OCH₃)₂]₄ | " |
| 72 | Cs₄[SiW11O₃₉-(SiCH₂CH₂CH₂CN)]₄ | " |
| 73 | Cs₄[SiW11O₃₉-(SiCH₂CH₂CH₂Cl)₂]₄ | " |
| 74 | Cs₄[SiW11O₃₉-(SiCH₂=CH₂)]₄ | " |
| 75 | [(CH₃)₄N+]₄SiW₁₁O₃₉-O- | " |
| | (SiCH₂CH₂CH₂Cl)₂ | |
| 76 | [(CH₃)₄N+]₄SiW₁₁O₃₉- | " |
| | O(SiCH₂CH₂CH₂CN)₂ | |
| 77 | [(CH₃)₄N+]₄SiW₁₁O₃₉-O(SiCH₂=CH₂)₂ | " |
| 78 | [(CH₃)₄N+]₄SiW₁₁O₃₉-O[SiC(CH₃)]₂ | " |
| 79 | [(CH₃)ₐN⁺]₄SiW₁₁O₃₉-O[SiCH₂CH(CH₃)]₂ | " |
| 80 | [(CH₃)₄N+]₄SiW₁₁O₃₉- | " |
| | O[SiCH₂CH₂C(O)OCH₃]₂ | |
| 81 | K₅Mn(II)PW₁₁O₃₉.nH₂O | **Mit Übergangsmetallen substituierte Struktur** |
| 82 | K₈Mn(II)P₂W₁₇O₆₁.nH₂O | " |
| 83 | K₆Mn(II)SiW₁₁O₃₉.nH₂O | " |
| 84 | K₅PW₁₁O₃₉[Si(CH₃)₂].nH₂O | " |
| 85 | K₃PW₁₁O₄₁(PC₆H₅)₂.nH₂O | " |
| 86 | Na₃PW₁₁O₄₁(PC₆H₅)₂.nH₂O | " |
| 87 | K₅PTiW₁₁O₄₀ | " |
| 88 | Cs₅PTiW₁₁O₃₉ | " |
| 89 | K₆SiW₁₁O₃₉[Si(CH₃)₂].nH₂O | " |
| 90 | KSiW₁₁O₃₉[Si(C₆H₅)(tert.-C₄H₉)].nH₂O | " |
| 91 | K₆SiW₁₁O₃₉[Si(C₆H₅)₂].nH₂O | " |
| 92 | K₇SiW₉Nb₃O₄₀.nH₂O | " |
| 93 | Cs₇SiW₉Nb₃O₄₀.nH₂O | " |
| 94 | Cs₈Si₂W₁₈Nb₆O₇₇.nH₂O | " |
| 95 | [(CH₃)₃NH⁺]₇SiW₉Nb₃O₄₀.nH₂O | **Substituierte Keggin-Struktur** |
| 96 | (CN₃H₆)₇SiW₉Nb₃O₄₀.nH₂O | " |
| 97 | (CN₃H₆)₈Si₂W₁₈Nb₆O₇₇.nH₂O | " |
| 98 | Rb₇SiW₉Nb₃O₄₀.nH₂O | " |
| 99 | Rb₈Si₂W₁₈Nb₆O₇₇.nH₂O | " |
| 100 | K₈Si₂W₁₈Nb₆O₇₇.nH₂O | " |
| 101 | K₆P₂Mo₁₈O₆₂.nH₂O | " |
| 102 | (C₅H₅N)₇HSi₂W₁₈Nb₆O₇₇.nH₂O | " |
| 103 | (C₅H₅N)₇SiW₉Nb₃O₄₀.nH₂O | " |
| 104 | (ARGH⁺)₈SiW₁₈Nb₆O₇₇.18H₂O | " |
| 105 | (LYSH⁺)₇KSiW₁₈Nb₆O₇₇.18H₂O | " |
| 106 | (HISH⁺)₆K₂SiW₁₈Nb₆O₇₇.18H₂O | " |
| 107 | [(CH₃)₄N⁺]₄SiW₁₁O₃₉-O(SiCH₂CH₃)₂ | " |
| 108 | [(CH₃)₄N⁺]₄SiW₁₁O₃₉-O(SiCH₃)₂ | " |
| 109 | [(CH₃)₄N⁺]₄SiW₁₁O₃₉-O(SiC₁₆H₃₃)₂ | " |
| 110 | Li₉P₂V₃(CH₃)₃W₁₂O₆₂ | " |
| 111 | Li₇HSi₂W₁₈Nb₆O₇₇ | " |
| 112 | Cs₉P₂V₃CH₃W₁₂O₆₂ | " |
| 113 | Cs₁₂P₂V₃W₁₂O₆₂ | " |
| 114 | K₄H₂PV₄W₈O₄₀ | " |
| 115 | Na₁₂P₄W₁₄O₅₈ | " |
| 116 | Na₁₄H₆P₆W₁₈O₇₉ | " |
| 117 | a-K₅(NbO₂)SiW₁₁O₃₉ | " |
| 118 | aO₂)SiW₁₁O₃₉ | " |
| 119 | [(CH₃)₃NH⁺)₅NbSiW₁₁O₄₀ | " |
| 120 | [(CH₃)₃NH⁺]₅TaSiW₁₁O₄₀ | " |
| 121 | K₆Nb₃PW₉O₄₀ | **Peroxo-Keggin-Struktur** |
| 122 | [(CH₃)₃NH⁺]₅(NbO₂)SiW₁₁O₃₉ | " |
| 123 | [(CH₃)₃NH⁺]₅(TaO₂)SiW₁₁O₃₉ | " |
| 124 | K₄(NbO₂)PW₁₁O₃₉ | " |
| 125 | K₇(NbO₂)P₂W₁₂O₆₁ | " |
| 126 | [(CH₃)₃NH⁺]₇(NbO₂)₃SiW₉O₃₇ | " |
| 127 | Cs₇(NbO₂)₃SiW₉O₃₇ | " |
| 128 | K₆(NbO₂)₃PW₉O₃₇ | " |
| 129 | Na₁₀(H₂W₁₂O₄₂) | " |
| 130 | K₄NbPW₁₁O₄₀ | " |
| 131 | [(CH₃)₃NH₊]₄NbPW₁₁O₄₀ | " |
| 132 | K₅NbSiW₁₁O₄₀ | " |
| 133 | K₅TaSiW₁₁O₄₀ | " |
| 134 | K₇NbP₂W₁₇O₆₂ | **Wells-Dawson-Struktur** |
| 135 | K₇(TiO₂)₂PW₁₀O₃₈ | " |
| 136 | K₇(TaO₂)₃SiW₉O₃₇ | " |
| 137 | K₇Ta₃SiW₉O₄₀ | " |
| 138 | K₆(TaO₂)₃PW₉O₃₇ | " |
| 139 | K₆Ta₃PW₉O₄₀ | " |
| 140 | K₈Co₂W₁₁O₃₉ | " |
| 141 | H₂[(CH₃)₄N⁺]4(C₂H₅Si)₂CoW₁₁O₄₀ | " |
| 142 | H₂[(CH₃)₄N⁺]₄(iso-C₄H₉Si)₂CoW₁₁O₄₀ | " |
| 143 | K₉Nb₃P₂W₁₅O₆₂ | " |
| 144 | K₉(NbO₂)₃P₂W₁₅O₅₉ | " |
| 145 | K₁₂(NbO₂)₆P₂W₁₂O₅₆ | **Wells-Dawson-Peroxostruktur** |
| 146 | K₁₂Nb₆P₂W₁₂O₆₂ | **Wells-Dawson-Struktur ff.** |
| 147 | a₂-K₁₀P₂W₁₇O₆₁ | " |
| 148 | K₆Fe(III)Nb₃P₂W₁₅O₆₂ | " |
| 149 | K₇Zn(II)Nb₃P₂W₁₅O₆₂ | " |
| 150 | (NH₄)₆(a-P₂W₁₈O₆₂).nH₂O | " |
| 151 | K₁₂[H₂P₂W₁₂O₄₈].24H₂O | " |
| 152 | K₂Na₁₅H₅[PtMo₆O₂₄].8H₂O | " |
| 153 | K₈[a₂-P₂W₁₇MoO₆₂].nH₂O | " |
| 154 | KHP₂V₃W₁₅O₆₂.34H₂O | " |
| 155 | K₆[P₂W₁₂Nb₆O₆₂].24H₂O | " |
| 156 | Na₆[V₁₀O₂₈].H₂O | " |
| 157 | (Guanidinium)₈H[PV₁₄O₆₂].3H₂O | " |
| 158 | K8H[PV14O62] | " |
| 159 | Na₇[MnV₁₃O₃₈].18H₂O | " |
| 160 | K₆[BW₁₁O₃₉Ga(OH)₂].13H₂O | " |
| 161 | K₇H[Nb₆O₁₉].13H₂O | " |
| 162 | [(CH₃)₄N⁺/Na⁺/K⁺]₄[Nb₂W₄O₁₉] | " |
| 163 | [(CH₃)₄N⁺]₉[P₂W₁₅Nb₃O₆₂] | " |
| 164 | [(CH₃)₄N⁺]₁₅[HP₄W₃₀Nb₆O₁₂₃].16H₂O | " |
| 165 | [Na/K]₆[Nb₄W₂O₁₉] | " |
| 166 | [(CH₃)₄N⁺/Na⁺/K⁺]5[_{Nb3W3O19]}.6H₂O | " |
| 167 | K₅[CpTiSiW₁₁O₃₉].12H₂O | " |
| 169 | b₂-K₈[SiW₁₁O₃₉].14H₂O | " |
| 170 | a-K₈[SiW₁₀O₃₆].12H₂O | " |
| 171 | Cs₇Na₂[PW₁₀O₃₇].8H₂O | " |
| 172 | Cs₆[P₂W₅O₂₃].7,5H₂O | " |
| 173 | g-Cs₇[PW₁₀O₃₆].7H₂O | " |
| 174 | K₅[SiNbW₁₁O₄₀].7H₂O | " |
| 175 | K₄[PNbW₁₁O₄₀].12H₂O | " |
| 176 | Na₆[Nb₄W₂O₁₉].13H₂O | " |
| 177 | K₆[Nb₄W₂O₁₉].7H₂O | " |
| 180 | K₄[V₂W₄O₁₉].3,5H₂O | " |
| 181 | Na₅[V₃W₃O₁₉].12H₂O | " |
| 182 | K₆[PV₃W₉O₄₀].14H₂O | " |
| 183 | Na₉[A-b-GeW₉O₃₄].8H₂O | " |
| 184 | Na₁₀[A-a-GeW₉O₃₄].9H₂O | " |
| 185 | K₇[BV₂W₁₀O₄₀].6H₂O | " |
| 186 | Na₅[CH₃Sn(Nb₆O₁₉)].10H₂O | " |
| 187 | Na₈[Pt(P(m-SO₃C₆H₅)₃)₃Cl].3H₂O | " |
| 188 | [(CH₃)₃NH⁺]₁₀(H)[Si (H)₃W₁₈O₆₈].10H₂O | " |
| 189 | K₇[A-a-GeNb₃W₉O₄₀].18H₂O | " |
| 190 | K₇[A-b-SiNb₃W₉O₄₀].20H₂O | " |
| Brauchen | [(CH₃)₃NH⁺]₉[A-a-HGe₂Nb₆W₁₈O₇₈ | " |
| 191 | | |
| 192 | K₇(H)[A-a-Ge₂Nb₆W₁₈O₇₇].18H₂O | " |
| 193 | K₈[A-b-Si₂Nb₆W₁₈O₇₇] | " |
| 194 | [(CH₃)₃NH⁺]₈[A-B-Si₂Nb₆W₁₈O₇₇] | " |

a) vgl. US 6,020,369, TABLE 1, Spalten 3 bis 10;
b) Tierui Zhang, Shaoquin Liu, Dirk G. Kurth und Charl F. J. Faul, »Organized Nanostructured Complexes of Polyoxometalates and Surfactants that Exhibit Photoluminescence and Electrochromism, Advanced Functional Materials, 2009, 19, Seiten 642 bis 652;
n Zahl, insbesondere ganze Zahl, von 1 bis 50.

Weitere Beispiele geeigneter POM sind aus dem amerikanischen Patent US 7,097,858 B2, Spalte 14, Zeile 56, bis Spalte 17, Zeile 19, sowie aus TABLE 8a, Spalte 22, Zeile 41, bis Spalte 23, Zeile 28, Verbindungen Nummer 1-53, und TABLE 8b, Spalte 23, Zeile 30, bis Spalte 25, Zeile 34, Verbindungen Nummer 1 bis 150, bekannt.

Weitere bevorzugte POM, sind die von J. T.Rhule, C. L. Hill und D. A. Judd in dem Artikel »Polyoxometalates in Medicine« in Chemical Reviews, Band 98, Seiten 327 bis 357, in Tabelle 1 »In Vitro Antiviral Activities of Polyoxometalates«, Seiten 332 bis 347, und in Tabelle 2 »In Vivo Activities of Polyoxometalates«, Seite 351, aufgeführten Polyoxometallate, die auf ihre antivirale Aktivität getestet worden sind.

Bevorzugt werden POM mit Keggin-Struktur, Doppel-Keggin-Struktur und Wells-Dawson-Struktur verwendet.

### Ganz besonders bevorzugt werden

- Ammoniumheptamolybdat-Tetrahydrat {(NH₄)₆Mo₇O₂₄] · 4H₂O, CAS-Nr.13106-76-8 (wasserfrei), CAS-Nr. 12054-85-2 (Tetrahydrat), **AHMT**},
- Wolframatophosphorsäure-Hydrat {H₃[P(W₃O₁₀)₄] · xH₂O, CAS-Nr. 1343-93-7 (wasserfrei), CAS-Nr. 12067-99-1 (Hydrat), **Wo-Pho**},
- Molybdatophosphorsäure-Hydrat, {H₃P(Mo₃O₁₀)₄] · xH2O, CAS-Nr.:12026-57-2 (wasserfrei), CAS-Nr. 51429-74-4 (Hydrat), **Mo-Pho**} und/oder
- Wolframatokieselsäure {H₄[Si(W₃O₁₀)₄] · xH₂O, CAS-Nr. 12027-43-9, CAS-Nr. WKS}
und/oder ihre Salze verwendet.

In einer besonders bevorzugten Ausführungsform wird ein POM-Chitosan-Komposit verwendet.

Die vorstehend beschriebenen POM-Mikropartikel sind unverändert, sauerstoffersetzt, funktionalisiert, aggregiert, und/oder agglomeriert. Beispielsweise können sie funktionalisiert und agglomeriert sein. Sie können aber auch nicht funktionalisiert und aggregiert sein.

Die erfindungsgemäß zu verwendenden POM-Mikropartikel können mithilfe üblicher und bekannter nasschemischer Verfahren wie zum Beispiel Fällungsverfahren hergestellt werden. Es ist aber auch möglich, die POM in Wasser aufzulösen und die resultierende Lösung gegen einen warmen Luftstrom zu sprühen. Außerdem ist es möglich, die Lösung im Vakuum einzudampfen, wobei sie mit IR-Strahlung bestrahlt wird. Des Weiteren ist es möglich, Lösungen, insbesondere wässrige Lösungen, von POM auf kalte Oberflächen, wie tiefgekühlte, glatte Metalloberflächen, Trockeneis, tiefgekühlte organische Lösungsmittel und verflüssigte Gase, wie Methan, Ethan Propan, Butan, Methylcyclohexan oder Benzine, flüssigen Stickstoff oder flüssiges Helium aufzusprühen und das Trockeneis oder die flüssigen Substanzen zu verdampfen.

Weitere geeignete Bakterizide und/oder Viruzide sind ionische Flüssigkeiten. Sie bestehen ausschließlich aus Ionen (Kationen und Anionen). Dabei können sie aus organischen Kationen sowie organischen oder anorganischen Anionen oder aus anorganischen Kationen und organischen Anionen bestehen.

Prinzipiell sind ionische Flüssigkeiten Salzschmelzen mit niedrigem Schmelzpunkt. Man rechnet nicht nur die bei der Umgebungstemperatur flüssigen, sondern auch alle Salzverbindungen dazu, die vorzugsweise unter 150°C, bevorzugt unter 130°C und insbesondere unter 100°C schmelzen. Im Gegensatz zu herkömmlichen anorganischen Salzen wie Kochsalz (Schmelzpunkt 808°C) sind bei ionischen Flüssigkeiten durch Ladungsdelokalisierung die Gitterenergie und die Symmetrie verringert, was zur Erstarrungspunkten bis zu -80°C und darunter führen kann. Aufgrund der zahlreichen Kombinationsmöglichkeiten von Anionen und Kationen lassen sich ionische Flüssigkeiten mit sehr unterschiedlichen Eigenschaften herstellen (vgl. a. Römpp Online 2007, »ionische Flüssigkeiten«).

Als organische Kationen kommen alle Kationen in Betracht, wie sie üblicherweise in ionischen Flüssigkeiten verwendet werden. Vorzugsweise handelt es sich um nicht cyclische oder heterocyclische Oniumverbindungen.

Bevorzugt werden nicht cyclische und heterocyclische Oniumverbindungen aus der Gruppe, bestehend aus quartären Ammonium-, Oxonium-, Sulfonium- und Phosphonium-Kationen sowie aus Uronium-, Thiouronium- und Guanidinium-Kationen, bei denen die einfach positive Ladung über mehrere Heteroatome delokalisiert ist, verwendet.

Besonders bevorzugt werden quartäre Ammonium-Kationen und ganz besonders bevorzugt heterocyclische quartäre Ammonium-Kationen verwendet.

Insbesondere werden die heterocyclischen quartären Ammonium-Kationen aus der Gruppe, bestehend aus Pyrrolium-, Imidazolium-, 1H-Pyrazolium-, 3H-Pyrazolium-, 4H-Pyrazolium-, 1-Pyrazolinium-, 2-Pyrazolinium-, 3-Pyrazolinium-, 2,3-Dihydro-imidazolinium-, 4,5-Dihydro-imidazolinium-, 2,5-Dihydro-imidazolinium-, Pyrrolidinium-, 1,2,4-Triazolium- (quartäres Stickstoffatom in 1-Stellung), 1,2,4-Triazolium- (quartäres Stickstoffatom in 4-Stellung), 1,2,3-Triazolium- (quartäres Stickstoffatom in 1-Stellung), 1,2,3-Triazolium- (quartäres Stickstoffatom in 4-Stellung), Oxazolium-, Isooxazolium-, Thiazolium-, Isothiazolium-, Pyridinium-, Pyridazinium-, Pyrimidinium-, Piperidinium-, Morpholinium-, Pyrazinium-, Indolium-, Chinolinium-, Isochinolinium-, Chinoxalinium- und Indolinium-Kationen, ausgewählt.

Die vorstehend beschriebenen organischen Kationen sind an sich bekannte Spezies, die beispielsweise in den deutschen und internationalen Patentanmeldungen sowie in der amerikanischen Patentanmeldung
- DE 10 2005 055 815 A, Seite 6, Absatz [0033], bis Seite 15, Absatz [0074],
- DE 10 2005 035 103 A1, Seite 3, Absatz [0014], bis Seite 10, Absatz [0051], und
- DE 103 25 050 A1, der die Seiten 2 und 3 übergreifende Absatz [0006] in Verbindung mit Seite 3, Absatz [0011], bis Seite 5, Absatz [0020],
- WO 03/029329 A2, Seite 4, letzter Absatz, bis Seite 8, zweiter Absatz,
- WO 2004/052340 A1, Seite 8, erster Absatz, bis Seite 10, erster Absatz,
- WO 2004/084627 A2, Seite 14, zweiter Absatz, bis Seite 16, erster Absatz, und Seite 17, erster Absatz, bis Seite 19, zweiter Absatz,
- WO 2005/017252 A1, Seite 11, Zeile 20, bis Seite 12, Zeile 19,
- WO 2005/017001 A1, Seite 7, letzter Absatz, bis Seite 9, viertletzter Absatz,
- WO 2005/023873 A1, Seite 9, Zeile 7, bis Seite 10, Zeile 20,
- WO 2006/116126 A2, Seite 4, Zeile 1, bis Seite 5, Zeile 24,
- WO 2007/057253 A2, Seite 4, Zeile 24, bis Seite 18, Zeile 38,
- WO 2007/085624 A1, Seite 14, Zeile 27, bis Seite 18, Zeile 11, und
- US 2007/0006774 A1 Seite 17, Absatz [0157], bis Seite 19, Absatz [0167],

im Detail beschrieben werden. Auf die aufgeführten Passagen der Patentanmeldungen wird zu Zwecken der näheren Erläuterung der vorliegenden Erfindung ausdrücklich Bezug genommen.

Von den vorstehend beschriebenen organischen Kationen werden vor allem Imidazolium-Kationen, insbesondere das 1-Ethyl-3-methylimidazolium-Kation (EMIM) oder das 1-Butyl-3-methylimidazolium-Kation (BMIM), worin sich der quartäre Stickstoff jeweils in 1-Stellung befindet, verwendet.

Als anorganische Kationen kommen alle Kationen in Betracht, die mit den organischen Anionen der ionischen Flüssigkeiten (C) keine kristallinen Salze bilden, deren Schmelzpunkt oberhalb 150°C liegt. Beispiele geeigneter anorganischer Kationen sind die Kationen der Lanthanide.

Als anorganische Anionen kommen im Grunde alle Anionen in Betracht, die mit den organischen Kationen der ionischen Flüssigkeiten (C) keine kristallinen Salze bilden, deren Schmelzpunkt oberhalb 150°C liegt, und die auch keine unerwünschten Wechselwirkungen mit den organischen Kationen, wie chemische Reaktionen, eingehen.

Vorzugsweise werden die anorganischen Anionen aus der Gruppe, bestehend aus Halogenid-, Pseudohalogenid-, Sulfid-, Halometallat-, Cyanometallat-, Carbonylmetallat-, Haloborat-, Halophosphat-, Haloarsenat- und Haloantimonatanionen sowie den Anionen der Sauerstoffsäuren der Halogenide, des Schwefels, des Stickstoffs, des Phosphors, des Kohlenstoffs, des Siliziums, des Bors und der Übergangsmetalle, ausgewählt.

Bevorzugt werden als Halogenidanionen Fluorid-, Chlorid-, Bromid- und/oder Iodidionen, als Pseudohalogenidanionen Cyanid-, Cyanat-, Thiocyanat-, Isothiocyanat- und/oder Azidanionen, als Sulfidanionen Sulfid-, Hydrogensulfid-, Polysulfid- und/oder Hydrogenpolysulfidanionen, als Halometallatanionen Chlor- und/oder Bromaluminate und/oder -ferrate, als Cyanometallatanionen Hexacyanoferrat(II)- und/oder -(III)-Anionen, als Carbonylmetallatanionen Tetracarbonylferratanionen, als Haloboratanionen Tetrachlor- und/oder Tetrafluoroboratanionen, als Halophosphat-, Haloarsenat- und Haloantimonatanionen Hexafluorphosphat-, Hexafluorarsenat-, Hexachlorantimonat- und/oder Hexafluorantimonatanionen sowie als Anionen der Sauerstoffsäuren der Halogenide, des Schwefels, des Stickstoffs, des Phosphors, des Kohlenstoffs, des Siliziums, des Bors und der Übergangsmetalle Chlorat-, Perchlorat-, Bromat-, lodat-, Sulfat-, Hydrogensulfat-, Sulfit-, Hydrogensulfit-, Thiosulfat-, Nitrit-, Nitrat-, Phosphinat-, Phosphonat-, Phosphat-, Hydrogenphosphat-, Dihydrogenphosphat-, Carbonat-, Hydrogencarbonat-, Glyoxylat-, Oxalat-, Deltaat-, Quadrat-, Krokonat-, Rhodizonat-, Silikat-, Borat-, Chromat-, Peroxometallat- und/oder Permanganatanionen verwendet.

Besonders bevorzugt werden die vorstehend aufgeführten POM-Anionen verwendet.

In gleicher Weise kommen als organische Anionen kommen im Grunde alle Anionen in Betracht, die mit den organischen oder anorganischen Kationen der ionischen Flüssigkeiten keine kristallinen Salze bilden, deren Schmelzpunkt oberhalb 150°C, liegt und die auch keine unerwünschten Wechselwirkungen mit den organischen oder anorganischen Kationen, wie chemische Reaktionen eingehen.

Vorzugsweise leiten sich die organischen Anionen von aliphatischen, cycloaliphatischen und aromatischen Säuren aus der Gruppe, bestehend aus Carbonsäuren, Sulfonsäuren, sauren Sulfatestern, Phosphonsäuren, Phosphinsäuren, sauren Phosphatestern, Hypodiphosphinsäuren, Hypodiphosphonsäuren, Hypodiphosphonsäuren, sauren Borsäureestern, Borsäuren, sauren Kieselsäureestern und sauren Silanen, ab oder sie werden aus der Gruppe, bestehend aus aliphatischen, cycloaliphatischen und aromatischen Thiolat-, Alkoholat-, Phenolat-, Methid-, Bis(carbonyl)imid-, Bis(sulfonyl)imid- und Carbonylsulfonylimidanionen, ausgewählt.

Beispiele geeigneter anorganischer und organischer Anionen sind aus den internationalen Patentanmeldungen
- WO 2005/017252 A1, Seite 7, Seite 14, bis Seite 11, Seite 6, und
- WO 2007/057235 A2, Seite 19, Zeile 5, bis Seite 23, Seite 23,
bekannt.

Insbesondere wird 1-Ethyl-3-methylimidazolium-acetat (EMIM Ac) und Tetra-(1-ethyl-3-methylimidazolium)-wolframatosilikat als ionische Flüssigkeit verwendet.

Als partikuläre, anorganische, bakterizide und/oder biozide Materialien werden vorzugsweise Materialien verwendet, die in Wasser nur schlecht löslich sind, sodass die Materialien nicht durch Aerosole angegriffen werden. Außerdem sollen sie für Mensch und Tier nicht hoch toxisch und karzinogen sein und sicher zu handhaben und zu entsorgen sein.

Beispiele geeigneter partikulärer, anorganischer, bakterizider und/oder biozider Materialien sind Borsäure, Boroxid, Dinatriumoctaborattetrahydrat, Dinatriumtetraboratpentahydrat, Dinatriumtetraboratdecahydrat, Kupferhydroxyd, basisches Kupfercarbonat, granuliertes Kupfer, Kupferflocken, Dikupferoxid, Kupferoxid, Kupferthiocyanat, Silber, Silberchlorid, Silberbromid, Calciumcarbonat, Kalkhydrat, Kalk, Calcium-Magnesiumoxid-Tetrahydrat, Calcium-MagnesiumOxid und/oder Titandioxid.

Die bakteriziden und/oder viruziden Beschichtungen auf den vorstehend beschriebenen Trägermaterialien, enthaltend die vorstehend beschriebenen Biozide, werden vorzugsweise aus flüssigen oder pulverförmigen Beschichtungsstoffen hergestellt. Dabei werden Beschichtungsstoff auf Wasserbasis oder auf der Basis organischer Lösemittel verwendet. Die flüssigen Beschichtungsstoffe können molekulardispers gelöste Stoffe enthalten oder sie können vorzugsweise Dispersionen sein.

Die pulverförmigen Beschichtungsstoffe werden mithilfe üblicher und bekannter Verfahren wie elektrostatisches Sprühen, Wirbelbettbeschichtung und Coil-Coating-Verfahren und nachträglichen Aufschmelzen appliziert. Dabei ist es von Vorteil, wenn die Substrate thermisch stabil sind, sodass sie nicht durch die Erwärmung geschädigt werden. Es ist ein weiterer Vorteil der Pulverbeschichtung, dass die vorstehend beschriebenen festen Biozide und/oder Viruzide gleichzeitig mit den Pulvern gesprüht werden können. Dadurch sind die Mikropartikel an der Oberfläche der Pulverbeschichtung konzentriert, wo sie fest an der aushärtenden Beschichtung kleben und so für die Eliminierung von Bakterien und Viren zur Verfügung stehen.

Die flüssigen Beschichtungsmittel oder Beschichtungsstoffe können mithilfe üblicher und bekannter Beschichtungsverfahren wie Tauchverfahren, Sprühverfahren, insbesondere Druckluftsprühen, luftfreies Sprühen, Air-Mix-Sprühen, Streichen, Heißsprühen, Vorhanggießen, Rakeln, Zweikomponentenbeschichtung, elektrostatisches Sprühen, elektrostatisch unterstütztes Sprühen, Rollerapplikation, Wischen, Giesen, Abkellen, Streifenbeschichtung, Zentrifugieren und Trommelbeschichtung appliziert werden.

Die vorstehend beschriebenen festen Biozide können als feste Mikropartikel in den flüssigen Beschichtungsstoffen dispergiert sein. Es ist jedoch von Vorteil, wenn ein Teil oder die Gesamtmenge der Mikropartikel auf die beschichteten Trägermaterialien appliziert werden, bevor die flüssigen Beschichtungsstoffe aushärten. Dadurch entstehen leicht zugängliche viruzide und/oder bakterizide Zentren, die außerdem noch als Abstandshalter dienen können und so das Verkleben der beschichteten Trägermaterialien verhindern.

Im Falle von thermoplastischen Pulverbeschichtungsstoffen werden die applizierten Beschichtungsstoffe physikalisch gehärtet. D. h., sie verfestigen sich beim Abkühlen.

Je nachdem welche reaktive funktionelle Gruppen in den Beschichtungsstoffen vorhanden sind, werden Letztere thermisch oder durch radikalische Polymerization, die durch aktinische Strahlung oder durch radikalische Initiatoren in Gang gesetzt wird, ausgehärtet. Es können aber auch beide Mechanismen in einem Beschichtungsstoff kombiniert werden. Man spricht dann von "Dual Cure". Die Tabelle 3 gibt einen Überblick über geeignete funktionelle Gruppen für die Vernetzungsreaktionen.

**Tabelle 3: Beispiele von komplementären reaktiven funktionellen Gruppen für die thermische Härtung und die Strahlenhärtung**

| **Bindemittel** | **und** | **Vernetzunqsmittel** |
|---|---|---|
| | ***oder*** | |

| **Vernetzunqsmittel** | **und** | **Bindemittel** |
|---|---|---|
| -SH | | -C(O)-OH |
| -OH | | -C(O)-O-C(O)- |
| | | -NH-C(O)-OR¹ |
| | -CH₂-OH | |
| | -CH₂-O-CH₃ | |
| | -NH-C(O)-CH(-C(O)OR¹)₂ | |
| | -NH-C(O)-CH(-C(O)OR¹)(-C(O)-R¹) | |
| | >Si(OR¹)₂ | |
| -C(O)-OH | O | |
| | -CH-CH₂ | |
| -O-C(O)-CR⁵=CH₂ | -OH | |
| -O-CR=CH₂ | -C(O)-CH₂-C(O)-R¹ | |
| -O-CR=CH₂ | -CH=CH₂ | |
| -CR=CH₂ | -CH=CH₂ | |

Bei einem bevorzugten Verfahren zur Beschichtung der vorstehend beschriebenen, partikulären, anorganischen, inerten Trägermaterialien wird eine Anlage verwendet, die eine automatische Zuführung der partikulären Trägermaterialien auf ein über mindestens zwei Transportrollen geführtes Endlosband mit einer Rüttelstrecke umfasst. Die zugeführten partikulären Trägermaterialien werden gerüttelt und in einer ersten Station mit mindestens einem der nachstehend beschriebenen flüssigen Beschichtungsstoffe besprüht. Durch das Rütteln wird sichergestellt, dass die Trägermaterialien möglichst gleichmäßig beschichtet werden. Bevor die Beschichtungsstoffe ausgehärtet werden, werden sie in einer zweiten Station unter Rütteln mit Mikropartikeln mindestens einer Art von Biozid besprüht, sodass die Mikropartikel auf und in den Beschichtungsstoffen gleichmäßig verteilt werden und kleben bleiben. In einer dritten Station werden die Beschichtungsstoffe - ebenfalls unter Rütteln - thermisch in einem Durchlaufofen, mit IR-Strahlern und/oder mit UV-Strahlung gehärtet, sodass sich die biozide Beschichtung auf den Trägermaterialien bildet. Durch das Rütteln wird verhindert, dass die Partikel nicht zusammenkleben und die ausgehärteten Beschichtungsstoffe keine fest anhaftenden Filme auf dem Endlosband bilden. Sofern dies doch in einem gewissen Umfang geschieht, werden die Partikel in einer dritten Station vereinzelt und danach in ein Vorratsgefäß ausgetragen. Die an dem Endlosband anhaftenden Reste von ausgehärteten Beschichtungsstoffen werden nach dem Umlenken des Endlosbands mit Rakeln abgekratzt.

Die Beschichtungsmittel oder Beschichtungsstoffe können anhand ihrer Bindemittel in Gruppen eingeteilt werden.

### Beschichtungsstoffe auf der Basis von Ölen

Diese Beschichtungsstoffe enthalten natürliche, trocknende Öle, die eine autooxidative Polymerisation in der Gegenwart von Katalysatoren und atmosphärischem Sauerstoff eingehen. Chemisch modifizierte oxidativ trocknende Öle wie Polyurethanöle oder niedermolekulare Polybutadienöle können ebenfalls verwendet werden.

### Beschichtungsstoffe auf der Basis von Cellulose

Nitrozelluloselacke enthalten üblicherweise zusätzliche Harze und Lösemittel, um ihre anwendungstechnischen Eigenschaften zu verbessern. Weitere Beschichtungsstoffe auf der Basis von Cellulose enthalten organische Celluloseester wie Celluloseacetat, Celluloseacetatbutyrat oder Celluloseacetatpropionat.

### Beschichtungsstoffe auf der Basis von Kautschuk

Diese Beschichtungsstoffe auf der Basis von chloriertem Kautschuk, chlorierten Kautschuk-Alkydharz-Kombinationen und chlorierten Kautschuk-Acrylatharz-Kombinationen sind gegen Wasser beständig, was für die vorliegende Erfindung bei speziellen Anwendungen von Vorteil sein kann.

### Beschichtungsstoffe auf der Basis von Vinylharzen

Diese Beschichtungsstoffe enthalten Polyolefine und Polyolefinderivate wie Polyethylen und Polyisobutene. Ethylencopolymere wie Ethylen-Vinylacetat-Copolymere oder Ethylen-Maleinsäureanhydrid-Copolymere sind wasserlöslich und können Salze bilden. Chlorsulfonierte Polyethylene haben eine sehr hohe chemische Widerstandsfähigkeit, insbesondere gegen Oxidationsmittel.

### Beschichtungsstoffe auf der Basis von Polyvinylhalogeniden und Vinylhalogenid-Copolymeren

Beschichtungsstoffe, die Polyvinylchlorid und chloriertes Polyvinylchlorid enthalten, haben vorteilhafte mechanische Eigenschaften und eine hohe Abriebfestigkeit. Beschichtungsstoffe mit Vinylidenchlorid-Copolymeren haben zahlreiche technische Anwendungsmöglichkeiten. Wichtige Beispiele von Copolymeren ohne zusätzliche funktionelle Gruppen sind Vinylacetat-, Dibutylmaleinat- oder Isobutylvinylether-Copolymere. Terpolymere mit Carboxylgruppen werden mit Dibutylmaleinat oder Vinylacetat und einer Dicarbonsäure hergestellt. Copolymere und Terpolymere mit Hydroxylgruppen werden mit Hydroxyacrylaten oder mit Vinylacetat und Vinylalkohol erhalten. Vinylidenchloridcopolymere mit Acrylnitril oder Acrylharzen haben eine hervorragende Chemikalienbeständigkeit und werden vor allem als Folien für Nahrungsmittelverpackungen verwendet. Polyvinylidendifluorid ist ein thermoplastisches Fluorpolymer und kann deshalb für Pulverbeschichtungsstoffe verwendet werden. Weitere Beispiele von Fluorpolymeren für Beschichtungsstoffe sind cyclische perfluorierte Polymere. Vernetzbare perfluorierte Polymere enthalten Epoxygruppen, Ethergruppen, Hydroxylgruppen oder Carboxylgruppen als vernetzende funktionelle Gruppen.

### Beschichtungsstoffe auf der Basis von Vinylesterpolymeren und -copolymeren

Beschichtungsstoffe auf der Basis von Vinylacetatpolymeren und Vinylacetatcopolymeren mit Vinyllaurat, Dibutylmaleinat oder Crotonsäure haben eine besonders hohe Lichtfestigkeit und eine starke Haftung auf Metallen. Polyvinylalkohol wird üblicherweise durch die Hydrolyse von Polyvinylacetat hergestellt. Er hat eine hohe Wasserlöslichkeit und ist beständig gegenüber Ölen und Fetten, Schmiermitteln und Wachsen. Polyvinylacetale wie Polyvinylformal und Polyvinylbutyral werden durch die Reaktion von Polyvinylacetat mit Aldehyden hergestellt. Sie können als Terpolymere von Vinylacetat, Vinylalkohol und Vinylacetal angesehen werden. Sie sind physikalisch vernetzbar, können aber auch durch Hydroxylgruppen chemisch vernetzt werden.

### Beschichtungsstoffe auf der Basis von Vinyletherpolymeren

Vinyletherpolymere werden aus Methylvinylether oder Isobutylvinylether hergestellt und werden oft als weichmachende Harze in Beschichtungsstoffen verwendet.

### Polystyrol und Styrolcopolymere

Wegen ihrer hohen Glastemperatur bilden Polystyroldispersionen keine Filme bei Raumtemperatur. Die Härte von Polystyrol kann jedoch über einen weiten Temperaturbereich durch die Copolymerisation mit weichen Monomeren wie Butadien und Acrylatester dem jeweiligen Verwendungszweck des Beschichtungsstoffs angepasst werden.

### Beschichtungsstoffe auf der Basis von Acrylaten

Polyacrylate werden nur wenig von Chemikalien angegriffen und verleihen daher den Beschichtungsstoffen eine hohe Widerstandsfähigkeit. Sie sind farblos, transparent und vergilben auch nicht nach länger andauernder thermischen Belastung. Sie absorbieren keine Strahlung über 300 nm und werden deshalb auch nicht durch UV-Strahlung abgebaut, solange sie kein Styrol oder ähnliche aromatische Verbindungen enthalten. Sie haben keine instabilen Doppelbindungen, haben aber einen hohen, beständigen Glanz und sind hydrolysestabil. Je nach ihren lateralen funktionellen Gruppen können sie in vielfältiger Weise vernetzt werden (vgl. Tabelle 3).

### Alkyd-Beschichtungsstoffe

Alkydharzbindemittel enthalten Öle/Fettsäuren, Dicarbonsäuren (hauptsächlich Phthalsäure oder Phthalsäureanhydrid) und Polyalkohole. Sie werden als kurzölige, mittelölige oder langölige Harze klassifiziert. Sie werden mit natürlichen Fettsäuren oder Ölen modifiziert. Für spezielle technische Anwendungen werden die Alkydharze zusätzlich mit Harzsäuren, Benzoesäure, Styrol, Vinyltoluol, Isocyanaten, Acrylaten, Epoxiden und Silikonverbindungen modifiziert.

### Beschichtungsstoffe auf der Basis von gesättigten Polyestern

Gesättigte Polyester werden durch die Polykondensation von Polycarbonsäuren wie Terephtalsäure und Polyalkoholen wie Ethylenglykol hergestellt. Hydroxylgruppen enthaltende Polyester können durch Melaminharze, Benzoguanaminharze und blockierte Isocyanate vernetzt werden. Carboxylgruppen enthaltende gesättigte Polyester können mit Triglycidylisocyanurat oder Epoxidharzen vernetzt werden. Laterale Acrylatgruppen enthaltende gesättigte Polyester können durch UV-Strahlung und Elektronenstrahlung gehärtet werden.

### Beschichtungsstoffe auf der Basis ungesättigter Polyester

Ungesättigte Polyesterharze oder UP-Harze sind lösliche lineare Polykondensationsprodukte, die durch die Reaktion von üblicherweise ungesättigten mehrwertigen Carbonsäuren wie Malieinsäure oder Fumarsäure und zweiwertigen Alkoholen wie Ethylenglykol hergestellt werden. Die Härtung wird durch Peroxid- oder C-C-Radikalstarter initiiert. Sie können aber auch in der Gegenwart von Photoinitiatoren durch UV-Strahlung gehärtet werden.

### Beschichtungsstoffe auf der Basis von Polyurethanen

Polyurethane sind Bindemittel mit Urethan-, Harnstoff-, Biuret- oder Allophanatgruppen als verbindende Gruppen. Die Beschichtungsstoffe enthalten niedermolekulare, oligomere oder polymere Komponenten, die durch blockierte Polyisocyanate in Einkomponentensystemen und mit freien Polyisocyanaten in Zweikomponentensystemen vernetzt werden können. Die ausgehärteten Beschichtungen haben eine hohe chemische Beständigkeit und eine exzellente Lichtstabilität und Wetterstabilität.

### Beschichtungsstoffe auf der Basis von Epoxidharzen

Es gibt verschiedene Arten von Epoxidharzen wie Bisphenol A Harze, Bisphenol F Harze, Epoxynovolacke, aliphatische Epoxidharzen, cycloaliphatische Epoxidharze, Glycidylester und heterocyclische Epoxidverbindungen. Sie können durch Amine, Polyester, Phenolharze, Aminoharze oder Polyisocyanate vernetzt werden. Außerdem können sie chemisch modifiziert werden, sodass Epoxidharzester und Epoxidharz(meth)acrylsäureester resultieren.

### Beschichtungsstoffe auf der Basis von Harnstoff-, Benzoguanamin- und Melaminharzen

Alkylierte Harnstoff-, Benzoguanamin-, Glycoluril-, Toluolsulfonamid- und Melaminformaldehydharze sind eine vielseitige Gruppe von Vernetzungsmittel für Hydroxyl-, Amid-, Carboxylgruppen enthaltende Polymere. Sie werden sowohl in Beschichtungsstoffen auf Wasserbasis und auf Lösemittelbasis verwendet.

### Beschichtungsstoffe auf der Basis von Phenolharzen

Phenolharze sind Polykondensationsprodukte von Phenolen und Formaldehyd und sind in der Gegenwart von Basen oder basischen Salzen selbstvernetzend. Phenolharze sind schwach gelb bis dunkelbraun gefärbt und kommen daher für eine Farbanwendung nicht in Betracht. Da sie harte und brüchige Filme bilden, müssen sie mit anderen Harzen wie Epoxidharzen, Polyvinylbutyral-Harzen oder Polyesterharzen vermischt werden.

### Beschichtungsstoffe auf der Basis von Silikonharzen und organisch-anorganischen Hybridharzen

Polysiloxan oder Silikonharze für Beschichtungszwecke werden aus Mischungen von di- oder trifunktionellen Organosilanmonomeren wie Phenyl- und Methyltriethoxysilan, Methylphenyldichlorsilan und Dimethyldichlorsilan durch kontrollierte Hydrolyse und Kondensation hergestellt. Reine Silikonharze sind aber oftmals zu weich und zu thermoplastisch für Beschichtungszwecke. Deshalb werden sie mit üblichen und bekannten Harzen wie Alkydharzen, Acrylharzen, Epoxidharzen und Phenolharzen vermischt.

Eine besonders gut geeignete Gruppe von Silikonharzen sind Hybride von Siloxan-Polyurethan- und Siloxan-Epoxy- oder Siloxan-Acrylat- und Sol-Gel-Polymeren.

Die Sol-Gel-Hybridpolymere werden durch die Polykondensation von Siloxanen, die hydrolysierbare Gruppen und die nachstehend beschriebenen organischen Reste enthalten, hergestellt.

Der wesentliche Bestandteil der Hybrid-Dispersionen sind oberflächenmodifizierte, kationisch stabilisierte, anorganische Nanopartikel mindestens einer Art, insbesondere einer Art.

Vorzugsweise werden die zu modifizierenden Nanopartikel aus der Gruppe, bestehend aus Haupt- und Nebengruppen-Metallen und deren Verbindungen ausgewählt. Bevorzugt werden die Haupt- und Nebengruppen-Metalle aus Metallen der dritten bis fünften Hauptgruppe, der dritten bis sechsten sowie der ersten und zweiten Nebengruppe des Periodensystems der Elemente sowie den Lanthaniden ausgewählt. Besonders bevorzugt werden Bor, Aluminium, Gallium, Silizium, Germanium, Zinn, Arsen, Antimon, Silber, Zink, Titan, Zirkonium, Hafnium, Vanadium, Niob, Tantal, Molybdän, Wolfram und Cer, insbesondere Aluminium, Silizium, Silber, Cer, Titan und Zirkonium, eingesetzt.

Vorzugsweise handelt es sich bei den Verbindungen der Metalle um die Oxide, Oxidhydrate, Sulfate oder Phosphate.

Bevorzugt werden Silber, Siliziumdioxid, Aluminiumoxid, Aluminiumoxidhydrat, Titandioxid, Zirkoniumoxid, Ceroxid und Mischungen hiervon, besonders bevorzugt Silber, Ceroxid, Siliziumdioxid, Aluminiumoxidhydrat und Mischungen hiervon, ganz besonders bevorzugt Aluminiumoxidhydrat und insbesondere Böhmit verwendet.

Vorzugsweise weisen die zu modifizierenden Nanopartikel eine Primärpartikelgröße <50 nm, bevorzugt 5 nm bis 50 nm, insbesondere 10 nm bis 30 nm, auf.

Die Nanopartikel bzw. deren Oberfläche sind mit mindestens einer Verbindung der allgemeinen Formel XIV:

**[(S-)ₒ-L-]ₘM(R)ₙ(H)ₚ** **(XIV)**

**,** modifiziert.

In der allgemeinen Formel XIV haben die Indizes und der Variablen die folgende Bedeutung:
- S: reaktive funktionelle Gruppe;
- L: mindestens zweibindige organische verknüpfende Gruppe;
- H: hydrolysierbare einbindige Gruppe oder hydrolysierbares Atom;
- M: zwei- bis sechswertiges Hauptgruppen- und Nebengruppen-Metall;
- R: einbindiger organischer Rest;
- o: eine ganze Zahl von 1 bis 5, insbesondere 1;
- m + n+ p: eine ganze Zahl von 2 bis 6, insbesondere 3 oder 4;
- p: eine ganze Zahl von 1 bis 6, insbesondere 1 bis 4;
- m und n: Null oder eine ganze Zahl von 1 bis 5, vorzugsweise 1 bis 3, insbesondere 1, speziell m = 1 und n = 0.

Dabei kann die Modifizierung durch physikalische Adsorption der Verbindungen XIV an die Oberfläche der unmodifizierten Nanopartikel und/oder durch chemische Reaktion der Verbindungen XIV mit geeigneten reaktiven funktionellen Gruppen an der Oberfläche der unmodifizierten Nanopartikel erfolgen. Vorzugsweise erfolgt die Modifizierung über chemische Reaktionen.

Beispiele geeigneter Metalle M sind die vorstehend beschriebenen.

Vorzugsweise wird die reaktive funktionelle Gruppe S aus der Gruppe, bestehend aus (S 1) reaktiven funktionellen Gruppen, die mindestens eine mit aktinischer Strahlung aktivierbare Bindung enthalten, und (S 2) reaktiven funktionellen Gruppen, die mit Gruppen ihrer Art ("mit sich selbst") und/oder mit komplementären reaktiven funktionellen Gruppen thermisch initiierte Reaktionen eingehen, ausgewählt. Beispiele geeigneter reaktiver funktioneller Gruppen (S 2) sind die vorstehend beschriebenen Gruppen, insbesondere Epoxidgruppen.

Im Rahmen der vorliegenden Erfindung wird unter einer mit aktinischer Strahlung aktivierbaren Bindung eine Bindung verstanden, die bei Bestrahlen mit aktinischer Strahlung reaktiv wird und mit anderen aktivierten Bindungen ihrer Art Polymerisationsreaktionen und/oder Vernetzungsreaktionen eingeht, die nach radikalischen und/oder ionischen Mechanismen ablaufen. Beispiele geeigneter Bindungen sind Kohlenstoff-Wasserstoff-Einzelbindungen oder Kohlenstoff-Kohlenstoff-, Kohlenstoff-Sauerstoff-, Kohlenstoff-Stickstoff-, Kohlenstoff-Phosphor - oder Kohlenstoff-Silizium-Einzelbindungen oder -Doppelbindungen. Von diesen sind die Kohlenstoff-Kohlenstoff-Doppelbindungen besonders vorteilhaft und werden deshalb erfindungsgemäß ganz besonders bevorzugt verwendet. Der Kürze halber werden sie im Folgenden als "Doppelbindungen" bezeichnet.

Demnach enthält die erfindungsgemäß bevorzugte reaktive Gruppe (S 1) eine Doppelbindung oder zwei, drei oder vier Doppelbindungen. Werden mehr als eine Doppelbindung verwendet, können die Doppelbindungen konjugiert sein. Erfindungsgemäß ist es indes von Vorteil, wenn die Doppelbindungen isoliert, insbesondere jede für sich endständig, in der hier in Rede stehenden Gruppe (S 1) vorliegen. Erfindungsgemäß ist es von besonderem Vorteil zwei Doppelbindungen, insbesondere eine Doppelbindung, zu verwenden.

Die mit aktinischer Strahlung aktivierbaren Bindungen können über Kohlenstoff-Kohlenstoffbindungen oder Ether-, Thioether-, Carbonsäureester-, Thiocarbonsäureester-, Carbonat-, Thiocarbonat-, Phosphorsäureester-, Thiophosphorsäureester-, Phosphonsäureester-, Thiophosphonsäureester-, Phosphit-, Thiophosphit-, Sulfonsäureester-, Amid-, Amin-, Thioamid-, Phosphorsäureamid-, Thiophosphorsäureamid-, Phosphonsäureamid-, Thiophosphonsäureamid-, Sulfonsäureamid-, Imid-, Urethan-, Hydrazid-, Harnstoff-, Thioharnstoff-, Carbonyl-, Thiocarbonyl-, Sulfon- oder Sulfoxidgruppen, insbesondere aber über Kohlenstoff-Kohlenstoff-Bindungen, Carbonsäureestergruppen und Ethergruppen, mit der verknüpfenden Gruppe L verbunden sein.

Besonders bevorzugte reaktive funktionelle Gruppen (S 1) sind daher (Meth)Acrylat-, Ethacrylat-, Crotonat-, Cinnamat-, Vinylether-, Vinylester-, Dicyclopentadienyl-, Norbornenyl-, Isoprenyl-, Isopropenyl-, Allyl- oder Butenylgruppen; Dicyclopentadienyl-, Norbornenyl-, Isoprenyl-, Isopropenyl-, Allyl- oder Butenylethergruppen oder Dicyclopentadienyl-, Norbornenyl-, Isoprenyl-, Isopropenyl-, Allyl- oder Butenylestergruppen, insbesondere aber Methcrylatgruppen (S 1).

Die Variable H steht für eine hydrolysierbare einbindige Gruppe oder für ein hydrolysierbares Atom.

Beispiele geeigneter hydrolysierbarer Atome sind Wasserstoffatome und Halogenatome, insbesondere Chlor- und Bromatome.

Vorzugsweise werden die hydrolysierbaren einbindigen Gruppen verwendet. Beispiele geeigneter Gruppen dieser Art sind Gruppen der allgemeinen Formel XV:

**-X-R** **(XV)**

**.**

In der allgemeinen Formel XV steht die Variable X für ein Sauerstoffatom, Schwefelatom und/oder eine Gruppe >NR², worin R² eine Alkylgruppe mit 1 bis 4 Kohlenstoffatomen, insbesondere Methyl, Ethyl, Propyl und n-Butyl, bedeutet. Bevorzugt steht X für ein Sauerstoffatom.

R steht für einen einbindigen organischen Rest. Der einbindige Rest R kann substituiert oder unsubstituiert sein; vorzugsweise ist er unsubstituiert. Er kann aromatisch, aliphatisch oder cycloaliphatisch sein. Ein einbindiger Rest R wird dann als aromatisch angesehen, wenn X direkt mit dem aromatischen Rest verbunden ist. Diese Regel ist sinngemäß auf die aliphatischen und cycloaliphatischen Reste anzuwenden. Vorzugsweise werden lineare oder verzweigte, insbesondere lineare, aliphatische Reste eingesetzt. Bevorzugt sind niedere aliphatische Reste. Von diesen werden die Methylgruppen oder die Ethylgruppen ganz besonders bevorzugt verwendet.

Die Variable L steht für eine mindestens zweibindige, insbesondere zweibindige, organische verknüpfende Gruppe.

Beispiele geeigneter zweibindiger organischer vernüpfender Gruppen L sind gegebenenfalls Heteroatome enthaltende, aliphatische, aromatische, cycloaliphatische und aromatischcycloaliphatische Kohlenwasserstoffreste, wie
(1) substituierte oder unsubstituierte, bevorzugt unsubstituierte, lineare oder verzweigte, vorzugsweise lineare, Alkandiyl-Reste mit 3 bis 30, bevorzugt 3 bis 20 und insbesondere 3 Kohlenstoffatomen, die innerhalb der Kohlenstoffkette auch cyclische Gruppen enthalten können, insbesondere Trimethylen, Tetramethylen, Pentamethylen, Hexamethylen, Heptamethylen, Octamethylen, Nonan-1,9-diyl, Decan-1,10-diyl, Undecan-1,11-diyl Dodecan-1,12-diyl, Tridecan-1,13-diyl, Tetradecan-1,14-diyl, Pentadecan-1,15-diyl, Hexadecan-1,16-diyl, Heptadecan-1,17-diyl, Octadecan-1,18-diyl, Nonadecan-1,19-diyl oder Eicosan-1,20-diyl, bevorzugt Tetramethylen, Pentamethylen, Hexamethylen, Heptamethylen, Octamethylen, Nonan-1,9-diyl, Decan-1,10-diyl, 2-Heptyl-1-pentyl-cyclohexan-3,4-bis(non-9-yl), Cyclohexan-1,2-, -1,4- oder -1,3-bis(methyl), Cyclohexan-1,2-, 1,4- oder 1,3-bis(eth-2-yl), Cyclohexan-1,3-bis(prop-3-yl) oder Cyclohexan-1,2-, 1,4- oder 1,3-bis(but-4-yl);
(2) substituierte oder unsubstituierte, bevorzugt unsubstituierte, lineare oder verzweigte, vorzugsweise lineare, Oxalkandiyl-Reste mit 3 bis 30, bevorzugt 3 bis 20 und insbesondere 3 bis 6 Kohlenstoffatomen, die innerhalb der Kohlenstoffkette auch cyclische Gruppen enthalten können, insbesondere Oxapropan-1,4-diyl, Oxabutan-1,5-diyl, Oxapentan-1,5-diyl, Oxahexan-1,7-diyl oder 2-Oxapentan-1,5-diyl;
(3) zweiwertige Polyesterreste mit wiederkehrenden Polyesteranteilen der allgemeinen Formel XV:

   **-(-CO-(CHR³)ᵣ-CH₂-O-)-** **(XVI)**

   **.** Hierbei ist der Index r bevorzugt 4 bis 6 und der Substitutent R³ = Wasserstoff, ein Alkyl-, Cycloalkyl- oder Alkoxy-Rest. Kein Substituent enthält mehr als 12 Kohlenstoffatome;
(4) lineare Polyetherreste, vorzugsweise mit einem zahlenmittleren Molekulargewicht von 400 bis 5.000, insbesondere von 400 bis 3.000, die sich von Poly(oxyethylen)glykolen, Poly(oxypropylen)glykolen und Poly(oxybutylen)glykolen ableiten;
(5) lineare Siloxanreste, wie sie beispielsweise in Siliconkautschuken vorliegen, hydrierte Polybutadien- oder Polyisoprenreste, statistische oder alternierende Butadien-Isopren-Copolymerisatreste oder Butadien-Isopren-Pfropfmischpolymerisatreste, die noch Styrol einpolymerisiert enthalten können, sowie Ethylen-Propylen-Dienreste;
(6) Phen-1,4-, -1,3- oder -1,2-ylen, Naphth-1,4-, -1,3-, -1,2-, -1,5- oder -2,5-ylen, Propan-2,2-di(phen-4'-yl), Methan-di(phen-4'-yl), Diphenyl-4,4'-diyl oder 2,4- oder 2,6-Toluylen; oder
(7) Cycloalkandiyl-Reste mit 4 bis 20 Kohlenstoffatomen, wie Cyclobutan-1,3-diyl, Cyclopentan-1,3-diyl, Cyclohexan-1,3- oder -1,4-diyl, Cycloheptan-1,4-diyl, Norbornan-1,4-diyl, Adamantan-1,5-diyl, Decalin-diyl, 3,3,5-Trimethyl-cyclohexan-1,5-diyl, 1-Methylcyclohexan-2,6-diyl, Dicyclohexylmethan-4,4'-diyl, 1,1'-Dicyclohexan-4,4'-diyl oder 1,4-Dicyclohexylhexan-4,4"-diyl, insbesondere 3,3,5-Trimethyl-cyclohexan-1,5-diyl oder Dicyclohexylmethan-4,4'-diyl.

Besonders bevorzugt werden die verknüpfenden Gruppen L (1) und L (2), ganz besonders bevorzugt Trimethylen, Tetramethylen, Pentamethylen, Hexamethylen, Heptamethylen, Octamethylen, Oxapropan-1,4-diyl oder 2-Oxapentan-1,5-diyl und insbesondere Trimethylen, Oxapropan-1,4-diyl oder 2-Oxapentan-1,5-diyl verwendet.

In der allgemeinen Formel XIV steht die Variable o für eine ganze Zahl von 1 bis 5, vorzugsweise 1 bis 4, bevorzugt 1 bis 3 und besonders bevorzugt 1 und 2. Insbesondere ist o gleich 1.

Die Verbindungen XIV können auch in komplexierter Form eingesetzt werden, wie dies beispielsweise in der internationalen Patentanmeldung WO 09/52964, Seite 8, Zeilen 12 bis 20, beschrieben wird.

Die Verbindungen XIV sind üblich und bekannt und zu einem großen Teil im Handel erhältlich. Gut geeignete Verbindungen XIV sind beispielsweise aus der
- internationalen Patentanmeldung WO 99/52964, Seite 6, Zeile 1, bis Seite 8, Zeile 20,
- der deutschen Patentanmeldung DE 197 26 829 A 1, Spalte 2, Zeile 27, bis Spalte 3, Zeilen 38,
- der deutschen Patentanmeldung DE 199 10 876 A 1, Seite 2, Zeile 35, bis Seite 3, Zeile 12,
- der deutschen Patentanmeldung DE 38 28 098 A 1, Seite 2, Zeile 27, bis Seite 4, Zeile 43, oder
- der europäischen Patentanmeldung EP 0 450 625 A 1, Seite 2, Zeile 57, bis 5, Zeile 32,
bekannt.

Bevorzugte hydrolysierbare Siloxane oder Silane, die zu Polymerisation oder zur Vernetzung oder als Ausgangsmaterialien hierfür (optional in der Form von Vorkondensaten und Polykondensaten) sind
- 3-Glycidyloxypropytrimethoxysilan,
- 3-Glycidyloxypropyltriethoxysilan,
- 3-Glycidyloxypropylmethyldimethoxysilan,
- 3-Glyxidyloxypropyldiethoxydimethoxysilan und
- 3-Methacryloxypropyltrimethoxysilan.

Weitere Alkoxysilane, die als solche oder vorzugsweise in Kombination mit den vorstehend aufgeführten Alkoxysilanen die zur Polyaddition oder Polykondensation befähigte Gruppen aufweisen sind Tetramethoxysilan, Tetraethoxysilan, Tetra-n-propoxysilan, tetra-n-Butoxysilan, Cyclohexyltrimethoxysilan, Cyclopentyltrimethoxysilan, Ethyltrimethoxysilan, Phenylethyltrimethoxysilan, Phenyltrimethoxysilan, n-Propyltrimethoxysilan, Cyclohexylmethyldimethoxysilan, Dimethyldimethoxysilan, Diisopropyldimethoxysilan, Phenylmethyldimethoxysilan, Phenylethyltriethoxysilan, Phenyltriethoxysilan, Phenylmethyldiethoxysilan und Phenyldimethylethoxysilan.

Des Weiteren können Alkoxide of Aluminium, Titan, Zirkon, Tantal, Niob, Zinn, Zink, Wolfram, Germanium und Bor mit 1 bis 4 Kohlenstoffatomen in ihren Alkyresten mitverwendet werden. Geeignete Vertreter solcher Alkoxide sind Aluminium-sec.-butylat, Titanisopropoxid, Titanpropoxid, Titanbutoxid, Zirkonisopropoxid, Zirkonpropoxid, Zirkonbutoxid, Zirkonethoxid, Tantalethoxide, Tantalbutoxid, Niobethoxid, Niobbutoxid, Zinn-tert-butoxid, Wolfram(VI)ethoxid, Germaniumethoxid, Germaniumisopropoxid und di-tert-Butoxyaluminiumtriethoxysilan.

Im Falle der besonders reaktiven Alkoxide von Titan, Aluminium oder Zirkon ist es von Vorteil, die Verbindungen in komplexierter Form zu verwenden. Beispiele für geeignete Komplexierungsmittel sind ungesättigte Carbonsäuren und beta-Dicarbonylgruppenverbindungen wie Methacrylsäure, Acetylaceton und Essigsäureethylester.

Methodisch gesehen bietet die Modifizierung der Oberfläche der Nanopartikel keine Besonderheiten, sondern erfolgt nach den üblichen und bekannten Verfahren, die beispielsweise aus der internationalen Patentanmeldung WO 99/52964, Seite 10, Zeile 22, bis Seite 11, Zeile 17, und Beispiele 1 bis 20, Seite 14, Zeile 10, bis Seite 20 Zeile 24, oder aus der deutschen Patentanmeldung DE 197 26 829 A 1, Beispiele 1 bis 6, Spalte 5, Zeile 63, bis Spalte 8, Zeile 38, bekannt sind. Vorzugsweise werden die dort angegebenen Mengenverhältnisse von Verbindungen XIV zu unmodifizierten Nanopartikeln angewandt.

Der Gehalt der Dispersionen an den oberflächenmodifizierten, anorganischen Nanopartikeln kann breit variieren und richtet sich nach den Erfordernissen des Einzelfalls. Vorzugsweise sind die Nanopartikel in den Dispersionen in einer Menge von, bezogen auf die Gesamtmenge der Bestandteile), 60 bis 98 Gew.-% enthalten.

Außer den vorstehend beschriebenen Harzen oder Bindemitteln können die Beschichtungsstoffe übliche und bekannte Additive enthalten. Vorzugsweise handelt es sich dabei um Reaktivverdünner, die thermisch oder durch aktinische Strahlung, insbesondere durch UV-Strahlung, gehärtet werden können, niedrigsiedende organische Lösemittel und hochsiedende organische Lösemittel, Wasser, UV-Absorber, Radikalfänger, thermolabile radikalische Initiatoren, Photoinitiatoren, Katalysatoren für die thermische Vernetzung, Entlüftungsmittel, Slipadditive, Polymerisationsinhibitoren, Entschäumer, Netzmittel, Dispergiermittel, Haftverbesserer, Glättungsmittel, filmbildende Hilfsmittel, Mittel gegen das Absenken, Rheologiehilfsmittel (Verdicker), Flammschutzmittel, Trocknungsmittel, Hautverhinderungsmittel, Korrosionsinhibitoren, Wachse und Mattierungsmittel.

Bei einer weiteren vorteilhaften Ausführungsform werden die bakteriziden und/oder viruziden Beschichtungen durch das Aufsprühen von bioziden Metallen wie Kupfer mithilfe des elektrischen Doppeldrahtsprühens (twin-wire arc spraying) auf die vorstehend beschriebenen partikulären, anorganischen, inerten Trägermaterialien appliziert. Dies hat den wesentlichen Vorteil, dass es sich bei dieser Technologie um ein ausgereiftes Verfahren handelt und dass dadurch erhebliche Mengen an teuerem biozidem Kupfer oder Silber eingespart werden kann, wodurch auch das Gewicht der erfindungsgemäßen Vorrichtung verringert wird.

Vorzugsweise enthalten die Kontaktstellen in dem Wärmeleitsystem und dem Kälteleitsystem der erfindungsgemäßen Vorrichtung zur Intensivierung der thermischen Leitung Schichten aus einer Wärmeleitpaste, insbesondere einer Graphit-, Nanokohlenstoff-, Silber-, Diamant- oder Aluminiumnitrid-Wärmeleitpaste und/oder AIN-Keramikscheiben.

In den horizontalen Boden der erfindungsgemäßen Vorrichtung ist mindestens ein wieder aufladbarer Akkumulator als Stromquelle für das mindestens eine Peltier-Element eingebaut. Der mindestens eine Akkumulator ist dabei in eine entsprechend geformte Öffnung wieder entnehmbar eingeschoben. Er steht in Verbindung mit einem elektronischen Steuergerät im horizontalen Boden zur Regelung der Leistungsabgabe der mindestens zwei Peltier-Element.

Vorzugsweise ist das elektronische Steuergerät über Signalleitungen mit einem in der oberen Öffnung der Kartusche angeordneten Miniaturanemometer und einem Temperatursensor sowie mit einem Temperatursensor im Heizraum verbunden. Das elektronische Steuergerät regelt den Luftdurchfluss durch die erfindungsgemäße Vorrichtung auf der Basis der von den Sensoren erhaltenen Signale.

Die Vorderseite des elektronischen Steuergeräts umfasst vorzugsweise einen Drehknopf zum Einschalten und zur manuellen Regelung, eine Aufnahme für den Stecker eines Ladekabels, Leuchtdioden zur Funktionsanzeige und eine visuelle Anzeige, die die Temperaturdifferenz zwischen den Temperatursensor in der Öffnung der Kartusche und dem Temperatursensor im Heizraum zeigt.

Unterhalb des luftdurchlässigen Kartuschenbodens kann ein automatisch oder manuell abschaltbarer Axialventilator mit einem elektronisch gesteuerten Elektromotor mit mindestens zwei Flügeln als Hilfsaggregat an einer Aufhängung in dem zweiten Innenrohr horizontal befestigt sein. Als Axialventilator und kommen insbesondere Papst-Ventilatoren, wie sie zur Belüftung von elektronischen Geräten verwendet werden, in Betracht. Der horizontale Axialventilator kann in das elektronische Steuerungssystem integriert sein und kann bei Bedarf in Gang gesetzt werden, um den Luftstrom durch die Kartusche zu starten. Wenn der Kamineffekt dann den Luftstrom verstärkt und am Laufen hält, kann der Axialventilator automatisch oder manuell abgeschaltet werden.

Die Kartusche kann außerdem durch ein umlaufendes, wärmebeständiges, stützendes Bauteil mit geringer Wärmeleitfähigkeit λ in der Form eines Trichteroberteils zur Luftlenkung stabilisiert werden. Außerdem kann die Kartusche durch thermisch isolierende Pfropfen, vorzugsweise aus wärmebeständigen Elastomeren, zur Schwingungsdämpfung oder mit seitlicher horizontaler Verlängerung zur Schwingungsdämpfung und als Strömungssperre stabilisiert werden.

Die erfindungsgemäße Vorrichtung kann außerdem noch auf einer Standhilfe wie eine horizontale Platte mit einem größeren Querschnitt oder auf schrägem oder geraden Standbeinen stehen. Ihrer Außenseite und die Standhilfen können dekorativ gestaltet, beispielsweise bemalt, sein.

Das erfindungsgemäße Verfahren zur Reinigung und Desinfizierung von Raumluft wird mithilfe der durch eine Temperaturdifferenz betriebenen erfindungsgemäßen Vorrichtung durchgeführt und umfasst zumindest die folgenden Verfahrensschritte:
(A) Ansaugen von Luft durch mindestens einen Lufteinlass in den Heizraum oberhalb der heißen Seite des mindestens einen Peltier-Elements,
(B) Aufheizen der einströmenden Luft,
(C) Erzeugen eines Kamineffekts in der Kartusche durch die Kühlung der umlaufenden, wärmeleitfähigen, kühlbaren Kartuschenhalterung der Kartusche mithilfe der kalten Seite des mindestens einen Peltier-Elements und des Kälteleitsystems oder Kühlsystems, wodurch ein Luftstrom durch (i) die mindestens eine Schüttung aus mindestens einer Art eines partikulären, anorganischen, bakterizid und/oder viruzid beschichteten, inerten Trägermaterials und/oder durch eine Schüttung aus einem partikulären, anorganischen, bakteriziden und/oder viruziden Material und durch (ii) eine Schüttung aus mindestens einer Art von eines partikulären Absorbens und/Adsorbens und
(D) Eliminierung der in dem Luftstrom vorhandenen freien oder an Aerosole gebundenen Bakterien, Viren, Virionen und anderen Noxen durch Kontakt-Eliminierung an dem partikulären, anorganischen, bakterizid und/oder viruzid beschichteten, inerten Trägermaterial und/oder an dem partikulären, anorganischen, bakteriziden und/oder viruziden Material und Adsorption und/oder Absorption der durch die Eliminierung erzeugten Zerfallsprodukte und Noxen an und/oder in der mindestens einen Art eines partikulären Adsorbens und/oder Adsorbens.

Die biozide, insbesondere bakterizide und viruzide, Wirkung der erfindungsgemäßen Vorrichtung kann mithilfe einer Testvorrichtung quantitativ und qualitativ gemessen werden. Die erfindungsgemäße Testvorrichtung umfasst
- eine erfindungsgemäße Vorrichtung in einer mit einer Dichtung abgedichteten, geschlossenen Kammer,
- eine Abzugshaube mit einer Luftableitung für die Abluft,
- eine Aerosolzuleitung mit einem Gebläse, einem Absperrventil und einer Vernebelungsdüse im Inneren der Kammer zur Erzeugung einer Aerosolwolke,
- einen Anschluss für gereinigte trockene Luft mit einem Absperrventil und einer Lufteinlassdüse im Inneren der Kammer,
- einen Sensor für die Partikelzählung durch Laserbeugung in der Luftableitung, der mit einer Signalleitung mit einem Partikelmessgerät verbunden ist,
- einen weiteren Sensor für die Partikelzählung durch Laserbeugung in der Kammer, der mit einer Signalleitung mit einem weiteren Partikelmessgerät verbunden ist,
- einen Sensor für die Strömungsmessung in der Luftableitung, der mit einer Signalleitung mit einem Strömungsmessgerät verbunden ist, wobei
- die Partikelmessgeräte und das Strömungsmessgerät über die Signalleitungen mit der zentralen Datenverarbeitungsanlage zur Verarbeitung der Signale der Messgeräte verbunden sind und wobei
- die in der Datenverarbeitungsanlage verarbeiteten Signale auf dem Anzeigegerät ausgegeben werden.

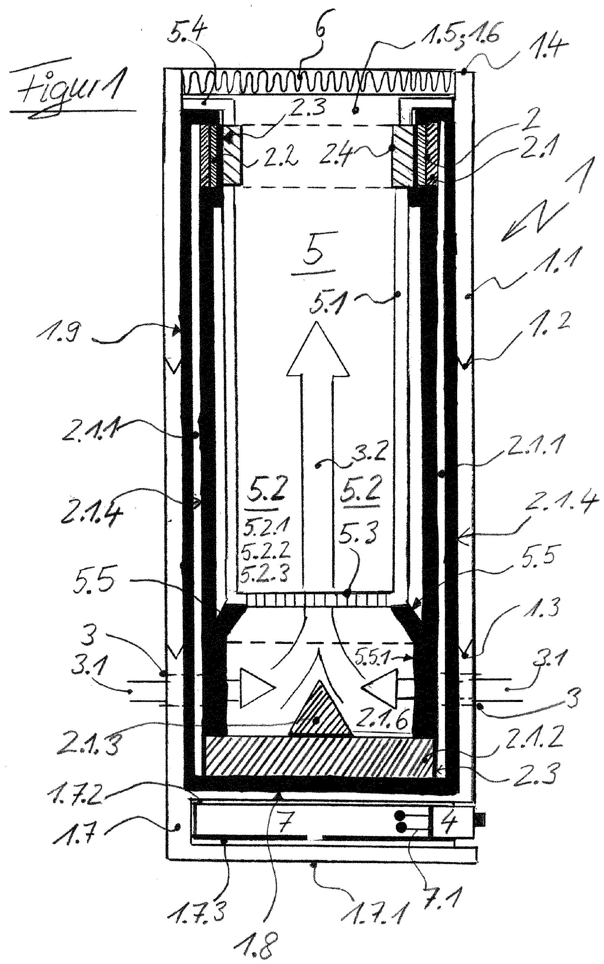

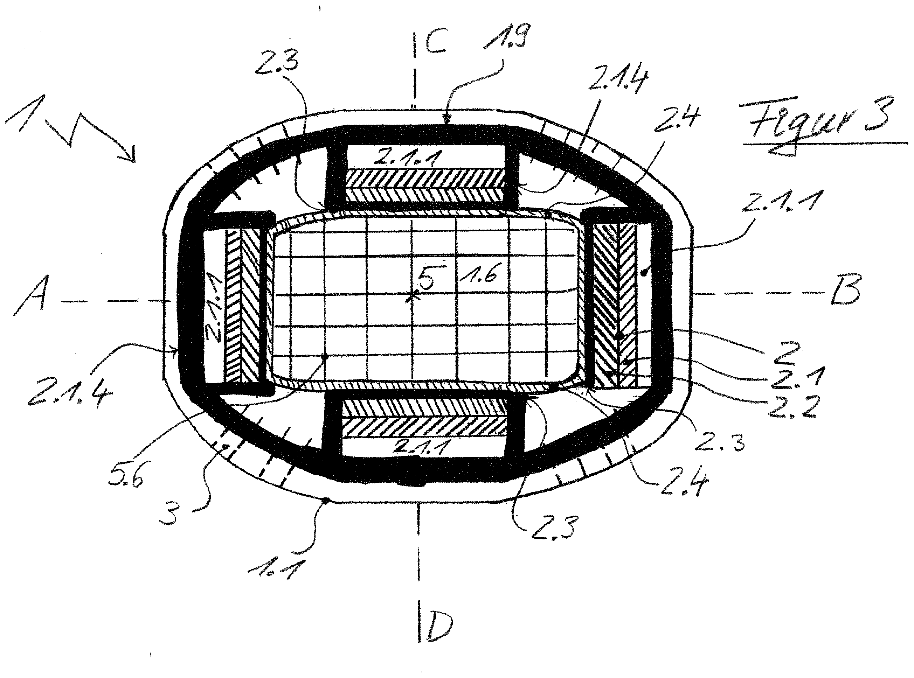

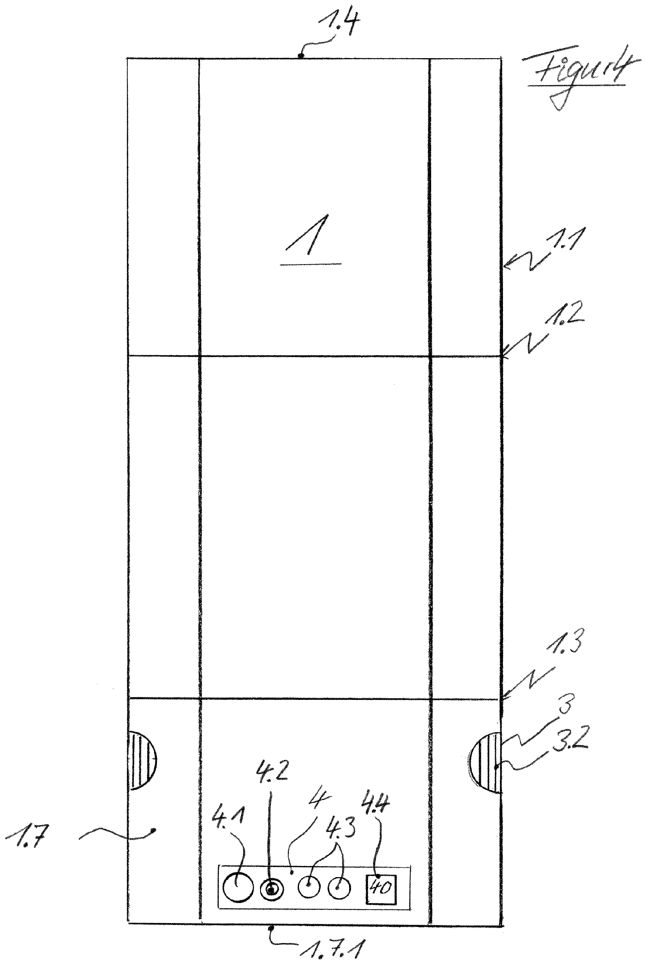

Die Figuren 1 bis 4 dienen der Veranschaulichung des Aufbaus der erfindungsgemäßen Vorrichtung und ihrer Wirkungsweise. Sie sind deshalb auch nicht maßstäblich ausgeführt, sondern betonen ihre wesentlichen Merkmale. Sie sind auch nur als beispielhaft und nicht als einschränkend aufzufassen. Es zeigt
- Figur 1: die Draufsicht auf einen mittigen Längsschnitt durch die erfindungsgemäße Vorrichtung 1;
- Figur 2: die Draufsicht auf das Heizsystem mit Peltier-Elementen 2, Wärmeleitungen 2.1.1 und Heizplatte 2.1.2;
- Figur 3: die Draufsicht auf einen Querschnitt durch die erfindungsgemäße Vorrichtung 1 auf der Höhe des umlaufenden Kühlrings 2.4 und
- Figur 4: die frontale Ansicht einer erfindungsgemäße Vorrichtung 1 mit elektronischem Steuergerät 4.

In den Figuren 1 bis 4 haben die Bezugszeichen die folgende Bedeutung:
- 1: Durch eine Temperaturdifferenz betriebene, mobile Vorrichtung zur Reinigung und Desinfizierung von Raumluft
- 1.1: Vertikale Außenwand
- 1.2: Obere Trennstelle
- 1.3: Untere Trennstelle
- 1.4: Oberes Ende der Außenwand 1.1
- 1.5: Obere Öffnung der Vorrichtung 1
- 1.6: Obere Öffnung der Kartusche 5
- 1.7: Bodenbereich
- 1.7.1: Standfläche
- 1.7.2: Raum für den Einschub des wieder aufladbaren Akkumulators 7 und des elektronischen Steuergeräts 4
- 1.7.3: Einschubschiene
- 1.8: Horizontaler Innenboden
- 1.9: Vertikale Innenwand
- 2: Peltier-Element
- 2.1: Heiße Seite des Peltier-Elements
- 2.1.1: Wärmeleitung
- 2.1.2: Heizplatte
- 2.1.3: Heiz- und Luftlenkkegel
- 2.1.4: Wärmedämmbeschichtung, Wärmedämmfarbe
- 2.1.5: Thermisch isolierender Pfropfen zur Schwingungsdämpfung oder mit seitlicher horizontaler Verlängerung zur Schwingungsdämpfung und als Strömungssperre
- 2.1.6: Heizraum
- 2.2: Kalte Seite des Peltier-Elements
- 2.3: Wärmeleitpaste, AIN-Keramikplatte
- 2.4: Kühlring im oberen Bereich der Kartusche 5
- 3: Lufteinlass
- 3.1: In den Heizraum 2.1.5 einströmende Luft 3
- 3.2: Durch die Kartusche 5 aufwärtsströmende Luft, Luftstrom
- 4: Elektronisches Steuergerät
- 4.1: Einschalt- und Regelknopf
- 4.2: Buchse für Ladekabel
- 4.3: Leuchtdioden
- 4.4: Anzeige der Temperaturdifferenz zwischen dem Heizraum 2.1.6 und dem Kühlring 2.4
- 5: Auswechselbare Kartusche
- 5.1: Kartuschenwand mit geringer Wärmeleitfähigkeit λ
- 5.2: Biozide partikuläre Kartuschenfüllung oder luftdurchlässige, biozide Schüttung mit geringer Wärmeleitfähigkeit λ
- 5.2.1: Partikuläre, anorganische, bakterizid und viruzid beschichtete, inerte Trägermaterialien
- 5.2.2: Partikuläre, anorganische, bakterizide und/oder viruzide Materialien
- 5.3: Luftdurchlässiger Kartuschenboden mit geringer Wärmeleitfähigkeit λ
- 5.4: Umlaufende Kartuschenhalterung mit geringer Wärmeleitfähigkeit λ
- 5.5: Umlaufendes, wärmebeständiges stützendes Bauteil mit geringer Wärmeleitfähigkeit λ in der Form eines Trichteroberteils mit Standbeinen 5.5.1 zur Luftlenkung und Stütze der Kartusche 5
- 5.5.1: Standbeine für 5.5
- 5.6: Gitterstreben mit hoher Wärmeleitfähigkeit λ
- 6: Filter

- A-B: Lange Achse des Oktagons
- C-D: Kurze Achse des Oktagons

### Ausführliche Beschreibung der Figuren

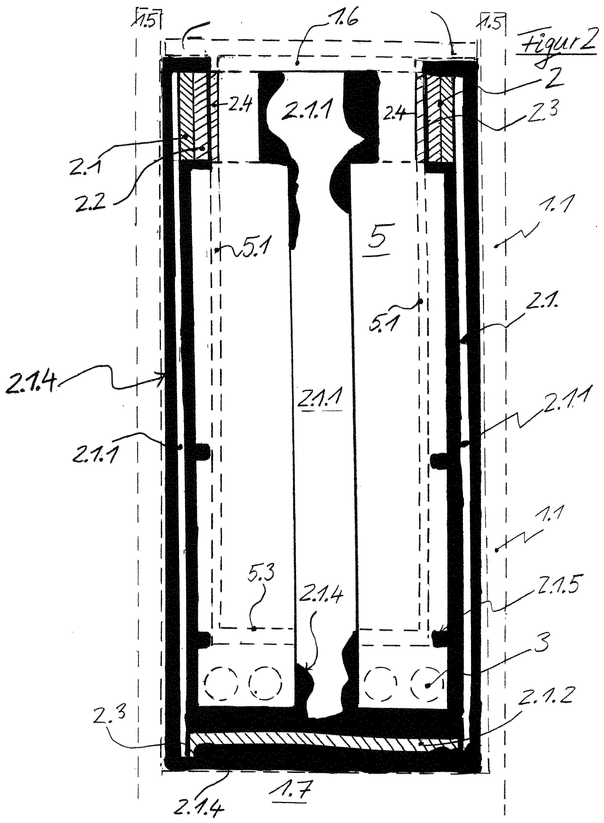

### Figur 1 in Verbindung mit den Figuren 2 bis 4

### Herstellbeispiel

### Die Herstellung von bakterizid und/oder viruzid beschichteten, inerten, partikulären Trägermaterialien 5.2.1 ― Allgemeine Vorschrift

### Die Herstellung eines Böhmit-Sols

2,78 Gewichtsteile Böhmit (Disperal ^{®} P 3 der Firma Sasol Germany GmbH) wurden zu 25 Gewichtsteilen verdünnter Salzsäure (0,1 N) gegeben und bei Raumtemperatur solange gerührt, bis das Böhmit vollständig gelöst war. Anschließend wurde die kolloidale Lösung während 5 min in einem Ultraschallbad behandelt. Es resultierte ein homogenes Böhmit-Sol.

### Die Herstellung einer Dispersion eines Copolymerisats

In einem Reaktionsgefäß, ausgerüstet mit einem Rührer und vier Zulaufgefäßen, wurden 1.361,1 Gewichtsteile deionisiertes Wasser vorgelegt und auf 75 °C erhitzt. Bei dieser Temperatur wurden drei Zuläufen parallel und gleichzeitig über einen Zeitraum von 30 min zudosiert. Zulauf 1 bestand aus 24,4 Gewichtsteilen Acrylsäure, 44 Gewichtsteilen Methylmethacrylat und 3,6 Gewichtsteilen Diphenylethylen. Zulauf 2 bestand aus einer 25-prozentige Ammoniaklösung. Zulauf 3 bestand aus einer Lösung von 5,4 Gewichtsteilen Ammoniumperoxodisulfat in 138,7 Gewichtsteilen deionisiertem Wasser. Nach der Zugabe wurde das resultierende Reaktionsgemisch noch während einer Stunde bei 75 °C nachpolymerisiert und anschließend auf 90 °C erhitzt. Bei dieser Temperatur wurde das Reaktionsgemisch während 4 h mit dem Zulauf 4 versetzt. Zulauf 4 bestand aus 191,7 Gewichtsteilen n-Butylmethyacrylat, 153,4 Gewichtsteilen Styrol, 93,3 Gewichtsteilen Hydroxypropylmethacrylat, 424,9 Gewichtsteilen Hydroxethylmethacrylat, 173,1 Gewichtsteilen Ethylhexylmethacrylat und 207,3 Gewichtsteilen einer 50-prozentigen Lösung von Tris(alkoxycarbonylamino)triazin (TACT^{®}; Vernetzungsmittel; vgl. Tabelle 3) in n-Butanol. Anschließend wurde das Reaktionsgemisch noch weitere 2 h bei 90 °C nachpolymerisiert und anschließend abgekühlt.

### Die Herstellung einer organisch-anorganischen Hybridpolymerdispersion als Beschichtungsstoff oder Beschichtungsmittel

Zu 5,5 Gewichtsteilen des vorstehend beschriebenen Böhmit-Sols wurden 3,3 Gewichtsteile GLYEO gegeben. Das so erhaltene Reaktionsgemisch wurde während 90 min bei Raumtemperatur gerührt. Anschließend wurden 2,2 Gewichtsteile Glycidyloxipropyltrimethoxysilan (GLYMO) zugegeben. Nach zweistündigem Rühren bei Raumtemperatur wurden 0,55 Gewichtsteile Acetessigsäureethylester (EEA) hinzugegeben, wonach das resultierende Reaktionsgemisch weitere 2 h bei Raumtemperatur gerührt wurde. Anschließend wurden 0,75 Gewichtsteilen Isopropanol zugegeben. Das resultierende Reaktionsgemisch wurde während 15 min gerührt, mit 1,25 Gewichtsteilen der Dispersion des Copolymerisats versetzt und weitere 2 h bei Raumtemperatur gerührt.

### Beschichtungsverfahren 1 - Allgemeine Vorschrift

Die verwendete Beschichtungsanlage war gegen elektrostatische Ladung gesichert und wies eine Absaugung auf. Die unbeschichteten, partikulären, anorganischen, inerten Trägermaterialien 5.2.1 wurden mithilfe einer automatischen Zuführungsvorrichtung auf ein über mindestens zwei Transportrollen geführtes glattes, mit einer Antihaftschicht beschichtetes Endlosband einer Gesamtlänge von 42 m mit einer horizontalen Rüttelstrecke von 20 m Länge dosiert. Die Rüttelstrecke wies seitliche Wände auf, um das Herunterfallen des unbeschichteten Trägermaterials zu verhindern Die zugeführten unbeschichteten Trägermaterialien 5.2.1 wurden gerüttelt und in einer ersten Station einer Länge von 8 m bei 30 °C mit dem flüssigen Beschichtungsstoff besprüht, sodass sich eine dünne unausgehärtete flüssige Schicht auf dem Trägermaterial bildete, die durch Verdunsten allmählich klebrig wurde. Durch das Rütteln wurde sichergestellt, dass die Trägermaterialien möglichst gleichmäßig beschichtet wurden und nicht verklebten. Bevor der Beschichtungsstoff ausgehärtet wurde, wurde er in einer zweiten Station einer Länge 5 m von unter Rütteln mit Mikropartikeln mindestens einer Art von Bioziden und/oder Viruziden besprüht, sodass die Mikropartikel auf und in dem Beschichtungsstoff gleichmäßig verteilt wurden und kleben blieben. In einer dritten Station wurden der nunmehr biozide und/oder viruzide Beschichtungsstoff - ebenfalls unter Rütteln - thermisch in einem 5 m langen Durchlaufofen bei 110 °C gehärtet, sodass sich die biozide und/oder viruzide Beschichtung auf dem Trägermaterial 5.2.1 bildete. Durch das Rütteln wurde verhindert, dass die Partikel 5.2.1 zusammenklebten und/oder auf dem Endlosband festklebten. Sofern dies doch in einem gewissen Umfang geschah, wurden die Partikel 5.8 in einer 2 m langen dritten Station vereinzelt und danach in ein Vorratsgefäß ausgetragen. Die an dem Endlosband gegebenenfalls anhaftenden Reste von ausgehärtetem Beschichtungsstoff wurden nach dem Umlenken des Endlosbands mit Rakeln abgekratzt.
- Als beschichtetes Trägermaterial 5.2.1 wurden bei einer ersten Ausführungsform die Granulate aus unregelmäßig geformten Schaumglasschottern mit einer durch Siebanalyse ermittelten mittleren Teilchengröße von 2 mm mit der viruziden und bakteriziden Beschichtung dünn beschichtet. Die Beschichtung enthielt, bezogen auf 1000 Gewichtsteile der Beschichtung, 3,2 Gewichtsteile 2-Octyl-2H-isothiazol-3-on, 2,0 Gewichtsteile, 1,0 Gewichtsteile Zinkpyrithion und 2,0 Gewichtsteile (±)-1-{[2-(2,4-dichlorophenyl)-4-propyl-1,3-dioxolane-2-yl]-methyl}-*H*-1,2,4-triazol.
- Bei einer zweiten Ausführungsform wurden die gemahlenen Brocken aus Bimsstein einer durch Siebanalyse ermittelten mittleren Teilchengröße von 2,5 mm verwendet. Die dünne, biozide und/oder viruzide Beschichtung enthielt, bezogen auf 1000 Gewichtsteile der Beschichtung, 1,1 Gewichtsteile Mikrokristalle von Cu₂{H₄[Si(W₃O₁₀)₄]} · xH₂O, 1,0 Gewichtsteile Fludioxonil: 4-(2,2-Difluor-benzo[1,3]dioxol-4-yl)pyrrol-3-carbonitril (IUPAC) und 2,0 Gewichtsteile Octenidin: *N*,*N'*-(Decan-1,10-diyldi-1(4*H*)-pyridyl-4-yliden)bis(octylammonium)dichlorid.
- Bei einer dritten Ausführungsform wurden die Raschigringe aus Glas einer Länge von 1 cm, eines Wandstärke von 2 mm und einer lichten Weite von 1,6 cm verwendet. Die dünne, biozide und/oder viruzide Beschichtung enthielt, bezogen auf 1000 Gewichtsteile der Beschichtung, 2 Gewichtsteile Diuron: 3-(3,4-Dichlorphenyl)-1,1-dimethylharnstoff und 3 Gewichtsteile Quaternisiertes Chitosan (vg., A. Domard et al., "New method for the quaternization of chitosan", International Journal of Biological Macromolecules, Band 8, Ausgabe 2, April, 1986, Seiten 105-107).

### Beschichtungsverfahren 2 ― Allgemeine Vorschrift

Die bei dem Beschichtungsverfahren 1 beschriebene Beschichtungsanlage wurde dahingehend modifiziert, dass anstelle einer Sprühanlage für flüssige Beschichtungsstoffe eine Sprühanlage für das elektrische Doppeldrahtsprühen (twin-wire arc spraying) verwendet wurde.
- Als beschichtetes Trägermaterial 5.2.1 wurden bei einer ersten Ausführungsform die vorstehend beschriebenen Granulate aus unregelmäßig geformten Schaumglasschottern mit elektrischem Doppeldrahtsprühen Kupfer besprüht.
- Bei einer zweiten Ausführungsform wurden die vorstehend beschriebenen gemahlenen Brocken aus Bimsstein verwendet, die ebenfalls mit Kupfer besprüht wurden.
- Bei einer dritten Ausführungsform wurden die vorstehend beschriebenen Raschigringe aus Glas mit Kupfer besprüht.
- In den vierten fünftens und sechsten Ausführungsformen wurden die Trägermaterialien 5.2.1 der ersten, zweiten und dritten Ausführungsformen nach dem analogen Verfahren mit Silber besprüht.

In allen Fällen resultierten inselartige und punktförmige, bakterizid und viruzid hochwirksame, dünne Metallbeschichtungen. Wegen dieser Art der Beschichtung konnten erhebliche Mengen an den teuren bioziden Metallen eingespart werden, ohne dass die biozide Wirkung beeinträchtigt wurde. Außerdem konnte im Vergleich zu einer Schüttung aus reinem Metall sehr viel Gewicht eingespart werden.

### Die erfindungsgemäßen Vorrichtungen 1 ― Allgemeine Beschreibung

Zur Durchführung der Wirksamkeitstests wurden mehrere erfindungsgemäße Vorrichtungen 1 hergestellt und mit Schüttungen aus den vorstehend beschriebenen beschichteten Trägermaterialien 5.2.1 und den bioziden Materialien 5.2.2 wie nachstehend beschrieben befüllt. Als Adsorbens und/oder Absorbens wurde getrockneter, plättchenförmiger Bentonit (Schichtsilikat, Phyllosilikat) einer durch Siebanalyse ermittelten mittleren Teilchengröße von 3 mm verwendet.

Die erfindungsgemäßen Vorrichtungen 1 waren von ihren Standfläche 1.7.1 bis zu ihren oberen Enden 1.4 100 cm hoch. Sie wiesen achteckige Außenwände 1.1 mit jeweils zwei einander gegenüberliegenden vertikalen planen Flächen oder Seiten eines jeweiligen horizontalen Außendurchmessers von 4 cm auf. Die vertikalen planen Flächen waren mit konvex gebogenen Flächen mit einem jeweiligen horizontalen Außendurchmesser von 5 cm verbunden. Die Außenwände 1.1 bestanden aus poliertem massivem Buchenholz einer Wanddicke von 0,8 cm. Die Außenwände 1.1 wiesen jeweils eine obere horizontal umlaufende Trennstelle 1.2 auf der halben Höhe der Außenwände 1.1 und jeweils eine untere horizontal umlaufende Trennstelle 1.3 oberhalb der Lufteinlässe 3 in Höhe der Heizräume 2.1.6 auf. Beide Trennstellen 1.2; 1.3 wurden von Steckverbindungen gebildet. Sie dienten dem leichteren Zusammenbau und der leichteren Wartung der erfindungsgemäßen Vorrichtungen 1. In den Bereichen der horizontalen Standflächen 1.7.1 waren die Außenwände 1.1 1,5 cm dick. Die Bodenbereiche 1.7 waren bis zu den horizontalen Innenböden 1.8 insgesamt 6 cm dick und wiesen horizontale Räume 1.7.2 für den Einschub wieder aufladbarer Akkumulatoren 7 und elektronischer Steuergeräte 4 auf. Diese Bauteile wurden auf Einschubschienen 1.7.3 in die Bodenbereiche 1.7 eingeschoben. Die vertikalen Innenwände 1.9 waren 94 cm hoch. Sie und die horizontalen Innenböden 1.8 waren mit einer 1 cm dicken Wärmedämmbeschichtung 2.1.4 aus einer Aerogel enthaltenden Wärmedämmfarbe beschichtet.

Die von den Außenwänden 1.1 gebildeten horizontalen Querschnitte waren somit Oktagone mit einer langen Achse A-B von Außenwand zu gegenüberliegender Außenwand einer Länge von 13 cm und einer durch den Mittelpunkt der Achse A-B quer verlaufende Achse C-D von Außenwand zu gegenüberliegender Außenwand einer Länge von 9,5 cm. Längs der vier planen Seiten verliefen die 4 cm breiten, 88 cm langen und 0,5 cm dicken vertikalen planen Wärmeleitungen 2.1.1 aus einer Aluminiumlegierung einer Wärmeleitfähigkeit λ von 200 W/m.K), die an ihren oberen Endbereichen in wärmeleitendem Kontakt mit den vier Peltier-Elementen 2 der Abmessungen 4 cm mal 4 cm mal 1,5 cm standen. In ihren unteren Endbereichen standen sie in wärmeleitendem Kontakt mit den 1,5 cm dicken, oktagonförmigen Heizplatten 2.1.2 aus der Aluminiumlegierung. Die Heizplatten 2.1.2 waren auf den Wärmedämmbeschichtungen 2.1.4 auf den horizontalen Innenböden 1.8 aufgelegt. Zwischen den Kontaktstellen befanden sich dünne Schichten aus Wärmeleitpasten, die Kohlenstoffnanopartikel enthielten.

Im Zentrum der Heizplatten 2.1.2 befanden sich 2 cm hohe Heiz- und Luftlenkkegel 2.1.3 deren jeweilige Basis einen Durchmesser von 2 cm hatte. Die Heiz- und Luftlenkkegel 2.1.3 waren integrale Bestandteile der Heizplatten 2.1.2.

Die Innenseiten und oberen Enden der Wärmeleitungen 2.1.1 sowie die jeweils drei freien Seitenflächen der Peltier-Elemente 2 und die umlaufenden vertikalen Seiten der Heizplatten 2.1.2 waren ebenfalls mit 1 cm dicken Wärmedämmbeschichtungen 2.1.4 aus der vorstehend aufgeführten Wärmedämmfarbe beschichtet. Diese Wärmedämmbeschichtungen 2.1.4 reichten bis zu den Oberflächen der Heizplatten 2.1.2. Auf den Heizplatten standen umlaufende, wärmebeständige, stützende Bauteile 5.5 aus dem Hochleistungskunststoff Polyethersulfon (PES) einer Glastemperatur von 225 °C und einer Wärmeleitfähigkeit λ von 0,17 W/(m.K). Die Bauteile 5.5 hatten die Form umgedrehter Trichteroberteile mit einem oktagonförmigen Umriss einer Wandstärke von 0,3 cm und einer Höhe von 2 cm. Sie standen auf jeweils vier 3 cm hohen Standbeinen 5.5.1 aus PES mit kreisförmigen Querschnitt eines Durchmessers von 0,3 cm, die räumlich den jeweils vier Wärmeleitungen 2.1.1 zugeordnet waren.

Die Bauteile 5.5 stützten die luftdurchlässigen Kartuschenböden 5.3 einer Wandstärke von 0,4 cm und Bohrungen einer lichten Weite von 0,15 cm in den Bereichen der Übergänge von den luftdurchlässigen Kartuschenböden 5.3 in die umlaufenden oktagonförmigen Kartuschenwände 5 aus PES einer Wandstärke von 0,4 cm ab. Die Innenseiten der Kartuschen 5 waren insgesamt 81,5 cm hoch. An ihren oberen Enden gingen die Kartuschenwände 5.1 in umlaufende oktagonförmige Flansche als Kartuschenhalterungen 5.4 einer Wandstärke von 0,4 cm und einer Breite von 3 cm über, die auf den horizontalen Wärmedämmbeschichtungen 2.1.4 auflagen. Die Kartuschen 5 wiesen längs der langen Achsen A-B Abstände von Außenwand zu gegenüberliegender Außenwand von 7,7 cm auf. Längs der kurzen Achsen C-D wiesen sie Abstände von Außenwand zu gegenüberliegender Außenwand von 4,2 cm auf.

1,4 cm unterhalb der oberen Öffnungen 1.6 der Kartuschen 5 waren 2 cm hohe umlaufende oktagonförmige Kühlringe 2.4 aus Kupfer einer Wandstärke von 0,4 cm angebracht, die in wärmeleitendem Kontakt mit den kalten Seiten 2.3 der Peltier-Elemente 2 standen. Zum Anbringen der Kühlring der 2.4 wurden die durch Spritzguss hergestellten Vorprodukte der Kartuschen 5 an den passenden Stellen zerschnitten, und es wurden die Kühlringe 2.4 mithilfe eines Konstruktionsklebstoffs eingesetzt und verklebt. Die Wände der Kühlringe 2.4 waren mit einem weitmaschigen Gitter aus 1 mm dünnen Streben 5.6 aus Kupferdraht verbunden. Dadurch wurden die Kühlwirkung und damit der Kamineffekt signifikant erhöht.

In den jeweils vier konvex gebogenen Flächen der Außenwände 1.1 befanden sich etwas oberhalb des Niveaus der Heizplatten 2.1.2 jeweils zwei rohrförmige Lufteinlässe 3 aus PES mit einem kreisförmigen Querschnitt einer Wandstärke von 0,15 cm und einer lichten Weite von 1,5 cm. Die rohrförmigen Lufteinlässe 3 verliefen horizontal durch die Wärmedämmbeschichtungen 2.1.4 zwischen den Wärmeleitungen 2.1.1 in die Heizräume 2.1.6. In den Heizräumen 2.1.6 wurde die einströmende Luft 3.1 aufgeheizt und mithilfe des Kamineffekts als Luftstrom 3.2 durch die durchlässigen Kartuschenböden 5.3 und die Kartuschen 5 mit den bioziden partikulären Kartuschenfüllungen gesaugt und durch die oberen Öffnungen 1.5; 1.6 dekontamiert in die Raumluft geblasen. Die Einlässe selbst waren mit Vorfiltern einer kleinsten filtrierbaren Partikelgröße von 1 µm verschlossen, um das Eindringen grober Partikel zu verhindern.

Die Kartuschwände 5.1 wiesen an ihren Innenseiten jeweils zwei umlaufende 0,3 cm horizontal vorspringende und 0,3 cm starke Ringe zur Auflage von dünnen luftdurchlässigen Zwischenböden mit Bohrungen einer lichten Weite von 0,15 cm auf (nicht dargestellt). Die ersten Zwischenböden waren 30 cm oberhalb der Kartuschenböden 5.3 angeordnet. Die zweiten Zwischenböden befanden sich 35 cm oberhalb der ersten Zwischenböden. Dadurch wurden jeweils drei Kammern gebildet, von denen die unterste mit partikulären, anorganischen, bakterizid und viruzid wirkenden, lockeren, luftdurchlässigen Schüttungen 5.2.2 aus unregelmäßig geformten Brocken einer Mischung von Kalk und Magnesium-Calcium-Oxid im Gewichtsverhältnis von 1:1 einer durch Siebanalyse ermittelten mittleren Teilchengröße von 2,5 mm gefüllt wurden. Die zweiten Kammern wurden mit lockeren Schüttungen aus jeweils einer Art der vorstehend beschriebenen, partikulären, anorganischen, bakterizid und viruzid beschichteten, inerten Trägermaterialien 5.2.1 gefüllt. Die obersten Kammern wurden mit lockeren Schüttungen aus Bentonit-Partikeln bis zu den oberen Öffnungen 1.6 der Kartuschen 5 gefüllt.

Im Bedarfsfall konnten in die oberen Öffnungen 1.5 der Vorrichtungen 1 noch Filter 6 mit unterschiedlichen kleinsten filtrierbaren Partikelgrößen eingelegt werden.

In den oberen Öffnungen 1.6 der Kartuschen 5 befanden sich jeweils ein Miniaturanemometer und ein Temperatursensor (nicht dargestellt), und in den Heizräumen 2.1.6 befand sich jeweils ein Temperatursensor (nicht dargestellt), die über Signalleitungen (nicht dargestellt) mit den elektronischen Steuergeräten 4 verbunden waren. Dadurch konnten die Leistungsabgaben der Peltier-Elemente 2 und damit die Temperaturen in den Heizplatten 2.1.2 und den gekühlten Öffnungen 1.6 der Kartuschen 5 und damit auch der Kamineffekt und die Stärke der Luftströme 3.2 geregelt werden. Die Temperaturdifferenzen zwischen den unteren und den oberen Temperatursensoren konnten an den Anzeigen 4.4 der elektronischen Steuergeräte 4 abgelesen werden und mithilfe der Einschalt- und Regelknöpfe 4.1 eingestellt werden. Außerdem enthielten die elektronischen Steuergeräte 4 noch Leuchtdioden 4.3 zur Funktionsanzeige und jeweils eine Buchse 4.2 für ein Ladekabel zum Aufladen der Akkumulatoren 7.

Wie vorstehend aufgezeigt, konnten durch die erfindungsgemäße Bauweise die unterschiedlichsten erfindungsgemäßen Vorrichtungen 1 verwirklicht werden, die den Bedingungen des Einzelfalls hervorragend angepasst werden konnten.

Waren die Schüttungen in den Kartuschen 5, insbesondere die Schüttungen der Absorbentien und/oder Adsorbentien, nach sehr lange Gebrauchsdauer so stark durch die Zersetzungsprodukte der Bakterien, Viren und Virionen kontaminiert, dass ein Austausch der Materialien notwendig wurde, konnten die kontaminierten Schüttungen durch mechanochemische Verfahren, wie sie beispielsweise in der deutschen Offenlegungsschrift DE 10 2019 006 084 A1 beschrieben werden, aufbereitet werden. Bei diesen Verfahren wuden die organischen Materialien, die an und/oder in den Schüttungen adsorbiert und/oder absorbiert waren, in Aktivkohle umgewandelt werden. Aufgrund ihrer geringeren Dichte konnte die erzeugte Aktivkohle durch Sichtung von den anorganischen Materialien mit höherer Dichte abgetrennt und anderweitig verwendet werden. Auch dies war ein weiterer wesentlicher Vorteil der erfindungsgemäßen Vorrichtung 1 und des erfindungsgemäßen Verfahrens.

### Test der Wirksamkeit der erfindungsgemäßen Vorrichtungen 1

Die Wirksamkeit der vorstehend beschriebenen erfindungsgemäßen Vorrichtungen 1 wurde mithilfe einer Testvorrichtung getestet. Dazu wurde jeweils eine erfindungsgemäße Vorrichtung 1 in eine entsprechend dimensionierte geschlossene Kammer platziert. Die Ausgangsöffnung der erfindungsgemäßen Vorrichtung 1 wurde mit einer Abzugshaube mit einer Luftableitung verbunden, durch die die Abluft ausgeleitet werden konnte. Der Übergang von der erfindungsgemäßen Vorrichtung 1 zur Abzugshaube war an dem oberen Ende mit einer umlaufenden Dichtung abgedichtet. In die Abzugshaube wurde über eine Schleuse ein festes zellbiologisches Nährmedium auf einer zuvor sterilisierten Folie auf einen luftdurchlässigen Träger platziert. In der Luftableitung waren ein Sensor für die Partikelzählung durch Laserbeugung, der mit einer Signalleitung mit einem Partikelmessgerät verbunden war, und ein Sensor für die Strömungsmessung, der mit einer Signalleitung mit einem Strömungsmessgerät verbunden war, angeordnet. Ein weiterer Sensor für die Partikelzählung war im Bereich der Lufteinlässe der jeweiligen erfindungsgemäßen Vorrichtung platziert und über eine Signalleitung mit einem weiteren Partikelmessgerät verbunden. Die zwei Messgeräte und das Messgerät sendeten die Messsignale an eine zentrale Datenverarbeitungsanlage, worin sie ausgewertet wurden und auf einem Anzeigegerät wiedergegeben wurden.

Vor den Bestimmungen der Wirksamkeit wurden die jeweilige Kammer und die jeweilige erfindungsgemäße Vorrichtung 1 mit hochreiner Nullpunktluft (analytische Luft) bei 23 °C und 1,2 bar über einen Anschluss, ein automatisch geregeltse Absperrventil und eine Lufteinlassdüse gespült. Sobald an dem Anzeigegerät keine Partikelsignale mehr auftauchten wurde die Spülung durch Schließen des Absperrventil beendet, und es wurden Aerosolwolken, die der Sicherheit wegen harmlose Darmbakterien wie Firmicutes, Bacteroidetes, Proteobacteria und Actinobateria enthielten, über eine Aerosolzuleitung, ein Gebläse, ein geöffnetes Absperrventil und eine im Inneren der Kammer befindliche Vernebelungsdüse bei 23 °C und 1,2 bar in die Kammer geblasen, durch die jeweilige erfindungsgemäße Vorrichtung 1 geleitet und über die Abzugshaube und die Luftableitung abgelassen.

In der Abzugshaube wurde die Abluft direkt auf das feste zellbiologische Nährmedium auf der zuvor sterilisierten Folie geblasen.

Nach einer Exposition von 15 Minuten, 30 Minuten, 45 Minuten, 60 Minuten wurde das zellbiologische Nährmedium auf der zuvor sterilisierten Folie aus der Entnahmeschleuse entnommen, und es wurde sofort ein neues Nährmedium eingeschleust. Danach wurde jeweils in üblicher und bekannter Weise getestet, ob sich noch vermehrungsfähige Darmbakterien auf dem Nährmedium befanden. Es zeigte sich, dass bereits nach 15 Minuten der Exposition keine vermehrungsfähigen Darmbakterien vorhanden waren. Dies befand sich in Übereinstimmung mit der Partikelmessung mit dem Sensor und dem Partikelmessgerät in der Abluft, mit denen keine Partikel in der fraglichen Größenordnung mehr festgestellt werden konnten.

## Patentansprüche

1. Durch eine Temperaturdifferenz betreibbare, mobile Vorrichtung (1) zur Reinigung und Desinfizierung von Raumluft, umfassend, von außen nach innen gesehen,
- eine vertikale, rohrförmige Außenwand (1.1) mit einem Bodenbereich (1.7) mit einem horizontalen Standboden (1.7.1), mindestens einer oberen Trennstelle (1.2), mindestens einer unteren Trennstelle (1.3), einer oberen Öffnung (1.5) am oberen Ende (1.5), mindestens einem Raum (1.7.2) für den Einschub mindestens eines wieder aufladbaren Akkumulators (7) und eines elektronischen Steuergeräts (4) zur Regelung des durch den Kamineffekt erzeugbaren Luftstrom (3.2) durch die Kartusche (5),
- eine auswechselbare Kartusche (5) mit einer oberen Öffnung (1.5), einer die obere Öffnung (1.5) umlaufenden Kartuschenhalterung (5.4) mit geringer Wärmeleitfähigkeit λ, einer Kartuschenwand (5.1) mit geringer Wärmeleitfähigkeit λ und einem luftdurchlässigen Kartuschenboden (5.3) mit geringer Wärmeleitfähigkeit λ und einem Kühlring (2.4) mit hoher Wärmeleitfähigkeit λ im oberen Bereich der Kartusche (5), der in wärmeleitendem Kontakt mit den kalten Seiten (2.2) von mindestens zwei Peltier-Elementen (2) steht,
- mindestens eine in der auswechselbaren Kartusche (5) enthaltenen, bioziden, partikulären Kartuschenfüllung (5.2) in der Form mindestens einer luftdurchlässigen Schüttung, enthaltend (i) mindestens eine Art eines partikulären, anorganischen, bakterizid und/oder viruzid beschichteten, inerten Trägermaterials (5.2.1) und/oder (ii) mindestens eine Art eines partikulären, anorganischen, per se bakteriziden und/oder viruziden Materials (5.2.2) sowie (iii) mindestens eine Art eines partikulären, anorganischen und/oder metallorganischen Absorbens und/oder Adsorbens (5.2.3),
- mindestens zwei einander gegenüberliegende, unterhalb der oberen Öffnung (1.6) der Kartusche (5) und der umlaufenden Kartuschenhalterung (5.4) vertikal angeordnete Peltier-Elemente (2), die mit ihren kalten Seiten (2.2) mit dem Kühlring (2.4) und mit ihren heißen Seiten (2.1) in wärmeleitendem Kontakt zu mindestens zwei vertikalen Wärmeleitungen (2.1.1) stehen,
- eine auf dem Bodenbereich (1.7) angeordnete, horizontale Heizplatte (2.1.2), die in wärmeleitendem Kontakt zu den mindestens zwei vertikalen Wärmeleitungen (2.1.1) steht, sodass sie aufheizbar ist,
- einen Heizraum (2.1.6) oberhalb der Heizplatte (2.1.2) zum Aufheizen der durch mindestens zwei Lufteinlässe (3) einströmenden Luft (3.1),
- eine Wärmedämmungsbeschichtung (2.1.4), die die Innenseite der Außenwand (1.1) und die Wärmeleitungen (2.1.1) gänzlich, die mindestens zwei Peltier-Elemente (2) seitlich und die Heizplatte (2.1.2) bis auf deren Oberseite umhüllt.

2. Mobile Vorrichtung nach Anspruch 1, **dadurch gekennzeichnet, dass** die Kartuschenwand (5.1) durch mindestens zwei thermisch isolierende Pfropfen (2.1.5) zur Schwingungsdämpfung oder mit seitlicher horizontaler Verlängerung als Strömungssperre gehalten ist oder durch ein umlaufendes, wärmebeständiges, als Luftlenkung dienendes Bauteil (5.5) mit geringer Wärmeleitfähigkeit λ in der Form eines Trichteroberteils an dem unteren Ende der Kartuschenwand (5.1) gestützt ist.

3. Mobile Vorrichtung nach Anspruch 1 oder 2, **dadurch gekennzeichnet, dass** die Wärmedämmungsbeschichtung (2.1.4) aus Dämmfarben, ausgewählt aus der Gruppe bestehend aus Korkfarben, diffusionsoffenen Dämmfarben auf Silikonbasis, Dämmfarben mit einem hohen Anteil an keramischen Hohlkugeln, Dämmfarben mit Aerogelen, Schaumzement und Dämmfarben mit mikrofeinen Hohlglaskugeln, hergestellt ist.

4. Mobile Vorrichtung nach einem der Ansprüche 1 bis 3, **dadurch gekennzeichnet, dass** die mindestens eine luftdurchlässige biozide Schüttung (5.2) (i) mindestens eine Art eines partikulären, anorganischen, bakterizid und/oder viruzid beschichteten, inerten Trägermaterials (5.2.1) und/oder (ii) mindestens eine Art eines partikulären, anorganischen, bakteriziden und/oder viruziden Materials (5.2.2) enthält.

5. Mobile Vorrichtung (1) nach Anspruch 4, **dadurch gekennzeichnet, dass**
- die mindestens eine Art eines partikulären, anorganischen, bakterizid und/oder viruzid beschichteten, inerten Trägermaterials (5.2.1) aus der Gruppe, bestehend aus Füllkörpern für Kolonnen, Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen von Schichtsilikaten, Schaumgläsern, Schaumglasschottern Bimssteinen, Zeolithen, Blähtonen, Blähgläsern, Blähglimmern, Blähperliten, Schaumzementen und Keramikschäumen, ausgewählt ist,
- die mindestens eine Art eines partikulären, anorganischen, bakteriziden und/oder viruziden Materials (5.2.2) aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen und Schäumen, ausgewählt ist und
- die mindestens eine Art eines partikulären, anorganischen und/oder metallorganischen Adsobentien und/oder Absorbentien (5.2.3) aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen ausgewählt.

6. Mobile Vorrichtung (1) nach Anspruch 4 oder 5, **dadurch gekennzeichnet, dass** die bakteriziden und viruziden Beschichtungen auf dem inerten Trägermaterial (5.2.1) mindestens eine Art von bakteriziden und viruziden Pharmazeutika und/oder Desinfektionsmitteln enthalten, die an einer ausgehärteten Bindemittelmatrix in der Form von Mikropartikeln fixiert und/oder in der ausgehärteten Bindemittelmatrix dispergiert und/oder darin gelöst sind, und/oder durch elektrisches Doppeldrahtsprühen auftragbare bakterizide und viruzide Metallpartikel enthalten

7. Mobile Vorrichtung (1) nach einem der Ansprüche 4 bis 6, **dadurch gekennzeichnet, dass** die bakteriziden und/oder viruziden Beschichtungen auf dem inerten Trägermaterial (5.2.1) einen Kontaktwinkel θ mit Wasser von ≤90° haben.

8. Mobile Vorrichtung (1) nach einem der Ansprüche 1 bis 7, **dadurch gekennzeichnet, dass** der Querschnitt der auswechselbaren Kartusche (5) kreisförmig, elliptisch, oval, quadratisch, viereckig, sechseckig oder achteckig ist und von einer über die gesamte Länge der Kartuschenwand (5.1) hinweg bis zur umlaufenden Kartuschenhalterung (5.4) von konstanter lichter Weite ist oder die Form eines Venturirohrs hat.

9. Mobile Vorrichtung (1) nach einem der Ansprüche 1 bis 8, **dadurch gekennzeichnet, dass** die Innenseite der Kartuschenwand (5.1) der auswechselbaren Kartusche (5) mit konstanter lichter Weite mindestens zwei horizontal umlaufende und vertikal voneinander beabstandete Auflagen für mindestens zwei luftdurchlässige Zwischenböden aufweist, sodass mindestens drei voneinander getrennte Kammern resultieren, von denen mindestens eine mit einer Schüttung (5.2) aus partikulären Absorbentien und/oder Adsorbentien (5.2.3) gefüllt ist und mindestens zwei mit (i) einer Schüttung (5.2) aus partikulären, anorganischen, bakterizid und viruzid beschichteten, inerten Trägermaterialien (5.2.1) und/oder (ii) mit einer Schüttung (5.2) aus partikulären, anorganischen, bakterizid und/oder viruziden Materialien (5.2.2) gefüllt sind.

10. Mobile Vorrichtung (1) nach einem der Ansprüche 5 bis 9, **dadurch gekennzeichnet, dass** die auswechselbare Kartusche (5) mit der Form eines Venturirohrs mit einer Schüttung (5.2) gefüllt ist, die ein Gemisch, enthaltend (i) partikuläre, anorganische, bakterizid und viruzid beschichtete, inerte Trägermaterialien (5.2.1) und/oder partikuläre, anorganische, bakterizide und/oder viruzide Materialien (5.2.2) sowie (ii) partikuläre, anorganische Adsorbentien und/oder Absorbentien (5.2.3) umfasst.

11. Mobile Vorrichtung (1) nach einem der Ansprüche 1 bis 10, **dadurch gekennzeichnet, dass** der umlaufende Kühlring (2.4), die Heizplatte (2.1.2) und die Wärmeleitungen (2.1.1) eine Wärmeleitfähigkeit λ von 20 W/(m.K) bis 430 W/(m.K) haben.

12. Mobile Vorrichtung (1) nach einem der Ansprüche 1 bis 11, **dadurch gekennzeichnet, dass** die Außenwand (1.1), das Trägermaterial (5.2.1), die Wärmedämmbeschichtung (2.1.4) und das Bauteil (5.5) eine Wärmeleitfähigkeit λ von 0,001 W/(m.K) bis 1,0 W/(m.K) haben.

13. Mobile Vorrichtung (1) nach einem der Ansprüche 1 bis 12, **dadurch gekennzeichnet, dass** zwischen den kalten Seiten (2.2) der mindestens zwei Peltier-Element (2) und dem Kühlring (2.4), den heißen Seiten (2.1) der mindestens zwei Peltier-Element (2) und/oder den Wärmeleitungen (2.1.1) eine Aluminiumnitrid-Keramikplatte (2.3) und/oder eine Schicht aus einer Wärmeleitpaste (2.3) angeordnet ist.

14. Mobile Vorrichtung (1) nach einem der Ansprüche 1 bis 13, **dadurch gekennzeichnet, dass** die Kartuschenwand (5.1) aus mindestens einem Kunststoff einer Wärmeleitfähigkeit λ von 0,1 W/(m.K) bis 0,5 W/(m.K) und/oder mindestens einem Glas einer Wärmeleitfähigkeit λ von 0,5 W/(m.K) bis 1,5 W/(m.K) aufgebaut ist.

15. Verfahren zur Reinigung und Desinfizierung von Raumluft mithilfe einer durch eine Temperaturdifferenz betriebenen mobilen Vorrichtung (1) gemäß einem der Ansprüche 1 bis 14, **gekennzeichnet durch** die folgenden Verfahrensschritte:
(A) Ansaugen von Luft (3) durch mindestens einen Lufteinlass (3.1) in den Heizraum (2.1.6) oberhalb der Heizplatte (2.1.2),
(B) Aufheizen der einströmenden Luft (3) mithilfe der von den heißen Seiten (2.1) der mindestens zwei Peltier-Elemente (2) über die mindestens zwei Wärmeleitungen (2.1.1) aufgeheizten Heizplatte (2.1.2)
(C) Erzeugen eines Kamineffekts in der Kartusche (5) durch die Kühlung der umlaufenden Kühlrings (2.4) im oberen Bereich der Kartusche (5) mithilfe der kalten Seiten (2.2) der mindestens zwei Peltier-Elemente (2), wodurch ein Luftstrom (3.3) durch (i) die Schüttung aus mindestens einer Art eines partikulären, anorganischen, bakterizid und/oder viruzid beschichteten, inerten Trägermaterials (5.2.1) und/oder (ii) die Schüttung aus mindestens einer Art eines partikulären, anorganischen, bakteriziden und/oder viruziden Materials (5.2.2) und
(D) Eliminierung der in dem Luftstrom (3.2) vorhandenen freien oder an Aerosole gebundenen Bakterien, Viren, Virionen und anderen Noxen durch Kontakt-Eliminierung an dem Trägermaterial (5.2.1) und/oder dem Material (5.2.2).
